# EUROPEAN PATENT SPECIFICATION

(11) **EP 4 509 515 B1**
(45) Date of publication and mention of the grant of the patent: **29.04.2026**
(21) Application number: 24785175.1
(22) Date of filing: 02.04.2024
(51) Int. Cl.: C07F 11/00, C07C 2/34, C07C 11/02, C07C 11/107, B01J 31/18, B01J 31/14

(54) **LIGAND COMPOUND, ORGANIC CHROMIUM COMPOUND, AND CATALYST COMPOSITION COMPRISING SAME**
LIGANDENVERBINDUNG, ORGANISCHE CHROMVERBINDUNG UND KATALYSATORZUSAMMENSETZUNG DAMIT
COMPOSÉ LIGAND, COMPOSÉ DE CHROME ORGANIQUE ET COMPOSITION CATALYTIQUE LE CONTENANT

(30) Priority: 04.04.2023 KR 20230043953
(43) Date of publication of application: 19.02.2025
(73) Proprietor: LG Chem, Ltd., Yeongdeungpo-gu Seoul 07336 (KR)
(72) Inventor: KIM, Seok Sun, Daejeon 34122 (KR); CHO, Yeon Ho, Daejeon 34122 (KR); LIM, Won Taeck, Daejeon 34122 (KR); JUNG, Seung Hwan, Daejeon 34122 (KR); SHIN, Eun Ji, Daejeon 34122 (KR); SA, Seok Pil, Daejeon 34122 (KR); KIM, Tae Hee, Daejeon 34122 (KR); MIN, Se Hye, Daejeon 34122 (KR)
(74) Representative: Hoffmann Eitle
(86) International application number: PCT/KR2024/004258
(87) International publication number: WO 2024/210470

(56) References cited:
- KR-A- 20190 139 028
- KR-A- 20220 162 021
- US-A1- 2003 166 456
- US-A1- 2003 166 456
- US-A1- 2011 282 016
- BRITOVSEK GEORGE J. ET AL: "Mechanistic study of ethylene tri- and tetramerisation with Cr/PNP catalysts: effects of additional donors", vol. 6, no. 23, 1 January 2016 (2016-01-01), UK, pages 8234 - 8241, XP093276336, ISSN: 2044-4753, Retrieved from the Internet <URL:https://pubs.rsc.org/en/content/articlepdf/2016/cy/c6cy02112c> DOI: 10.1039/C6CY02112C
- OVERETT, M. J. ET AL.: "Ethylene trimerisation and tetramerisation catalysts with polar-substituted diphosphinoamine ligands", CHEMICAL COMMUNICATIONS, vol. 5, no. 5, 2005, pages 622 - 624, XP002434088, DOI: 10.1039/b412432d

## Description

### TECHNICAL FIELD

The present invention relates to a ligand compound, an organic chromium compound, a catalyst composition including the organic chromium compound, and a method for oligomerizing ethylene by using the same.

### BACKGROUND ART

Linear alpha-olefins such as 1-hexene and 1-octene are used as cleaning agents, lubricants, plasticizers, etc., and particularly, are mainly used as a co-monomer for polymer density control during the production of linear low-density polyethylene (LLDPE).

In a typical production process of linear low-density polyethylene (LLDPE), in order to control the density by forming a branch in a polymer backbone with ethylene, copolymerization is performed with a co-monomer such as an alpha-olefin, for example, 1-hexene or 1-octene.

Therefore, in order to produce LLDPE having a high co-monomer content, there has been a problem in that the price of the co-monomer accounts for a large portion of the production cost. There have been many attempts to solve the above problem.

Such linear alpha-olefins have been mainly prepared through the Shell Higher Olefin Process. However, the above method simultaneously synthesizes alpha-olefins having various lengths according to the Schultz-Flory distribution, so that a separate separation process is required to obtain a specific alpha-olefin, which is cumbersome.

In order to solve the above problem, a method for selectively synthesizing 1-hexene through a trimerization reaction of ethylene or selectively synthesizing 1-octene through a tetramerization reaction of ethylene has been proposed. In addition, many studies have been conducted on a catalyst system that enables such selective oligomerization of ethylene.

### Prior Art Documents

Patent Document 1: US 5,064,802 B2
Patent Document 2: US 2003/166456 A1
Non-patent Document 1: Catalysis Science & Technology, 2016, vol. 6, pp. 8234-8241

Patent Document 2 and Non-patent Document 1 disclose aromatic PNP ligands for use as components of olefin oligomerization catalysts.

### DISCLOSURE OF THE INVENTION

The present invention provides a ligand compound having a novel structure, which exhibits high catalytic activity while exhibiting high 1-hexene and 1-octene selectivity, thereby allowing ethylene oligomerization to be achieved with excellent efficiency, and provides an organic chromium compound, a catalyst composition including the organic chromium compound, and an ethylene oligomerization method.
(1) The present invention provides a ligand compound represented by any one among Formula 2 to Formula 9 below: 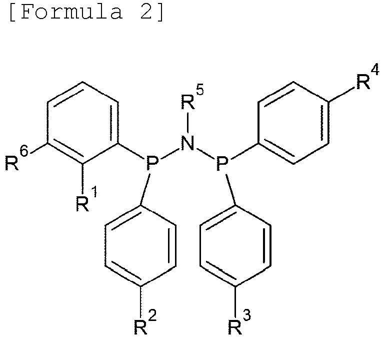 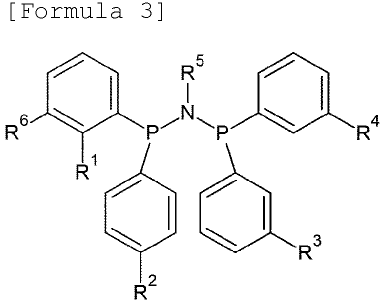 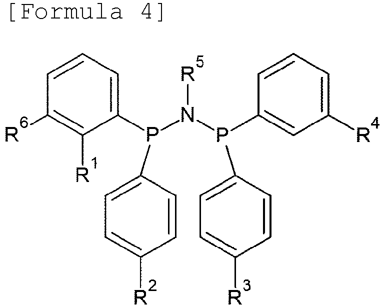 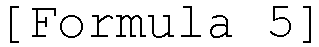 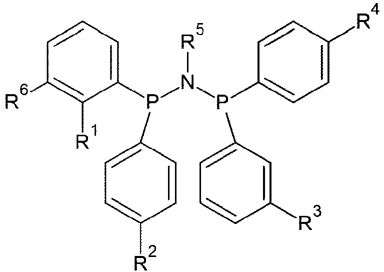 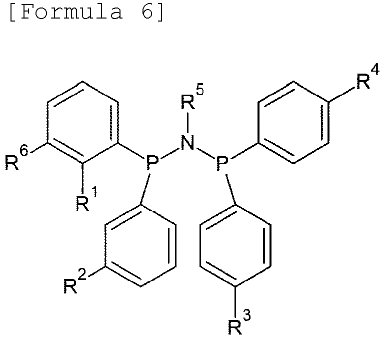 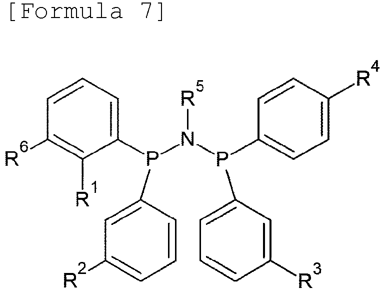 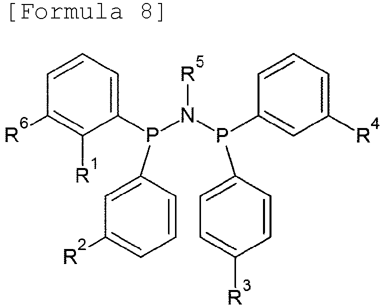 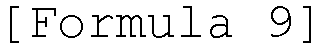 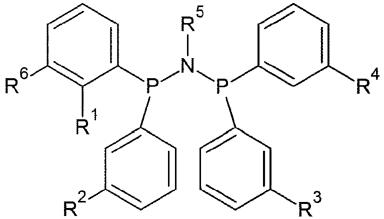 wherein in Formula 2 to Formula 9 above,
   R¹ is a halogen group, an alkyl group having 1 to 10 carbon atoms substituted or unsubstituted with a halogen group, an alkoxy group having 1 to 10 carbon atoms substituted or unsubstituted with a halogen group, an alkylthio group having 1 to 10 carbon atoms substituted or unsubstituted with a halogen group, an alkylsulfonate group having 1 to 10 carbon atoms substituted or unsubstituted with a halogen group, or a trialkylsilyl group in which the alkyl groups each independently have 1 to 10 carbon atoms, or R¹ is bonded to R⁶ to form a monocyclic or polycyclic aromatic hydrocarbon ring, or a monocyclic or polycyclic hetero ring, wherein if R⁶ and R¹ are not bonded to each other to form a monocyclic or polycyclic aromatic hydrocarbon ring, or a monocyclic or polycyclic hetero ring, R⁶ is hydrogen;
   R² to R⁴ are each independently hydrogen, an alkyl group having 5 to 20 carbon atoms, a cycloalkyl group having 5 to 20 carbon atoms, or a trialkylsilyl group in which the alkyl groups each independently have 1 to 10 carbon atoms, wherein R² to R⁴ are not hydrogen at the same time; and
   R⁵ is an alkyl group having 1 to 20 carbon atoms, an alkyl group having 1 to 20 carbon atoms and substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.
(2) In (1) above, the present invention provides a ligand compound, wherein R¹ is fluoro, an alkyl group having 1 to 5 carbon atoms substituted or unsubstituted with fluoro, an alkoxy group having 1 to 5 carbon atoms substituted or unsubstituted with fluoro, an alkylthio group having 1 to 5 carbon atoms substituted or unsubstituted with fluoro, an alkylsulfonate group having 1 to 5 carbon atoms substituted or unsubstituted with fluoro, or a trialkylsilyl group (alkyl groups of the trialkylsilyl group are each independently an alkyl group having 1 to 5 carbon atoms), or is bonded to R⁶ to form a monocyclic or polycyclic hetero ring.
(3) In (1) or (2) above, the present invention provides a ligand compound, wherein R¹ is a fluoro group, a trifluoromethyl group, a methoxy group, a methyl group, a trifluoromethoxy group, a trifluoromethylthio group, a methylthio group, a methylsulfonate group, or a trimethylsilyl group, or is bonded to R⁶ to form furan or dibenzofuran.
(4) In any one among (1) to (3) above, the present invention provides a ligand compound, wherein R² to R⁴ are each independently hydrogen, an alkyl group having 8 to 12 carbon atoms, a tripropylsilyl group, or a tributylsilyl group, wherein R² to R⁴ are not hydrogen at the same time.
(5) In any one among (1) to (4) above, the present invention provides a ligand compound, wherein R² to R⁴ are each independently an n-decyl group, a tripropylsilyl group, or a tributylsilyl group.
(6) In any one among (1) to (4) above, the present invention provides a ligand compound, wherein R² is hydrogen, and R³ and R⁴ are each independently an n-decyl group, a tripropylsilyl group, or a tributylsilyl group.
(7) In any one among (1) to (6) above, the present invention provides a ligand compound, wherein R⁵ is an alkyl group having 3 to 5 carbon atoms, an alkyl group having 1 to 5 carbon atoms substituted with an aryl group having 6 to 10 carbon atoms, a cycloalkyl group having 5 to 8 carbon atoms, or a cycloalkyl group having 5 to 10 carbon atoms fused with an aryl group having 6 to 10 carbon atoms.
(8) In any one among (1) to (7) above, the present invention provides a ligand compound represented by any one among Formula 2-1 to Formula 2-96 and Formula 9-1 to Formula 9-20 below. 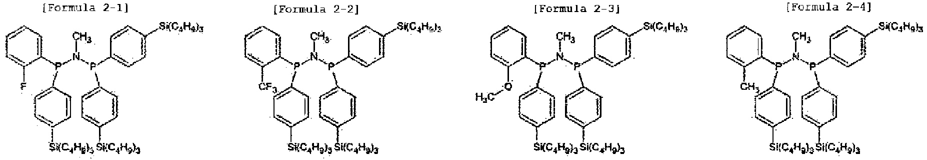 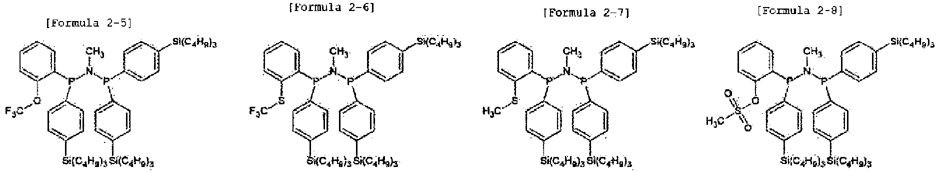 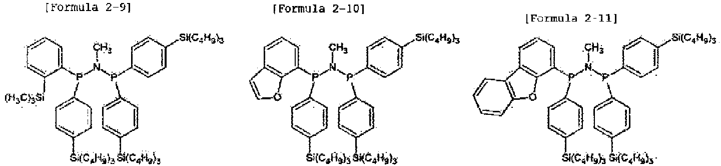 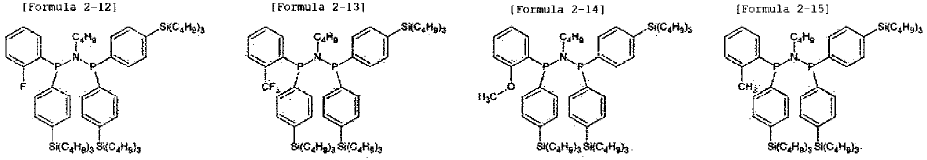 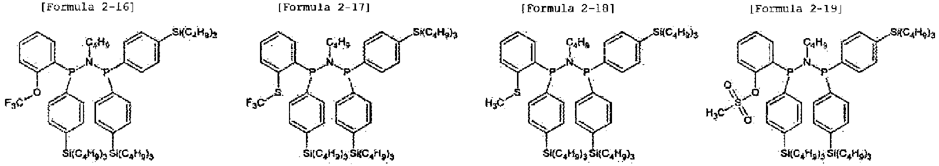 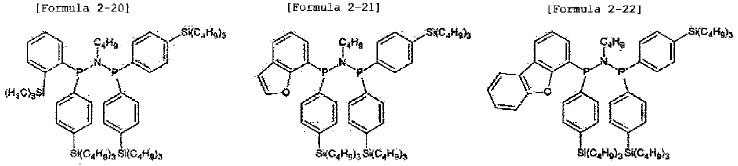 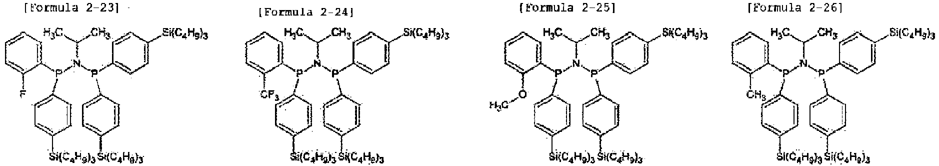 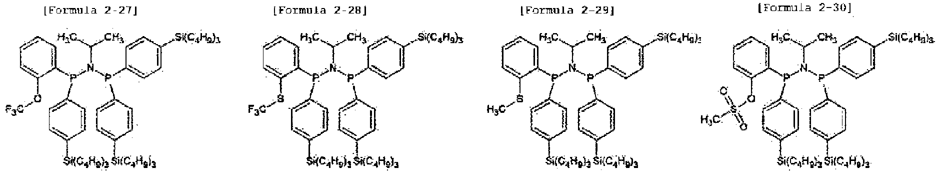 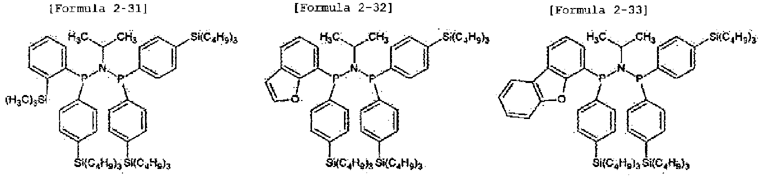 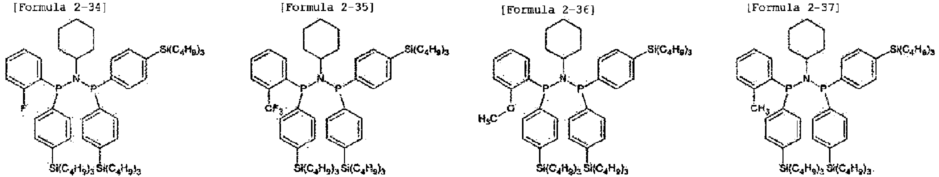 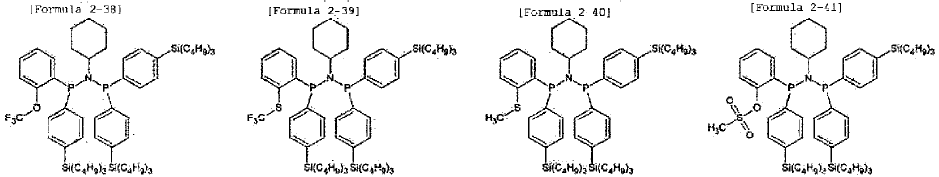 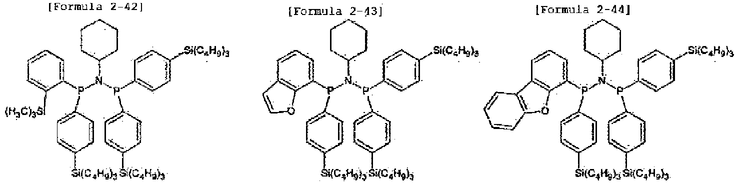 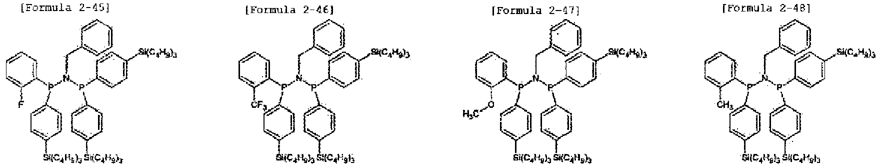 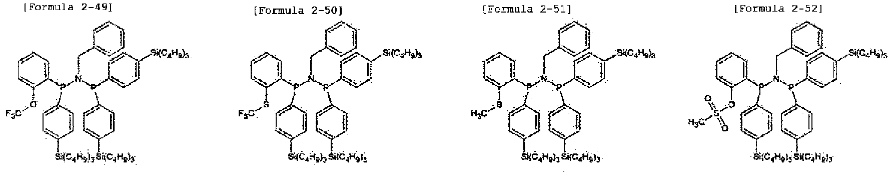 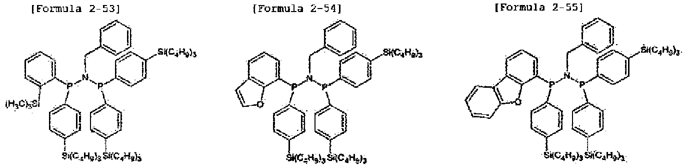 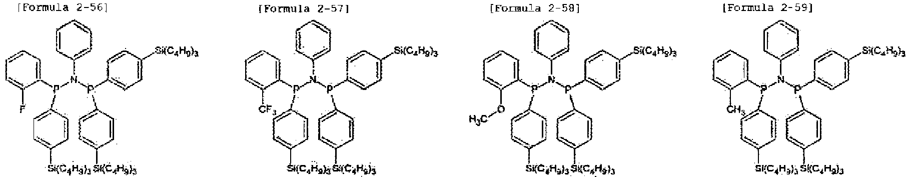 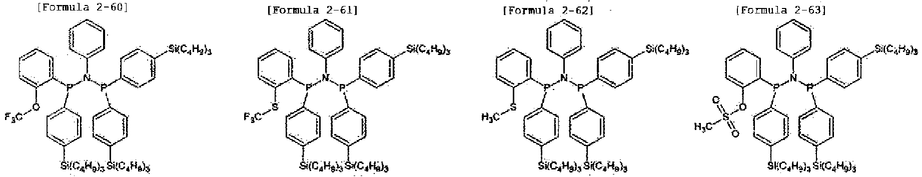 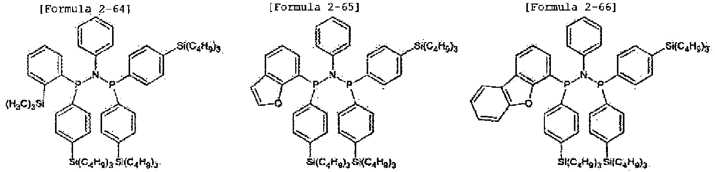 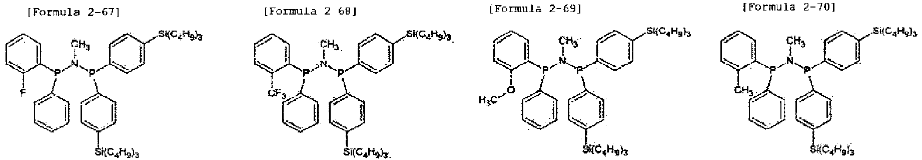 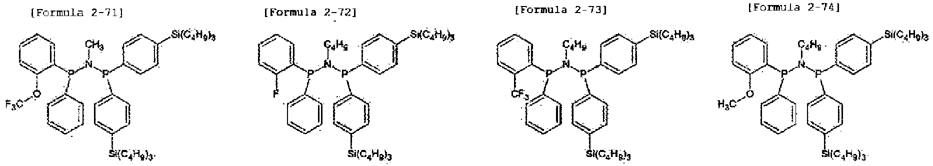 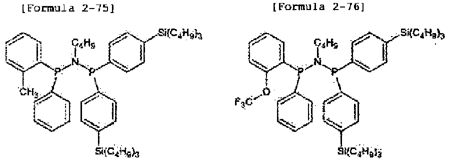 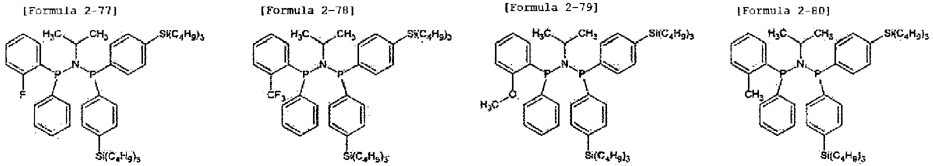 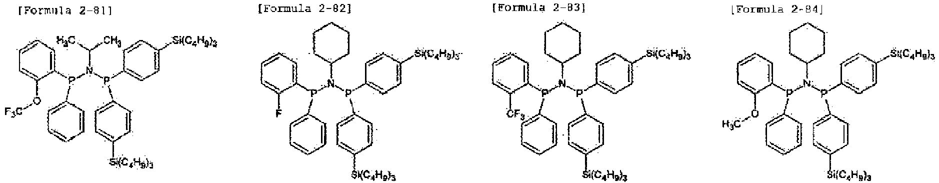 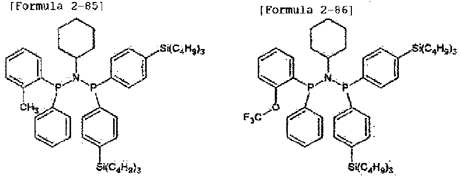 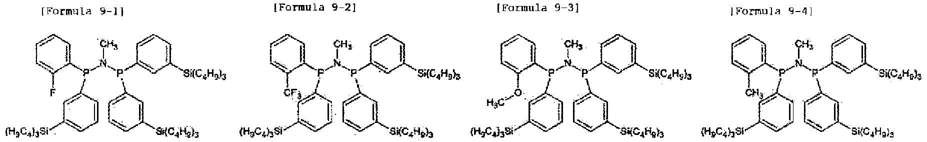 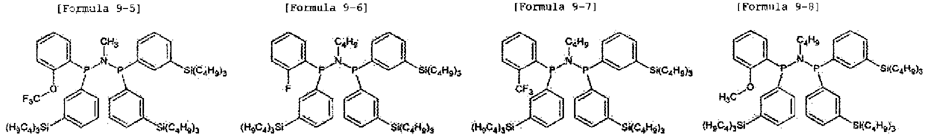 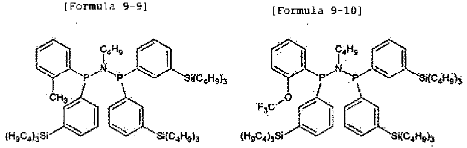 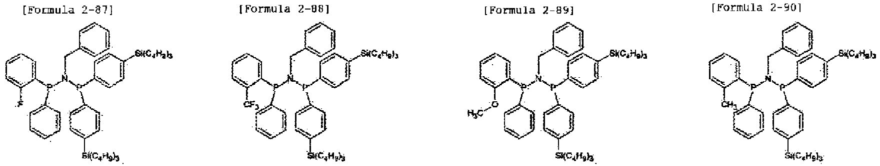 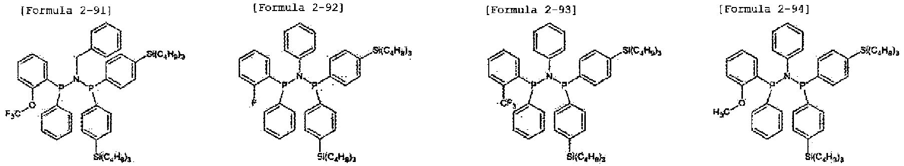 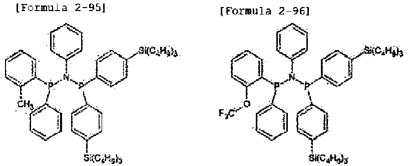 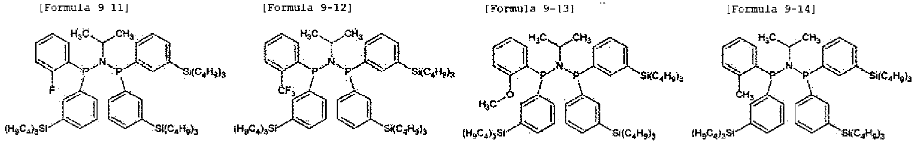 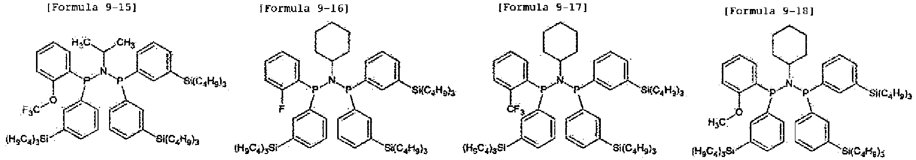 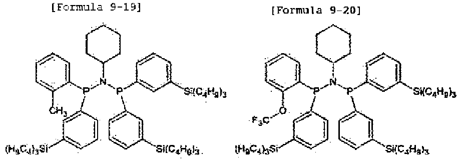
(9) The present invention provides an organic chromium compound including the ligand compound according to any one among (1) to (8) above and chromium bidentated to the ligand compound.
(10) In (9) above, the present invention provides an organic chromium compound, wherein in the ligand compound, at least one unshared electron pair of N and two Ps is in the form of being bidentated to chromium.
(11) The present invention provides a catalyst composition including the ligand compound according to any one among (1) to (8) above, chromium, and a co-catalyst.
(12) In (11) above, the present invention provides a catalyst composition, wherein the chromium is derived from a chromium source, wherein the chromium source includes at least one selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium( III) laurate, chromium(III) tris(2,2,6,6-tetramethyl-3.5-heptanedionate), and chromium(III) stearate.
(13) In (11) or (12) above, the present invention provides a catalyst composition, wherein the co-catalyst is at least one selected from the group consisting of compounds represented by Formula 10 to Formula 13 below.

   [Formula 10] - [Al(R¹³)-O]ₐ-

   In Formula 10 above, R¹³ is each independently a halogen group, a hydrocarbyl group having 1 to 20 carbon atoms, or a hydrocarbyl group having 1 to 20 carbon atoms substituted with a halogen group, and a is an integer of 2 or greater,

      [Formula 11] E(R¹⁴)₃
   in Formula 11 above, E is aluminum or boron, and R¹⁴ is each independently hydrogen, a halogen group, a hydrocarbyl group having 1 to 20 carbon atoms, or a hydrocarbyl group having 1 to 20 carbon atoms substituted with a halogen group, and

      [Formula 12] [L-H]⁺[G(Y)₄]⁻

      [Formula 13] [L]⁺[G(Y)₄]⁻
   in Formulas 12 and 13 above, L is a neutral or cationic Lewis acid, [L-H]⁺ is a Bronsted acid, G is a Group 13 element, and Y is each independently a substituted or unsubstituted alkyl group having 1 to 20 carbon atoms or a substituted or unsubstituted aryl group having 6 to 20 carbon atoms, and here, if the alkyl group or aryl group is substituted, the substituent is a halogen group, a hydrocarbyl group having 1 to 20 carbon atoms, an alkoxy group having 1 to 20 carbon atoms, or an aryloxy group having 6 to 20 carbon atoms.
(14) The present invention provides a method for preparing a linear alpha-olefin, the method including oligomerizing ethylene (S10) in the presence of the catalyst composition according to any one among (11) to (13) above.
(15) In (14) above, the present invention provides a method for preparing a linear alpha-olefin, wherein the linear alpha-olefin is 1-hexene, 1-octene, or a mixture thereof.

### ADVANTAGEOUS EFFECTS

When ethylene oligomerization is performed using an organic chromium compound including a ligand compound of the present invention and a catalyst composition, it is possible to prepare linear alpha-olefins with high 1-hexene and 1-octene selectivity while having excellent productivity due to high catalytic activity.

### MODE FOR CARRYING OUT THE INVENTION

Hereinafter, the present invention will be described in more detail to facilitate understanding of the present invention.

It will be understood that words or terms used in the description and claims of the present invention shall not be construed as being limited to having the meaning defined in commonly used dictionaries. It will be further understood that the words or terms should be interpreted as having meanings that are consistent with their meanings in the context of the relevant art and the technical idea of the invention, based on the principle that an inventor may properly define the meaning of the words or terms to best explain the invention.

### Ligand compound

The present invention provides a ligand compound applicable to a catalyst used in an ethylene oligomerization reaction. When the ligand compound is applied to an ethylene oligomerization reaction, specifically a catalyst composition for forming a linear alpha-olefin, the selectivity with respect to linear alpha-olefins is high while excellent catalytic activity is exhibited, and particularly, when compared with a catalyst including a symmetrical ligand compound as well as a typical PNP-based catalyst, the production amount of solid polyethylene is small even under the same reaction condition, so that linear alpha-olefins may be more efficiently prepared.

According to an embodiment of the present invention, an organic chromium compound to which the ligand compound is bidentated may be utilized for preparing a linear alpha-olefin using ethylene, and an oligomerization reaction proceeds in a reaction under the ethylene condition, so that an alpha-olefin in a liquid form, specifically, 1-hexene or 1-octene in a liquid form, may be formed with high selectivity. This is because selectivity with respect to alpha-olefins of a certain length increases through a transition state that forms metallacycles in the oligomerization reaction of ethylene.

According to an embodiment of the present invention, the ligand compound includes a diphosphino aminyl moiety, wherein an aryl having a specific substituent is connected to the end of the diphosphino aminyl moiety, and thus, may have a form that is capable of serving as a strong electron donating group itself. Due to the structural feature, the ligand compound may be applied to an oligomerization catalyst system for ethylene and exhibit high activity, and particularly, may exhibit high selectivity with respect to 1-hexene, 1-octene, or the like. This may be due to an interaction between adjacent chromium activity points, and particularly, when an aryl substituted with a specific substituent is connected to a phosphorus (P) atom of diphosphino aminyl, the electron density is increased in the phosphorus (P) atom and a nitrogen (N) atom included in the diphosphinoaminyl, and the electrical and steric properties of the entire ligand compound change. Accordingly, since there is a change in the bond between a ligand and a chromium atom, the structure of a catalyst may become more stable, and compared to a typical metallacycloheptane or metallacyclononane form, it is possible to form alpha-olefins with higher activity and selectivity by changing the energy (activation energy) in the transition state, and to further reduce the amount of by-products of high-molecular weight solid alpha-olefins and the like, such as polyethylene wax (PE wax).

According to an embodiment of the present invention, the ligand compound is characterized in that phenyl positioned at the end of the diphosphino aminyl moiety has, as a substituent, a halogen group, an alkyl group, an alkoxy group, or an alkoxy group substituted with at least one fluorine, at the ortho position, and has, as a substituent, an alkyl group having a specific carbon number, or a silyl group substituted with the alkyl group having a specific carbon number, at each of the meta position and the para position. The substituent substituted at the ortho position of the phenyl may increase a steric strain around a metal atom to increase the selectivity of 1-hexene, and also, may protect the metal atom or directly form a bidentated bond to serve to improve the stability of a metal complex compound. At the same time, the substituents substituted at the meta position and para position of the phenyl may respectively increase the solubility of the ligand compound and the metal complex compound in a polymerization solvent to improve the activity and selectivity. Accordingly, if the ligand compound is used, a chromium catalyst with high stability and excellent activity and selectivity may be prepared.

According to an embodiment of the present invention, in the ligand compound, a nitrogen atom to which two phosphorus atoms are bonded is bonded to a bulky substituent such as a cycloalkyl group or phenyl group, wherein the bulky substituent bonded to nitrogen may prevent the bond of nitrogen and phosphorus from rotating, so that the stability and the activity of the catalyst may be further improved. At this time, the activity, stability, and selectivity of the catalyst change according to the steric properties of the substituent entity bonded to the nitrogen atom. If the steric strain of the substituent bonded to the nitrogen atom is too high, there is a problem in that ligand synthesis and formation of a metal complex compound become difficult, and the generated complex compound becomes unstable. In addition, if the steric strain of the substituent bonded to the nitrogen atom is too high, there is a problem in that the access to raw materials such as ethylene become difficult, thereby degrading the activity of the catalyst. In addition, if the steric strain of the substituent bonded to the nitrogen atom is too low, it is not possible to prevent the rotation of the bond of the nitrogen atom and the phosphorus atom, and to protect a metal center atom, thereby causing the activity and stability of the catalyst to be degraded. That is, if the steric strain of the substituent bonded to the nitrogen atom is either too high or too low, the activity of the catalyst is lowered, and the stability there of is degraded, so that there is a problem in which the amount of generated by-products such as polyethylene wax will increase. Therefore, it is very important to select a substituent bonded to the nitrogen atom that has a suitable level of steric strain with respect to a substituent bonded to the phosphorus atom. In the ligand compound according to the present invention, the steric strain around a P-N-P functional group increases due to the introduction of a substituent at a meta position of a phenyl group bonded to a phosphorus atom, and therefore, by introducing a substituent represented by R⁵ of Formula 1 in order to prevent the steric strain of a substituent bonded to a nitrogen atom from increasing significantly, it is possible to improve the yield and selectivity during an oligomerization reaction of ethylene by using a catalyst composition including the same. Particularly, when a substituent in the form of a secondary alkyl group is introduced as the substituent represented by R⁵ of Formula 1, the efficiency is further improved, and a substituent in the form of a primary alkyl group may form a suitable steric strain by introducing an aryl group such as a phenyl group at positions of carbon number 1 and carbon number 2 to compensate for a low steric strain, so that yield and selectivity may be improved.

In the present invention, "halogen group" refers to fluorine (-F), chlorine (-Cl), bromine (-Br), and iodine (-I).

In the present invention, "substituted with a halogen group" may mean that at least one hydrogen atom bonded to a carbon atom of each substituent is substituted with a halogen atom, or if referring to the number of carbon atoms of each substituent, may refer to the number of carbon atoms of each substituent.

In the present invention, "alkyl group" may refer to a linear or branched hydrocarbon moiety, and a specific example thereof may be, depending on the number of carbon atoms defined, a methyl group, an ethyl group, an n-propyl group, an isopropyl group, an n-butyl group, an isobutyl group, a t-butyl group, an n-pentyl group, an isopentyl group, a hexyl group, or the like.

In the present invention, "alkylthio group" may mean that a substituent on either side of a sulfide bond is an alkyl group, and if referring to the number of carbon atoms, may refer to the number of carbon atoms of the alkyl group.

In the present invention, "alkyl sulfonate group" may mean that a sulfur atom of sulfonate is substituted with an alkyl group, and if referring to the number of carbon atoms, may refer to the number of carbon atoms of the alkyl group.

In the present invention, "trialkylsilyl group" is represented by -SiR₃, and refers a substituent in which each R is independently an alkyl group, and the carbon number of the trialkylsilyl group may refer to the sum of the carbon numbers of all of R.

In the present invention, "monocyclic aromatic hydrocarbon ring" may refer to an aromatic hydrocarbon composed of one ring, such as a benzene ring, and "polycyclic aromatic hydrocarbon ring" may refer to an aromatic hydrocarbon composed of two or more rings, such as a naphthalene ring.

In the present invention, "monocyclic hetero ring" may refer to a hetero ring composed of one ring containing hetero atoms, such as furan, thiophene, pyrrole, and pyridine, and "polycyclic hetero ring" may refer a hetero ring composed of two or more rings containing hetero atoms, such as benzofuran, dibenzofuran, benzothiophene, dibenzothiophene, benzopyrrole, and dibenzopyrrole. The hetero ring may form an aliphatic or aromatic ring.

In the present invention, "cycloalkyl group" may refer to a cyclic hydrocarbon moiety, and a specific example thereof may be, depending on the number of carbon atoms defined, a cyclopentyl group, a cyclohexyl group, a cycloheptyl group, a cyclooctyl group, or the like.

In the present invention, "alkoxyalkyl group" may refer to an alkyl group substituted with an alkoxy group, and the carbon number of the alkoxyalkyl group may refer to the sum of the carbon number of the alkoxy group and the carbon number of the alkyl group.

In the present invention, "arylalkoxyalkyl group" may refer to an alkyl group substituted with an alkoxy group including an aryl group as a substituent, and the carbon number of the arylalkoxyalkyl group may refer to the sum of the carbon number of the alkoxy group and the carbon number of the alkyl group.

In the present invention, unless otherwise specified, "aryl" refers to an arbitrarily substituted benzene ring, or to a ring system which may be formed by fusing one or more arbitrary substituents. An example of the arbitrary substituent includes a substituted alkyl group having 1 to 2 carbon atoms, a substituted alkenyl group having 2 to 3 carbon atoms, a substituted alkynyl group having 2 to 3 carbon atoms, a heteroaryl group, a heterocyclic group, an aryl group, an alkoxy arbitrarily having 1 to 3 fluorine substituents, an aryloxy, an aralkoxy, an acyl, an aroyl, a heteroaroyl, an acyloxy, an aroyloxy, a heteroaroyloxy, a sulfanyl, a sulfinyl, a sulfonyl, an aminosulfonyl, a sulfonylamino, a carboxyamide, an aminocarbonyl, a carboxy, an oxo, a hydroxy, a mercapto, an amino, a nitro, a cyano, a halogen or an ureido. Such a ring or ring system may be arbitrarily fused to an aryl ring arbitrarily having one or more substituents (e.g., a benzene ring), a carbocyclic ring, or a heterocyclic ring. It may include, but is not limited to, phenyl, naphthyl, tetrahydronaphthyl, biphenyl, indanyl, anthracyl, or phenanthryl, and a substituted derivative thereof.

According to an embodiment of the present invention, R¹ may be a fluoro group, a chloro group, a bromo group, an alkyl group having 1 to 5 carbon atoms substituted or unsubstituted with fluoro, an alkoxy group having 1 to 5 carbon atoms substituted or unsubstituted with fluoro, an alkylthio group having 1 to 5 carbon atoms substituted or unsubstituted with fluoro, an alkylsulfonate group having 1 to 5 carbon atoms substituted or unsubstituted with fluoro, or a trialkylsilyl group (alkyl groups of the trialkylsilyl group are each independently an alkyl group having 1 to 5 carbon atoms), or may be bonded to R⁶ to form a monocyclic or polycyclic hetero ring. As a specific example, R¹ may be a fluoro group, a trifluoromethyl group, a methoxy group, a methyl group, a trifluoromethoxy group, a trifluoromethylthio group, a methylthio group, a methylsulfonate group, or a trimethylsilyl group, or may be bonded to R⁶ to form furan or dibenzofuran.

According to an embodiment of the present invention, R² to R⁴ may each independently be hydrogen, an alkyl group having 5 to 20 carbon atoms, a cycloalkyl group having 5 to 12 carbon atoms, or a trialkylsilyl group, wherein R² to R⁴ may not be hydrogen at the same time, and at this time, alkyl groups of the trialkylsilyl group may each independently be an alkyl group having 1 to 5 carbon atoms. As a specific example, R² to R⁴ may each independently be hydrogen, an alkyl group having 8 to 12 carbon atoms, a tripropylsilyl group, or a tributylsilyl group, wherein R² to R⁴ may not be hydrogen at the same time. As a more specific example, R² to R⁴ may each independently be an n-decyl group, a tripropylsilyl group, or a tributylsilyl group, and as another example, R² may be hydrogen, and R³ and R⁴ may each independently be an n-decyl group, a tripropylsilyl group, or a tributylsilyl group. That is, in the ligand compound, phenyl positioned at the end of a diphosphino aminyl moiety may have a substituent of R¹ at one ortho position, and at the same time, may have, as a substituent, an alkyl group having 10 carbon atoms, or a silyl group substituted with an alkyl group having 3 or 4 carbon atoms, at the meta or para position.

According to an embodiment of the present invention, R⁵ may be an alkyl group having 1 to 10 carbon atoms, an alkyl group having 1 to 10 carbon atoms substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms. As a specific example, R⁵ may be an alkyl group having 3 to 5 carbon atoms, an alkyl group having 1 to 5 carbon atoms substituted with an aryl group having 6 to 10 carbon atoms, a cycloalkyl group having 5 to 8 carbon atoms, or a cycloalkyl group having 5 to 8 carbon atoms fused with an aryl group having 6 to 10 carbon atoms.

According to an embodiment of the present invention, the ligand compound may be represented by any one among Formula 2-1 to Formula 2-96 and Formula 9-1 to Formula 9-20 below.

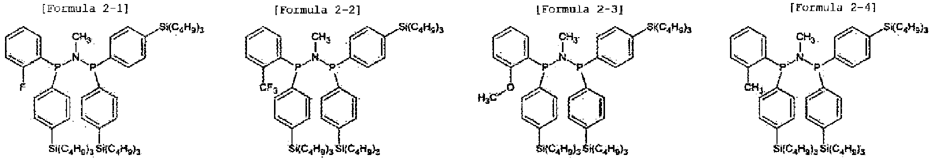

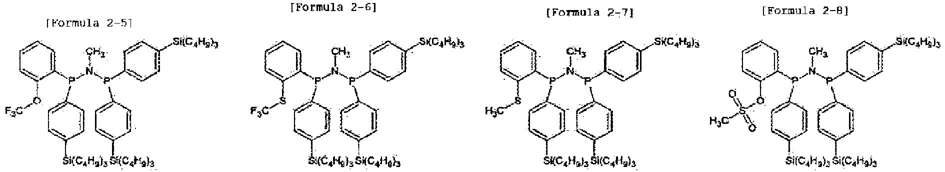

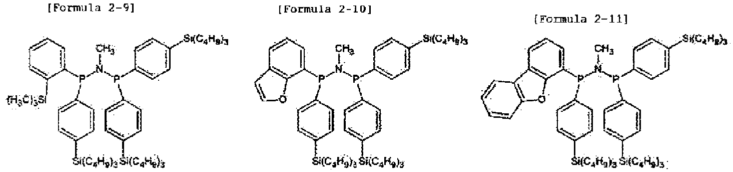

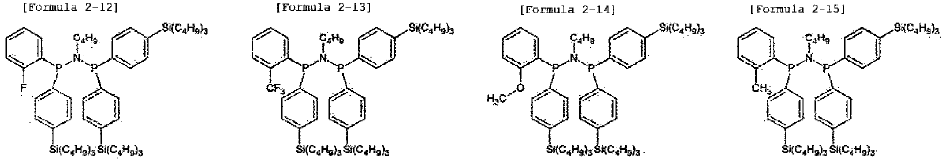

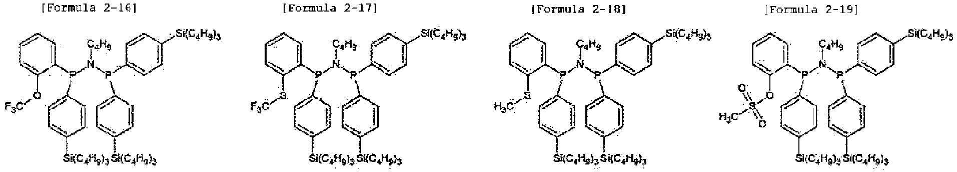

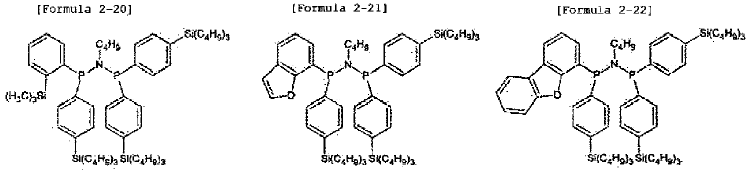

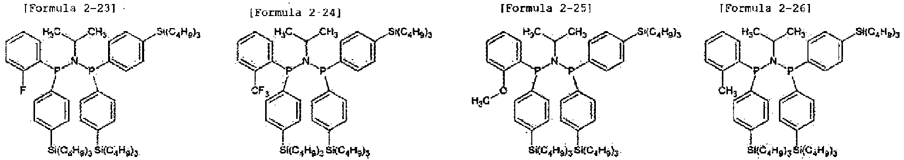

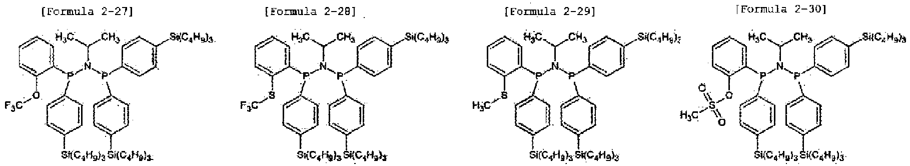

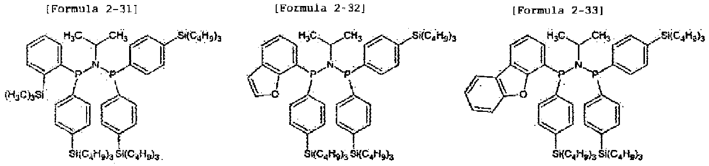

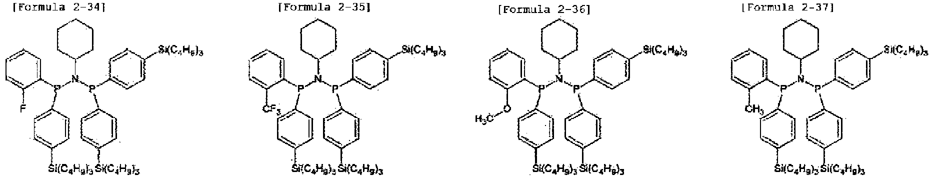

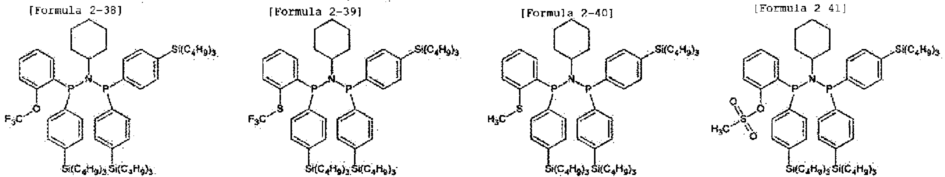

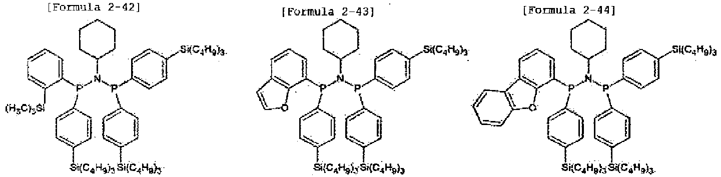

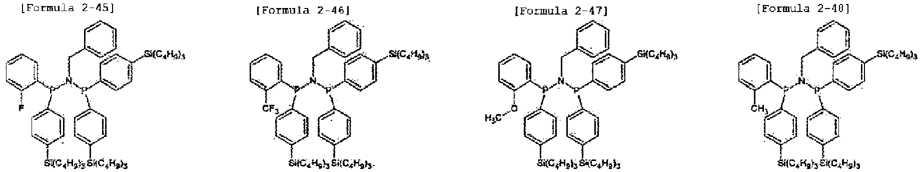

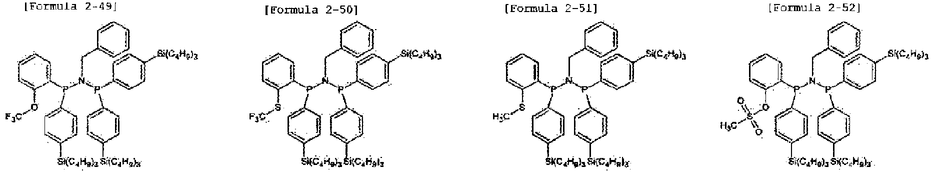

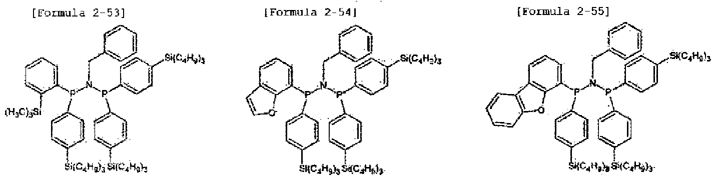

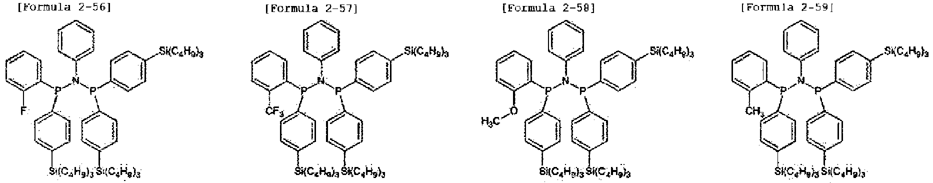

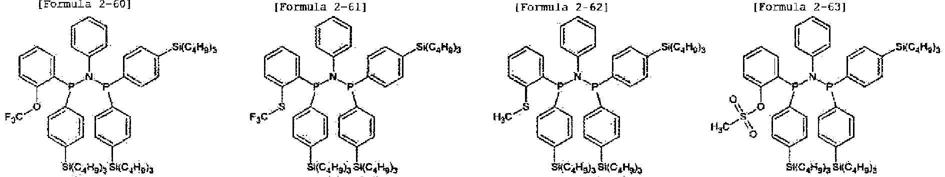

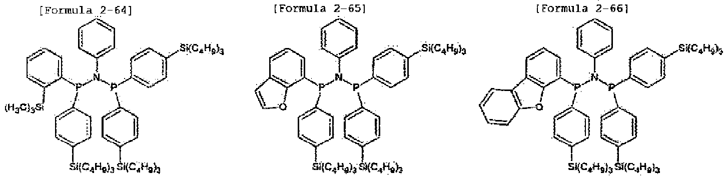

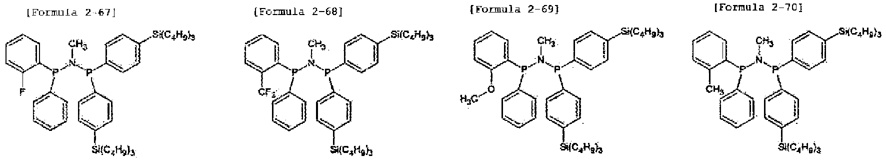

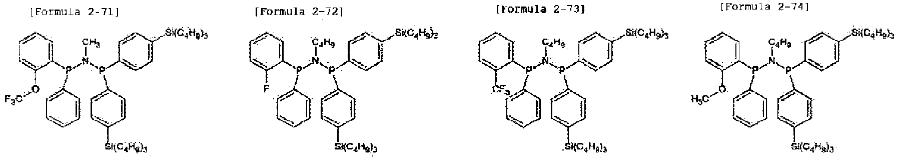

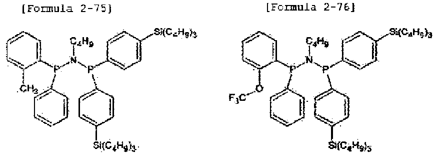

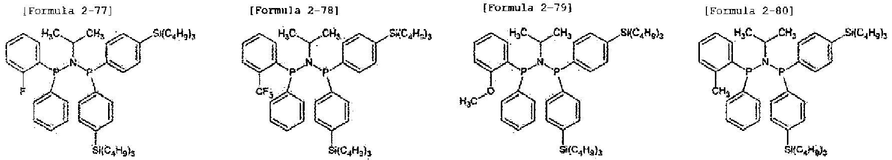

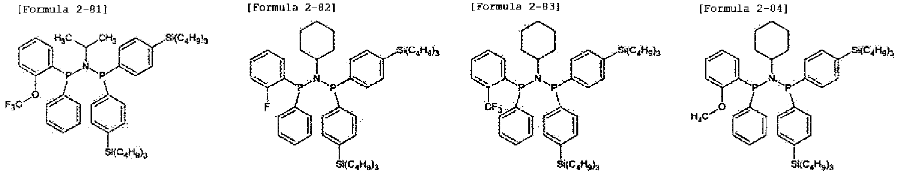

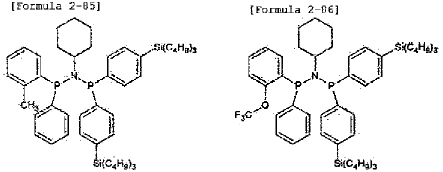

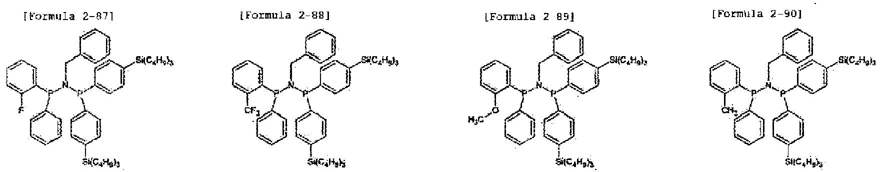

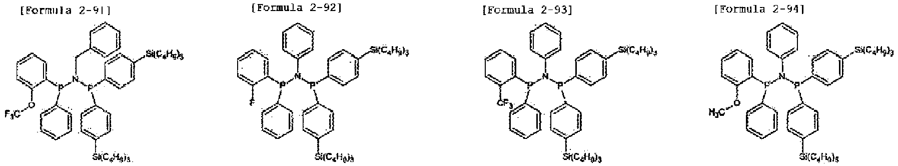

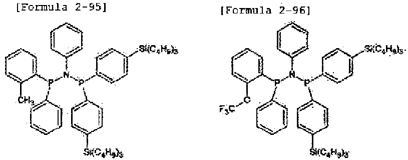

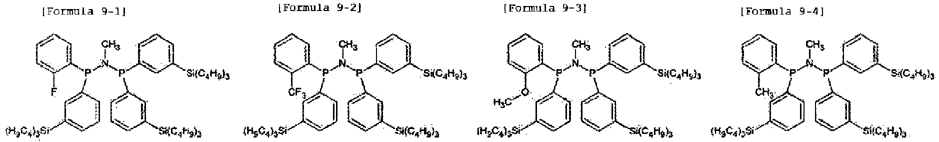

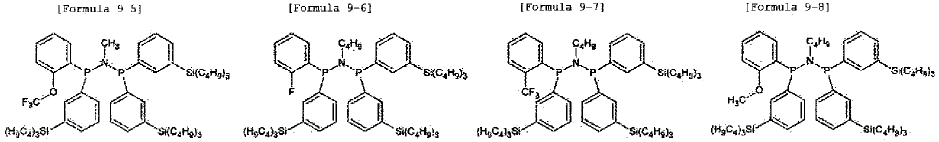

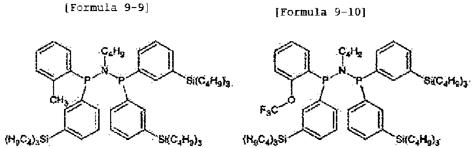

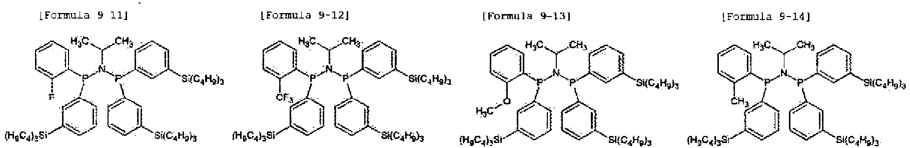

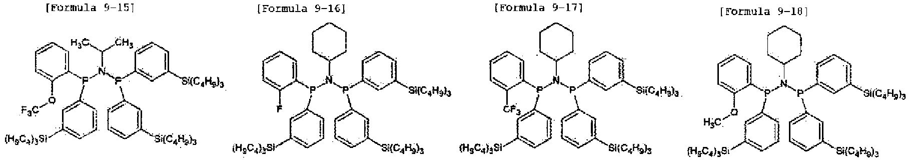

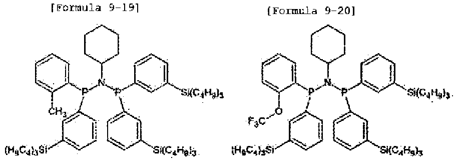

According to an embodiment of the present invention, in addition to the above-described embodiments, the ligand compound may be implemented in various combinations within a range that satisfies the above-described conditions, and any compound represented by Formula 1 may be applied as the ligand compound of the present invention.

### Organic chromium compound and catalyst composition

The present invention provides an organic chromium compound including the ligand compound represented by Formula 1 above and chromium (Cr) bidentated to the ligand compound.

According to an embodiment of the present invention, the organic chromium compound is a chromium complex compound of the ligand compound, in which chromium of a chromium source may have a form in which at least one unshared electron pair of N and two Ps has a bidentated bond in the ligand compound represented by Formula 1. That is, it is a structure in which a phosphorus atom or a nitrogen atom of a diphosphino aminyl moiety provides an unshared electron pair to a chromium atom, and particularly, a bidentated state in which two pairs of unshared electron pairs are bidentated may be preferred. The organic chromium compound may be applied to a catalyst system for an oligomerization reaction of ethylene and exhibit excellent catalytic activity and high selectivity with respect to 1-hexene or 1-octene.

In the present invention, "catalyst composition" may refer to one in a state in which three components including a chromium source, a ligand compound, and a co-catalyst, or two components of a transition metal compound and a co-catalyst are added simultaneously or in an arbitrary order to be obtained as an active catalyst composition. Here, the catalyst composition may also be referred to as a catalyst system, and in the present invention, the catalyst composition and catalyst system represent the same meaning. The three or two components of the catalyst composition may be added in the presence or absence of a solvent and a monomer, and may be used in a supported or non-supported state.

The present invention provides a catalyst composition including the above ligand compound, chromium, and a co-catalyst. As described above, the ligand compound represented by Formula 1 above and chromium may be bidentated to form an organic chromium compound. That is, the catalyst system may be a three-component catalyst system including chromium, the ligand compound represented by Formula 1 above, and a co-catalyst, or a two-component catalyst system including the organic chromium compound and a co-catalyst. As a specific example, the catalyst composition may include the ligand compound, an organic chromium compound including chromium bidentated to the ligand compound, and a co-catalyst. In addition, the catalyst composition may include a chromium compound in which some components of the co-catalyst are bonded to the organic chromium compound.

According to an embodiment of the present invention, the chromium may be derived from a chromium source, and the chromium source may be an organic or inorganic chromium compound in which the oxidation state of chromium is about 0 to about 6. As a specific example, the chromium source may be a chromium metal, or a compound in which any organic or inorganic radical is bonded to chromium. Here, the organic radical may be an alkyl radical, an alkoxy radical, an ester radical, a ketone radical, an amido radical, a carboxylate radical, or the like having 1 to 20 carbon atoms per radical, and the inorganic radical may be a halide, a sulfate, an oxide, or the like.

According to an embodiment of the present invention, the chromium source is a compound which exhibits high activity in oligomerization of olefins, and which is easy to use and obtain, and may be at least one compound selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium( III) laurate, chromium(III) tris(2,2,6,6-tetramethyl-3.5-heptanedionate), and chromium(III) stearate.

According to an embodiment of the present invention, the co-catalyst may be one or more selected from the group consisting of compounds represented by Formula 10 to Formula 13 below.

[Formula 10] -[Al(R¹³)-O]ₐ-

In Formula 10 above, R¹³ is each independently a halogen group, a hydrocarbyl group having 1 to 20 carbon atoms, or a hydrocarbyl group having 1 to 20 carbon atoms substituted with a halogen group, and a is an integer of 2 or greater.

[Formula 11] E(R¹⁴)₃

In Formula 11 above, E is aluminum or boron, and R¹⁴ is each independently hydrogen, a halogen group, a hydrocarbyl group having 1 to 20 carbon atoms, or a hydrocarbyl group having 1 to 20 carbon atoms substituted with a halogen group.

[Formula 12] [L-H]⁺[G(Y)₄]⁻

[Formula 13] [L]⁺[G(Y)₄]⁻

In Formulas 12 and 13 above, L is a neutral or cationic Lewis acid, [L-H]⁺ is a Bronsted acid, G is a Group 13 element, and Y is each independently a substituted or unsubstituted alkyl group having 1 to 20 carbon atoms or a substituted or unsubstituted aryl group having 6 to 20 carbon atoms, and here, if the alkyl group or aryl group is substituted, the substituent is a halogen group, a hydrocarbyl group having 1 to 20 carbon atoms, an alkoxy group having 1 to 20 carbon atoms, or an aryloxy group having 6 to 20 carbon atoms.

According to an embodiment of the present invention, the catalyst composition may be prepared by a plurality of methods. As a specific example, first, the catalyst composition may be prepared by contacting the organic chromium compound with a compound represented by Formula 10 or Formula 11 above. Second, the catalyst composition may be prepared by contacting the organic chromium compound with a compound represented by Formula 10 or Formula 11 above to obtain a mixture, and adding a compound represented by Formula 12 or Formula 13 above to the mixture. Third, the catalyst composition may be prepared by contacting the organic chromium compound with a compound represented by Formula 12 or Formula 13 above. Fourth, the catalyst composition may be prepared by contacting the organic chromium compound with a compound represented by Formula 12 or Formula 13 above to obtain a mixture, and adding a compound represented by Formula 10 or Formula 11 above to the mixture. Fifth, the catalyst composition may be prepared by contacting the chromium source with a compound represented by Formula 12 or Formula 13 above to obtain a reactant, and contacting the reactant with the ligand compound.

According to an embodiment of the present invention, in the case of the first method or the third method among the above methods for preparing the catalyst composition, the molar ratio of the compound represented by Formula 10 or Formula 11 above with respect to the organic chromium compound may be 1:2 to 5,000 respectively, as a specific example, may be 1:100 to 3,000, and as a more specific example, may be 1:300 to 1,500, and within the above range, alkylation of the organic chromium compound may be completely achieved, so that the activity of the catalyst composition may be improved, and degradation in the activation of the alkylated organic chromium compound due to a side reaction with the remaining alkylating agent may be prevented, and at the same time, economic feasibility and purity of prepared linear alpha-olefins may be improved.

According to an embodiment of the present invention, in the case of the second method among the above methods for preparing the catalyst composition, the molar ratio of the compound represented by Formula 12 or Formula 13 above with respect to the organic chromium compound may be 1:1 to 500 respectively, as a specific example, may be 1:1 to 50, and as a more specific example, may be 1:1 to 25, and within the above range, the amount of an activator is sufficient, so that activation of a metal compound may be completely achieved, thereby improving the activity of the catalyst composition, and the remaining activator is reduced to the minimum, so that economic feasibility and purity of prepared linear alpha-olefins may be improved.

According to an embodiment of the present invention, the compound represented by Formula 10 above may be alkylaluminoxane, may be methylaluminoxane, ethylaluminoxane, isobutylaluminoxane, butylaluminoxane, or the like as a specific example, and may be methylaluminoxane as a more specific example.

According to an embodiment of the present invention, the compound represented by Formula 11 above may be trialkyl aluminum, dialkyl aluminum halide, alkyl aluminum dihalide, dialkyl aluminum hydride, alkyl aluminum dihydride, trialkyl boron, or the like. As a specific example, the compound represented by Formula 6 above may be trialkyl aluminum such as trimethyl aluminum, triethyl aluminum, triisobutyl aluminum, tripropyl aluminum, tributyl aluminum, triisopropyl aluminum, tri-s-butyl aluminum, tricyclopentyl aluminum, tripentyl aluminum, triisopentyl aluminum, trihexyl aluminum, trioctyl aluminum, ethyldimethyl aluminum, methyldiethyl aluminum, triphenyl aluminum, and tri-p-tolyl aluminum; dialkyl aluminum halide such as diethylaluminum chloride; dialkyl aluminum hydride such as diethyl aluminum hydride, di-n-propyl aluminum hydride, diisopropyl aluminum hydride, di-n-butyl aluminum hydride, dibutyl aluminum hydride, diisobutyl aluminum hydride (DIBAH), di-n -octyl aluminum hydride, diphenyl aluminum hydride, di-p-tolyl aluminum hydride, dibenzyl aluminum hydride, phenylethyl aluminum hydride, phenyl-n-propyl aluminum hydride, phenylisopropyl aluminum hydride, phenyl-n-butyl aluminum hydride, phenylisobutyl aluminum hydride, phenyl-n-octyl aluminum hydride, p-tolylethyl aluminum hydride, p-tolyl-n-propyl aluminum hydride, p-tolylisopropyl aluminum hydride. p-tolyl-n-butyl aluminum hydride, p-tolylisobutyl aluminum hydride, p-tolyl-n-octyl aluminum hydride, benzylethyl aluminum hydride, benzyl-n-propyl aluminum hydride, benzylisobutyl aluminum hydride, benzyl-n-butyl aluminum hydride, benzylisobutyl aluminum hydride, and benzyl-n-octyl aluminum hydride; alkyl aluminum dihydride such as n-propyl aluminum dihydride, isopropyl aluminum dihydride, n-butyl aluminum dihydride, isobutyl aluminum dihydride, and n-octyl aluminum dihydride; trialkyl boron such as trimethyl boron, triethyl boron, triisobutyl boron, tripropyl boron, and tributyl boron, or the like.

According to an embodiment of the present invention, the compound represented by Formula 12 or Formula 13 may be trimethylammonium tetraphenylborate, triethylammonium tetraphenylborate, tripropylammonium tetraphenylborate, tributylammonium tetraphenylborate, methyl dioctadecylammonium tetraphenylborate, N,N-dimethylanilinium tetraphenylborate, N,N-diethylanilinium tetraphenylborate, N,N-Dioctadecylanilinium tetraphenylborate, trimethylammonium tetra (p-tolyl)borate, triethylammonium tetra (p-tolyl)borate, tripropylammonium tetra(p-tolyl) Borate, tributylammonium tetra(p-tolyl)borate, methyl dioctadecylammonium tetra(p-tolyl)borate, N,N-dimethylanilinium tetra(p-tolyl)borate, N,N-diethylanilinium tetra(p-tolyl)borate, N,N-dioctadecylanilinium tetra(p-tolyl)borate, trimethylammonium tetra (o,p-dimethylphenyl) borate, triethylammonium tetra(o,p-dimethylphenyl) borate, tripropylammonium tetra(o,p-dimethylphenyl) borate, tributylammonium tetra(o,p-dimethylphenyl) borate, methyl dioctadecylammonium tetra(o,p-dimethylphenyl)borate, N,N-dimethylanilinium tetra(o,p-dimethylphenyl)borate, N,N-diethylanilinium tetra(o,p-dimethylphenyl)borate, N,N-dioctadecylanilinium tetra(o,p-dimethylphenyl) borate, trimethylammonium tetrakis(p-trifluoromethylphenyl) borate, triethylammonium tetrakis(p-trifluoromethylphenyl) borate, tripropylammonium tetrakis(p-trifluoromethylphenyl) borate, tributylammonium tetrakis(p-trifluoromethylphenyl)borate, N,N-dimethylanilinium tetrakis(p-trifluoromethylphenyl)borate, N,N-diethylanilinium tetrakis(p-trifluoromethylphenyl)borate, N,N-dioctadecylanilinium tetrakis(p-trifluoromethylphenyl)borate, trimethylammonium tetrakis(pentafluorophenyl)borate, triethylammonium tetrakis(pentafluorophenyl)borate, tripropylammonium tetrakis(pentafluorophenyl)borate, tributylammonium tetrakis(pentafluorophenyl)borate, methyl dioctadecylammonium tetrakis(pentafluorophenyl)borate, N,N-dimethylanilinium tetrakis(pentafluorophenyl)norate, N,N-diethylanilinium tetrakis(pentafluorophenyl)borate, N,N-dioctadecylanilinium tetrakis(pentafluorophenyl)borate, trimethylphosphonium tetraphenylborate, triethylphosphonium tetraphenylborate, tripropylphosphonium tetraphenylborate, tributylphosphonium tetraphenylborate, trimethylcarbonium tetraphenylborate, triethylcarbonium tetraphenylborate, tripropylcarbonium tetraphenylborate, tributylcarbonium tetraphenylborate , trimethylammonium tetraphenylaluminate, triethylammonium tetraphenylaluminate, tripropylammonium tetraphenylaluminate, tributylammonium tetraphenylaluminate, trimethylammonium tetra(p-tolyl) aluminate, triethylammonium tetra(p-tolyl)aluminate, tripropylammonium tetra(p-tolyl) aluminate, tributylammonium tetra(p-tolyl)aluminate, or the like.

According to an embodiment of the present invention, the content ratio of components for forming the catalyst composition may be determined in consideration of catalyst activity and selectivity to a linear alpha-olefin. As a specific example, when the catalyst composition is a three-component catalyst composition, the molar ratio of the diphosphino aminyl moiety of the ligand compound:chromium source:co-catalyst may be controlled to about 1:1:1 to about 10:1:10,000, or to about 1:1:100 to about 5:1:3,000. In addition, when the catalyst composition is a two-component catalyst composition, the molar ratio of the diphosphino aminyl moiety of the ligand compound:co-catalyst may be controlled to about 1:1 to about 1:10,000, about 1:1 to about 1:5,000, or about 1:1 to about 1:3,000.

According to an embodiment of the present invention, when preparing the catalyst composition, as a reaction solvent, a hydrocarbon-based solvent such as pentane, hexane, and heptane, or an aromatic solvent such as benzene and toluene may be used.

According to an embodiment of the present invention, the components for forming the catalyst composition may be added simultaneously or in an arbitrary order in the presence or absence of a suitable solvent and a monomer, and act as an active catalyst composition. At this time, the suitable solvent may be heptane, toluene, cyclohexane, methylcyclohexane, 1-hexene, 1-octene, diethylether, tetrahydrofuran, acetonitrile, dichloromethane, chloroform, chlorobenzene, methanol, acetone, or the like.

According to an embodiment of the present invention, the organic chromium compound and the co-catalyst may be used in the form of being supported on a carrier, and at this time, the carrier may be silica or alumina.

According to an embodiment of the present invention, the catalyst composition may further include a carrier. As a specific example, the ligand compound, represented by Formula 1 above may be applied to an ethylene oligomerization reaction in the form of being supported on a carrier. The carrier may be a metal, a metal salt, or a metal oxide applied to a carrier catalyst, and as a specific example, the carrier may be silica, silica-alumina, silica-magnesia, or the like, and may include an oxide, carbonate, sulfate, or nitrate component of a metal, such as Na₂O, K₂CO₃, BaSO₄, and Mg(NO₃)₂.

According to an embodiment of the present invention, the catalyst composition may be used for a trimerization or tetramerization reaction of ethylene, and may produce 1-hexene or 1-octene due to the high selectivity described above.

### Ethylene oligomerization method

The present invention provides a linear alpha-olefin preparation method as a method for oligomerizing ethylene, wherein the method includes oligomerizing ethylene in the presence of the catalytic composition S10.

In the present invention, "oligomerization" refers to polymerization of an olefin. Depending on the number of olefins polymerized, the "oligomerization" is called trimerization or tetramerization, which is collectively referred to as multimerization. Particularly, in the present specification, the "oligomerization" may refer to selectively preparing 1-hexene and 1-octene, which are main co-monomers of LLDPE, from ethylene.

According to an embodiment of the present invention, the oligomerization reaction of ethylene may be a trimerization or tetramerization reaction of ethylene, and as a result of the reaction, 1-hexene or 1-octene is formed as reaction products, so that the linear alpha-olefin may be 1-hexene, 1-octene, or a mixture thereof.

According to an embodiment of the present invention, the oligomerization method of ethylene may be performed by using ethylene as a raw material and applying the previously described catalyst composition, a typical apparatus, and a contact technique. As a specific example, the oligomerization reaction of ethylene may be performed, in the presence or absence of an inert solvent, as a homogeneous liquid-phase reaction, a slurry reaction in which some or all of the catalyst composition is in an undissolved form, a bulk-phase reaction in which an alpha-olefin, which is a product, serves as a main medium, or a gas-phase reaction.

According to an embodiment of the present invention, the oligomerization reaction of ethylene may be performed in an inert solvent. As a specific example, the inert solvent may be benzene, toluene, xylene, cumene, chlorobenzene, dichlorobenzene, heptane, cyclohexane, methylcyclohexane, methylcyclopentane, n-hexane, 1-hexene, 1-octene, 2,2,4-trimethylpentane, or the like.

According to an embodiment of the present invention, the oligomerization reaction of ethylene may be performed at a temperature of about 0 °C to about 200 °C, about 0 °C to about 150 °C, about 30 °C to about 100 °C, or about 50 °C to about 100 °C. In addition, the reaction may be performed under a pressure of about 15 psig to about 3000 psig, about 15 psig to about 1500 psig, or about 15 psig to about 1,000 psig.

Hereinafter, embodiments of the present invention will be described in detail so that those skilled in the art may easily carry out the present invention. However, the present invention may be embodied in many different forms, and is not limited to the embodiments set forth herein.

### Synthesis Examples and Comparative Examples

### Synthesis Example 1 to 66: Synthesis of ligand compound represented by Formula 2-1 to Formula 2-66

20 mmol (2 eq) of p-bromo-(tri-n-butylsilyl)-C₆H₄ was dissolved in 20 ml of tetrahydrofuran, and then cooled to - 78 °C. While maintaining the temperature, 20 mmol (2 eq) of n-butyllithium was added dropwise thereto, and then stirred for 3 hours. Subsequently, 10 mmol (1 eq) of dichloro(diethylamino)phosphine dissolved in 10 mL of tetrahydrofuran was added dropwise thereto, and then the temperature was raised to room temperature, and the mixture was stirred overnight. Thereafter, the solvent was removed using vacuum, and the obtained intermediate was dissolved in 30 mL of hexane, and then HCl (in ether, 2 eq) was added thereto, stirred for 15 minutes, and filtered. The filtrate was dried in a vacuum state to obtain (p-(tri-n-butylsilyl)-C₆H₄)₂PCl. 11 mmol (2.2 eq) of R⁵-NH₂ and 10 ml of dichloromethane were introduced into a well-dried flask under a nitrogen atmosphere and stirred. 5 mmol (1 eq) of (p-(tri-n-butylsilyl)-C₆H₄)₂PCl was diluted in 10 ml of dichloromethane and then slowly introduced into the flask. After the reaction was completed by stirring for 4 hours, the precipitated solids were removed through a filter, the solvent was removed under reduced pressure, and then (p-(tri-n-butylsilyl)-C₆H₄)₂P-NHR⁵ was obtained through column chromatography.

5 mmol (1 eq) of (p-(tri-n-butylsilyl)-C₆H₄)PCl₂ was dissolved in 10 ml of ether, and then cooled to 0 °C. Subsequently, 10 mmol (2 eq) of HNEt₂ dissolved in 10 ml of ether was slowly introduced thereto, and stirred overnight. The solids were removed through a filter, and 1 eq of ArMgBr prepared from Ar and Mg pieces (Mg turning) was slowly introduced thereto, and then stirred at room temperature overnight. Thereafter, HCl (in ether, 2 eq) was introduced thereto and stirred for 30 minutes at room temperature. The solids were removed through a filter and the pressure was reduced to obtain (p-(tri-n-butylsilyl)-C₆H₄)ArPCl. 5 mmol (1 eq) of (p-(tri-n-butylsilyl)-C₆H₄)ArPCl was dissolved in 10 ml of dichloromethane, and then 15 mmol (3 eq) of triethylamine was added thereto. Subsequently, 5 mmol (1 eq) of (p-(tri-n-butylsilyl)-C₆H₄)₂P-NHR⁵ was dissolved in dichloromethane and then slowly introduced thereto, and the mixture was stirred at room temperature overnight. The solvent was removed using vacuum, dissolved in hexane, and ligand compounds represented by Formula 2-1 to Formula 2-66 were obtained by column chromatography.

R⁵ and Ar, which are substituents of the reactants used in Synthesis Example 1 to Synthesis Example 66, are each shown in Table 1 below, and the structure of the ligand compound prepared in each Synthetic Example and ¹H NMR data are described below.

**[Table 1]**

| Classific ation | R⁵ | Ar(*binding position) |
|---|---|---|
| Synthesis Example 1 | methyl(-CH₃) | 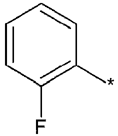 |
| Synthesis Example 2 | methyl(-CH₃) | 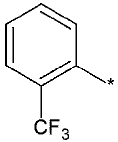 |
| Synthesis Example 3 | methyl(-CH₃) | 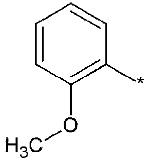 |
| Synthesis Example 4 | methyl(-CH₃) | 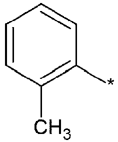 |
| Synthesis Example 5 | methyl(-CH₃) | 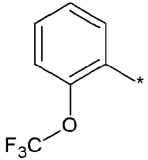 |
| Synthesis Example 6 | methyl(-CH₃) | 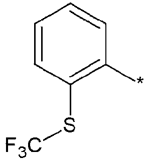 |
| Synthesis Example 7 | methyl(-CH₃) | 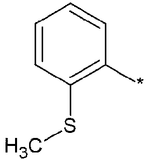 |
| Synthesis Example 8 | methyl(-CH₃) | 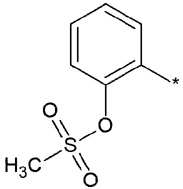 |
| Synthesis Example 9 | methyl(-CH₃) | 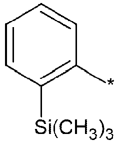 |
| Synthesis Example 10 | methyl(-CH₃) | 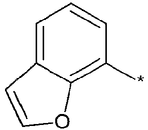 |
| Synthesis Example 11 | methyl(-CH₃) | 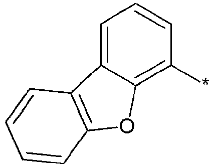 |
| Synthesis Example 12 | n-butyl(-C₄H₉) | 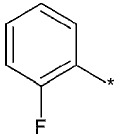 |
| Synthesis Example 13 | n-butyl(-C₄H₉) | 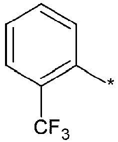 |
| Synthesis Example 14 | n-butyl(-C₄H₉) | 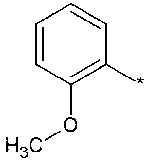 |
| Synthesis Example 15 | n-butyl(-C₄H₉) | 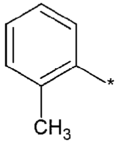 |
| Synthesis Example 16 | n-butyl(-C₄H₉) | 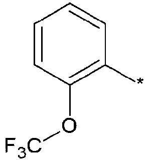 |
| Synthesis Example 17 | n-butyl(-C₄H₉) | 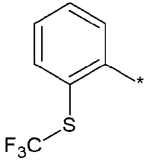 |
| Synthesis Example 18 | n-butyl(-C₄H₉) | 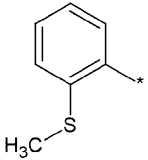 |
| Synthesis Example 19 | n-butyl(-C₄H₉) | 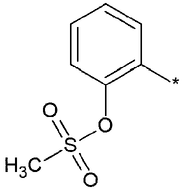 |
| Synthesis Example 20 | n-butyl(-C₄H₉) | 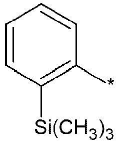 |
| Synthesis Example 21 | n-butyl(-C₄H₉) | 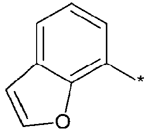 |
| Synthesis Example 22 | n-butyl(-C₄H₉) | 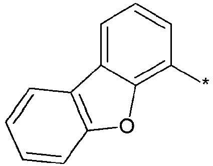 |
| Synthesis Example 23 | iso-propyl (-CH(CH₃)₂) | 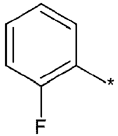 |
| Synthesis Example 24 | iso-propyl (-CH(CH₃)₂) | 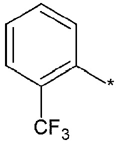 |
| Synthesis Example 25 | iso-propyl(-CH(CH₃)₂) | 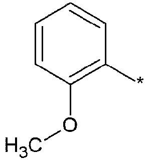 |
| Synthesis Example 26 | iso-propyl(-CH(CH₃)₂) | 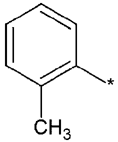 |
| Synthesis Example 27 | iso-propyl(-CH(CH₃)₂) | 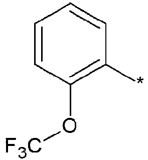 |
| Synthesis Example 28 | iso-propyl(-CH(CH₃)₂) | 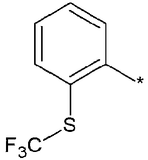 |
| Synthesis Example 29 | iso-propyl(-CH(CH₃)₂) | 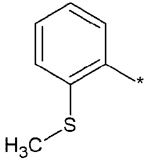 |
| Synthesis Example 30 | iso-propyl(-CH(CH₃)₂) | 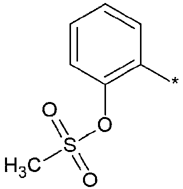 |
| Synthesis Example 31 | iso-propyl(-CH(CH₃)₂) | 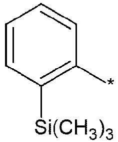 |
| Synthesis Example 32 | iso-propyl(-CH(CH₃)₂) | 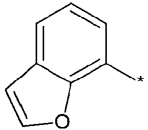 |
| Synthesis Example 33 | iso-propyl(-CH(CH₃)₂) | 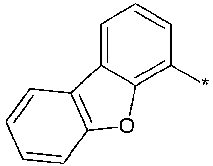 |
| Synthesis Example 34 | cyclohexyl(-C₆H₁₁) | 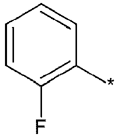 |
| Synthesis Example 35 | cyclohexyl(-C₆H₁₁) | 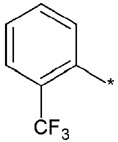 |
| Synthesis Example 36 | cyclohexyl(-C₆H₁₁) | 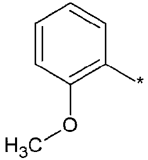 |
| Synthesis Example 37 | cyclohexyl(-C₆H₁₁) | 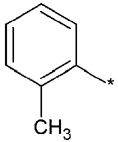 |
| Synthesis Example 38 | cyclohexyl(-C₆H₁₁) | 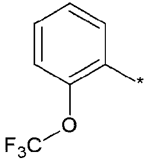 |
| Synthesis Example 39 | cyclohexyl(-C₆H₁₁) | 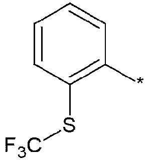 |
| Synthesis Example 40 | cyclohexyl(-C₆H₁₁) | 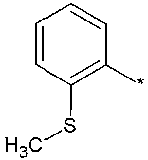 |
| Synthesis Example 41 | cyclohexyl(-C₆H₁₁) | 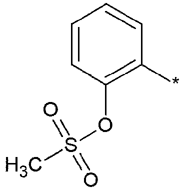 |
| Synthesis Example 42 | cyclohexyl(-C₆H₁₁) | 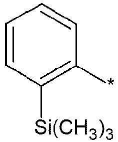 |
| Synthesis Example 43 | cyclohexyl(-C₆H₁₁) | 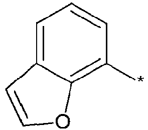 |
| Synthesis Example 44 | cyclohexyl(-C₆H₁₁) | 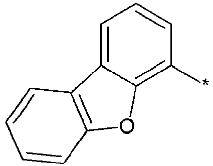 |
| Synthesis Example 45 | benzyl(-CH₂-Ph) | 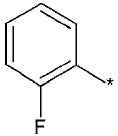 |
| Synthesis Example 46 | benzyl(-CH₂-Ph) | 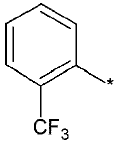 |
| Synthesis Example 47 | benzyl(-CH₂-Ph) | 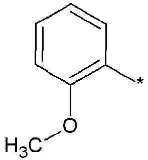 |
| Synthesis Example 48 | benzyl(-CH₂-Ph) | 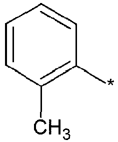 |
| Synthesis Example 49 | benzyl(-CH₂-Ph) | 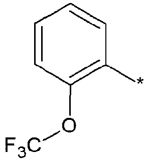 |
| Synthesis Example 50 | benzyl(-CH₂-Ph) | 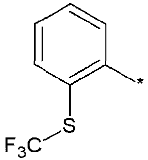 |
| Synthesis Example 51 | benzyl(-CH₂-Ph) | 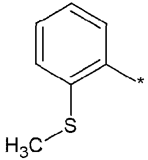 |
| Synthesis Example 52 | benzyl(-CH₂-Ph) | 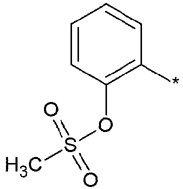 |
| Synthesis Example 53 | benzyl(-CH₂-Ph) | 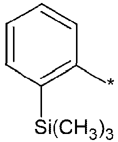 |
| Synthesis Example 54 | benzyl(-CH₂-Ph) | 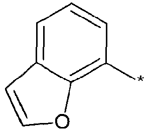 |
| Synthesis Example 55 | benzyl(-CH₂-Ph) | 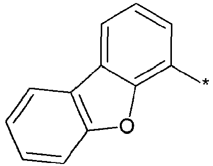 |
| Synthesis Example 56 | phenyl (-Ph) | 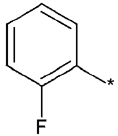 |
| Synthesis Example 57 | phenyl (-Ph) | 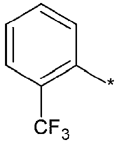 |
| Synthesis Example 58 | phenyl (-Ph) | 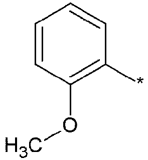 |
| Synthesis Example 59 | phenyl (-Ph) | 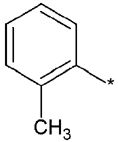 |
| Synthesis Example 60 | phenyl (-Ph) | 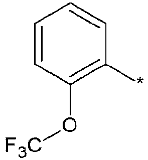 |
| Synthesis Example 61 | phenyl (-Ph) | 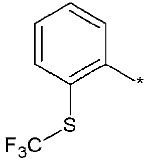 |
| Synthesis Example 62 | phenyl (-Ph) | 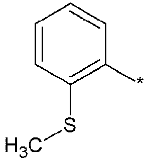 |
| Synthesis Example 63 | phenyl (-Ph) | 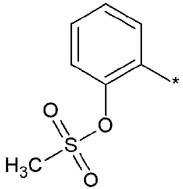 |
| Synthesis Example 64 | phenyl (-Ph) | 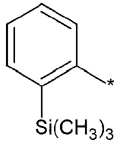 |
| Synthesis Example 65 | phenyl (-Ph) | 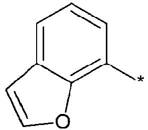 |
| Synthesis Example 66 | phenyl (-Ph) | 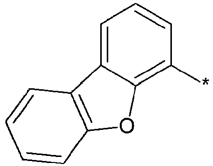 |

### Synthesis Example 1 (Formula 2-1)

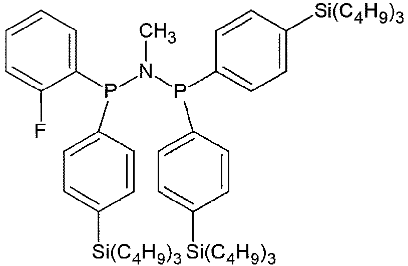

### N-(bis(4-(tributylsilyl)phenyl)phosphaneyl)-1-(2-fluorophenyl)-N-methyl-1-(4-(tributylsilyl)phenyl)phosphanamine

¹H NMR(500 MHz, CDCl₃): δ 7.48 (m, 6H), 7.39(m, 1H), 7.27(m, 1H), 7.14(m, 7H), 7.12(m, 1H), 2.57(m, 3H), 1.46(m, 36H), 1.23 (m, 18H), 0.97(m, 27H)

### Synthesis Example 2 (Formula 2-2)

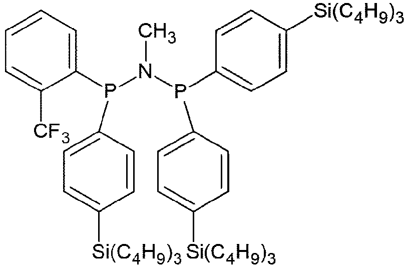

### N-(bis(4-(tributylsilyl)phenyl)phosphaneyl)-N-methyl-1-(4-(tributylsilyl)phenyl)-1-(2-(trifluoromethyl)phenyl)phosphanamine

¹H NMR(500 MHz, CDCl₃): δ 7.52 (m, 1H), 7.48 (m, 1H), 7.44 (m, 6H), 7.17(m, 1H), 7.16(m, 6H), 7.12(m, 1H), 2.54 (m, 3H), 1.49(m, 18H), 1.39(m, 18H), 1.31(m, 18H), 0.95(m, 27H)

### Synthesis Example 3 (Formula 2-3)

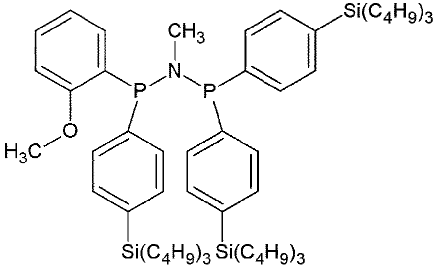

### N-(bis(4-(tributylsilyl)phenyl)phosphaneyl)-1-(2-methoxyphenyl)-N-methyl-1-(4-(tributylsilyl)phenyl)phosphanamine

¹H NMR(500 MHz, CDCl₃): δ 7.45(m, 6H), 7.39(m, 1H), 7.15(m, 1H), 7.14(m, 6H), 7.12(m, 1H), 6.98(m, 1H), 3.97 (m, 3H), 2.55(m, 3H), 1.51(m, 18H), 1.42(m, 18H), 1.27(m, 18H), 0.96(m, 27H)

### Synthesis Example 4 (Formula 2-4)

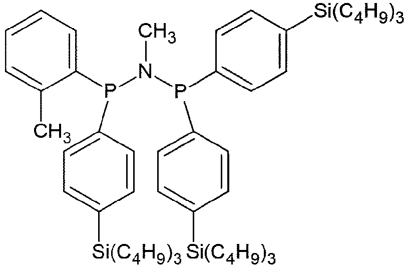

### N-(bis(4-(tributylsilyl)phenyl)phosphaneyl)-N-methyl-1-(o-tolyl)-1-(4-(tributylsilyl)phenyl)phosphanamine

¹H NMR (500 MHz, CDCl₃): δ 7.46 (m, 6H), 7.36 (m, 1H), 7.24(m, 1H), 7.23(m, 1H), 7.13(m, 6H), 7.10(m, 1H), 2.55(m, 3H), 2.38(m, 3H), 1.45(m, 18H), 1.44(m, 18H), 1.28(m, 18H), 0.96(m, 27H)

### Synthesis Example 5 (Formula 2-5)

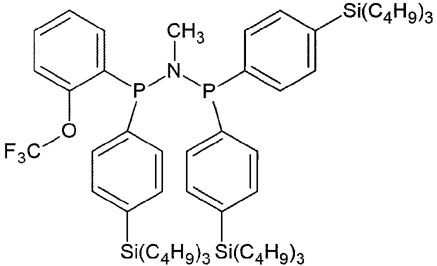

### N-(bis(4-(tributylsilyl)phenyl)phosphaneyl)-N-methyl-1-(4-(tributylsilyl)phenyl)-1-(2-(trifluoromethoxy)phenyl)phosphanamine

¹H NMR(500 MHz, CDCl₃): δ 7.47(m, 6H), 7.38(m, 1H), 7.14(m, 7H), 7.13(m, 1H), 6.97(m, 1H), 2.49(m, 3H), 1.48(m, 18H), 1.38(m, 18H), 1.30(m, 18H), 1.00(m, 27H)

### Synthesis Example 6 (Formula 2-6)

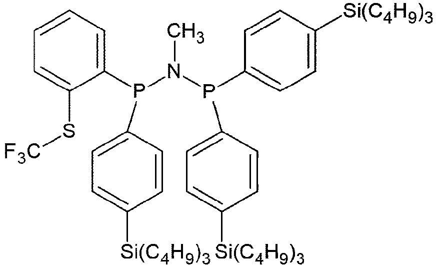

### N-(bis(4-(tributylsilyl)phenyl)phosphaneyl)-N-methyl-1-(4-(tributylsilyl)phenyl)-1-(2-((trifluoromethyl)thio)phenyl)phosphanamine

¹H NMR(500 MHz, CDCl₃): δ 7.41(m, 6H), 7.26(m, 1H), 7.23(m, 2H), 7.16(m, 6H), 7.03(m, 1H), 2.57(m, 3H), 1.46(m, 18H), 1.38(m, 18H), 1.28(m, 18H), 0.99(m, 27H)

### Synthesis Example 7 (Formula 2-7)

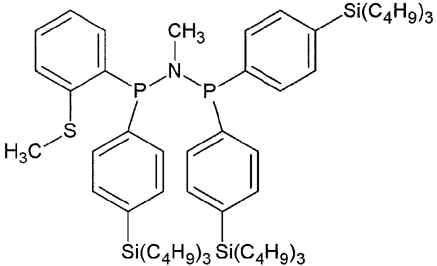

### N-(bis(4-(tributylsilyl)phenyl)phosphaneyl)-N-methyl-1-(2-(methylthio)phenyl)- 1-(4-(tributylsilyl)phenyl)phosphanamine

¹H NMR(500 MHz, CDCl₃): δ 7.44(m, 6H), 7.28(m, 2H), 7.25(m, 1H), 7.17(m, 6H), 7.07(m, 1H), 2.53 (m, 3H), 2.47 (m, 3H), 1.45(m, 18H), 1.37(m, 18H), 1.25(m, 18H), 0.97 (m, 27H)

### Synthesis Example 8 (Formula 2-8)

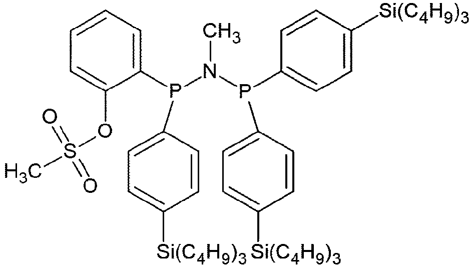

### 2-(((bis(4-(tributylsilyl) phenyl) phosphaneyl) (methyl) amino) (4-tributylsilyl)phenyl)phosphaneyl)phenyl methanesulfonate

¹H NMR(500 MHz, CDCl₃): δ 7.54 (m, 1H), 7.44 (m, 6H), 7.43(m, 1H), 7.36(m, 1H), 7.25(m, 1H), 7.11(m, 6H), 3.55(m, 3H), 2.57(m, 3H), 1.53(m, 18H), 1.46(m, 18H), 1.29(m, 18H), 0.94 (m, 27H)

### Synthesis Example 9 (Formula 2-9)

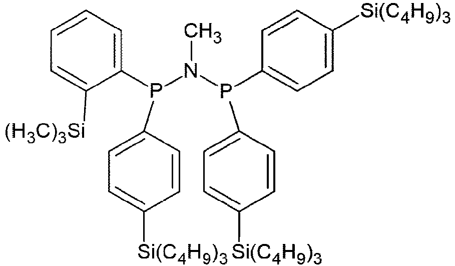

### N-(bis(4-(tributylsilyl)phenyl)phosphaneyl)-N-methyl-1-(4-(tributylsilyl)phenyl)-1-(2-(trimethylsilyl)phenyl)phosphanamine

¹H NMR (500 MHz, CDCl₃): δ 7.45 (m, 8H), 7.33 (m, 1H), 7.12(m, 7H), 2.48(m, 3H), 1.49(m, 18H), 1.40(m, 18H), 1.24 (m, 18H), 0.92(m, 27H), 0.35(m, 9H)

### Synthesis Example 10 (Formula 2-10)

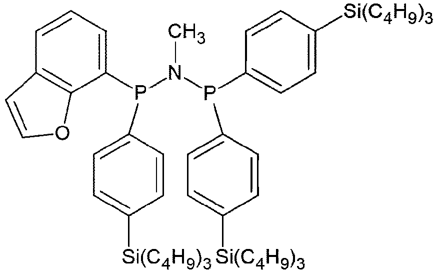

### 1-(benzofuran-7-yl)-N-(bis(4-(tributylsilyl)phenyl)phosphaneyl)-N-methyl-1-(4-(tributylsilyl)phenyl)phosphanamine

¹H NMR(500 MHz, CDCl₃): δ 7.66(m, 1H), 7.46(m, 6H), 7.35(m, 1H), 7.32(m, 1H), 7.15(m, 6H), 6.82(m, 1H), 2.54 (m, 3H), 1.50(m, 18H), 1.44(m, 18H), 1.28(m, 18H), 1.01 (m, 27H)

### Synthesis Example 11 (Formula 2-11)

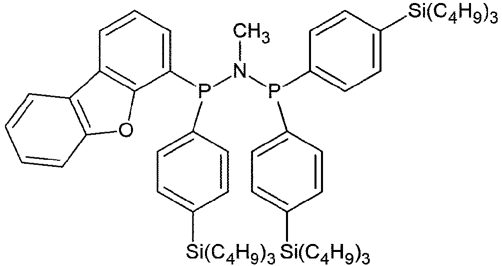

### N-(bis(4-(tributylsilyl)phenyl)phosphaneyl)-1-(dibenzo[b,d]furan-4-yl)-N-methyl-1-(4-(tributylsilyl)phenyl)phosphanamine

¹H NMR(500 MHz, CDCl₃): δ 7.99(m, 2H), 7.55(m, 1H), 7.43(m, 6H), 7.41(m, 1H), 7.33(m, 2H), 7.28(m, 1H), 7.19(m, 6H), 2.52(m, 3H), 1.52(m, 18H), 1.41 (m, 18H), 1.33 (m, 18H), 1.00(m, 27H)

### Synthesis Example 12 (Formula 2-12)

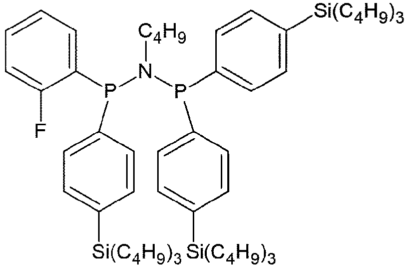

### N-(bis(4-(tributylsilyl)phenyl)phosphaneyl)-N-butyl-1-(2-fluorophenyl)-1-(4-(tributylsilyl)phenyl)phosphanamine

¹H NMR(500 MHz, CDCl₃): δ 7.50(m, 6H), 7.35(m, 1H), 7.25(m, 1H), 7.17(m, 7H), 7.11(m, 1H), 2.71 (m, 2H), 1.49(m, 18H), 1.45(m, 2H), 1.42(m, 18H), 1.41(m, 2H), 1.25(m, 18H), 1.00(m, 27H), 0.94 (m, 3H)

### Synthesis Example 13 (Formula 2-13)

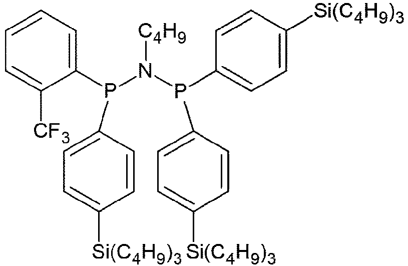

### N-(bis(4-(tributylsilyl)phenyl)phosphaneyl)-N-butyl-1-(4-(tributylsilyl)phenyl)-1-(2-(trifluoromethyl)phenyl)phosphanamine

¹H NMR(500 MHz, CDCl₃): δ 7.44 (m, 8H), 7.23 (m, 1H), 7.14(m, 6H), 7.11(m, 1H), 2.67(m, 2H), 1.52(m, 2H), 1.46(m, 18H), 1.41(m, 18H), 1.33(m, 2H), 1.30(m, 18H), 0.93(m, 27H), 0.92(m, 3H)

### Synthesis Example 14 (Formula 2-14)

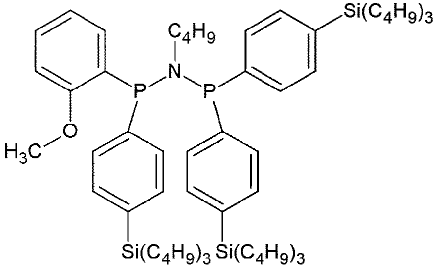

### N-(bis(4-(tributylsilyl)phenyl)phosphaneyl)-N-butyl-1-(2-methoxyphenyl)-1-(4-(tributylsilyl)phenyl)phosphanamine

¹H NMR (500 MHz, CDCl₃): δ 7.43 (m, 6H), 7.38(m, 1H), 7.18(m, 1H), 7.13(m, 7H), 6.97(m, 1H), 3.93 (m, 3H), 2.65(m, 2H), 1.50(m, 2H), 1.49(m, 18H), 1.42(m, 2H), 1.41(m, 18H), 1.27(m, 18H), 0.98 (m, 27H), 0.95(m, 3H)

### Synthesis Example 15 (Formula 2-15)

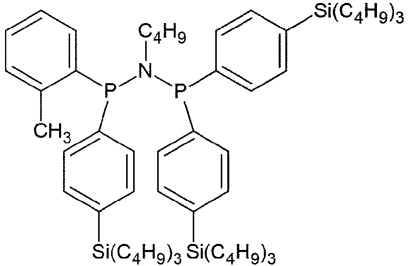

### N-(bis(4-(tributylsilyl)phenyl)phosphaneyl)-N-butyl-1-(o-tolyl)-1-(4-(tributylsilyl)phenyl)phosphanamine

¹H NMR(500 MHz, CDCl₃): δ 7.45 (m, 6H), 7.36 (m, 1H), 7.22(m, 1H), 7.19(m, 1H), 7.17(m, 6H), 7.16(m, 1H), 2.67(m, 2H), 2.32(m, 3H), 1.48(m, 18H), 1.47(m, 2H), 1.44(m, 18H), 1.33(m, 18H), 1.32(m, 2H), 0.99(m, 3H), 0.95(m, 27H)

### Synthesis Example 16 (Formula 2-16)

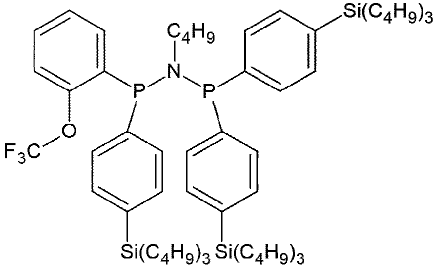

### N-(bis(4-(tributylsilyl)phenyl)phosphaneyl)-N-butyl-1-(4-(tributylsilyl)phenyl)-1-(2-(trifluoromethoxy)phenyl)phosphanamine

¹H NMR(500 MHz, CDCl₃): δ 7.46(m, 1H), 7.42 (m, 6H), 7.19(m, 1H), 7.15(m, 6H), 7.14(m, 1H), 6.98(m, 1H), 2.66(m, 2H), 1.48(m, 2H), 1.45(m, 18H), 1.36(m, 18H), 1.35(m, 2H), 1.24(m, 18H), 0.99(m, 3H), 0.96(m, 27H)

### Synthesis Example 17 (Formula 2-17)

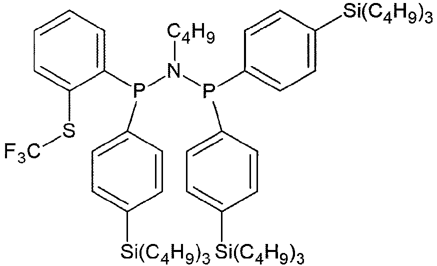

### N-(bis(4-(tributylsilyl)phenyl)phosphaneyl)-N-butyl-1-(4-(tributylsilyl)phenyl)-1-(2-((trifluoromethyl)thio)phenyl)phosphanamine

¹H NMR(500 MHz, CDCl₃): δ 7.49(m, 6H), 7.24(m, 1H), 7.22(m, 2H), 7.13(m, 6H), 7.03(m, 1H), 2.73 (m, 2H), 1.50(m, 2H), 1.43(m, 18H), 1.41(m, 18H), 1.38(m, 2H), 1.31(m, 18H), 0.95(m, 27H), 0.94 (m, 3H)

### Synthesis Example 18 (Formula 2-18)

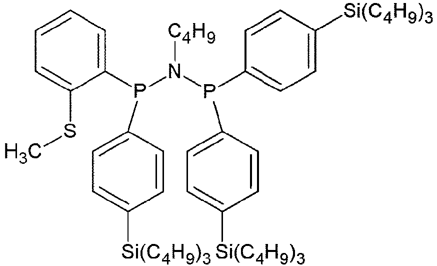

### N-(bis(4-(tributylsilyl)phenyl)phosphaneyl)-N-butyl-1-(2-(methylthio)phenyl)-1-(4-(tributylsilyl)phenyl)phosphanamine

¹H NMR(500 MHz, CDCl₃): δ 7.45(m, 6H), 7.21(m, 2H), 7.17(m, 1H), 7.14(m, 6H), 7.04(m, 1H), 2.69(m, 2H), 2.47 (m, 3H), 1.45(m, 2H), 1.43(m, 18H), 1.38(m, 18H), 1.33(m, 2H), 1.23(m, 18H), 1.02(m, 27H), 0.93(m, 3H)

### Synthesis Example 19 (Formula 2-19)

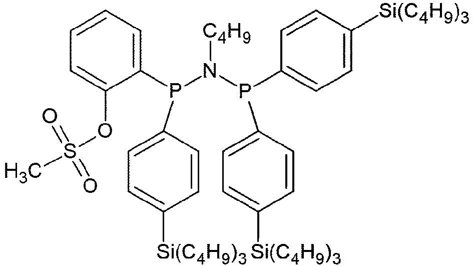

### 2-(((bis(4-(tributylsilyl)phenyl)phosphaneyl)(butyl)amino)(4-tributylsilyl)phenyl)phosphaneyl)phenyl methanesulfonate

¹H NMR(500 MHz, CDCl₃): δ 7.51 (m, 1H), 7.47 (m, 1H), 7.43 (m, 6H), 7.35(m, 1H), 7.23(m, 1H), 7.17(m, 6H), 3.62 (m, 3H), 2.65(m, 2H), 1.53(m, 2H), 1.45(m, 18H), 1.41(m, 18H), 1.39(m, 2H), 1.31(m, 18H), 0.94(m, 27H), 0.92 (m, 3H)

### Synthesis Example 20 (Formula 2-20)

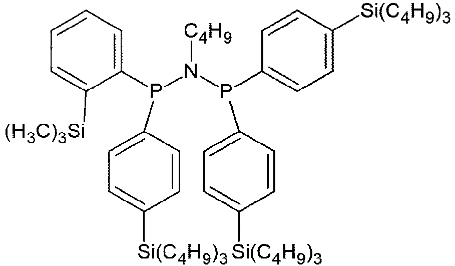

### N-(bis(4-(tributylsilyl)phenyl)phosphaneyl)-N-butyl-1-(4-(tributylsilyl)phenyl)-1-(2-(trimethylsilyl)phenyl)phosphanamine

¹H NMR(500 MHz, CDCl₃): δ 7.43 (m, 8H), 7.26(m, 1H), 7.16(m, 7H), 2.64(m, 2H), 1.45(m, 36H), 1.44(m, 2H), 1.37(m, 2H), 1.30(m, 18H), 1.01(m, 27H), 0.93(m, 3H), 0.28(m, 9H)

### Synthesis Example 21 (Formula 2-21)

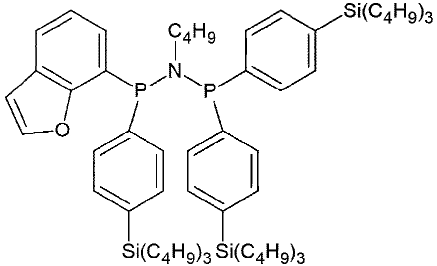

### 1-(benzofuran-7-yl)-N-(bis(4-(tributylsilyl)phenyl)phosphaneyl)-N-butyl-1-(4-(tributylsilyl)phenyl)phosphanamine

¹H NMR(500 MHz, CDCl₃): δ 7.68(m, 2H), 7.41(m, 6H), 7.36(m, 1H), 7.28(m, 1H), 7.19(m, 6H), 6.84(m, 1H), 2.63(m, 2H), 1.50(m, 2H), 1.48(m, 18H), 1.39(m, 2H), 1.38(m, 18H), 1.29(m, 18H), 1.00(m, 27H), 0.91(m, 3H)

### Synthesis Example 22 (Formula 2-22)

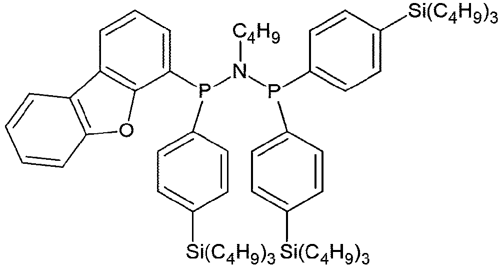

### N-(bis(4-(tributylsilyl)phenyl)phosphaneyl)-N-butyl-1-(dibenzo[b,d]furan-4-yl)-1-(4-(tributylsilyl)phenyl)phosphanamine

¹H NMR(500 MHz, CDCl₃): δ 8.02(m, 2H), 7.57(m, 1H), 7.47 (m, 1H), 7.44(m, 6H), 7.38(m, 1H), 7.32(m, 1H), 7.30(m, 1H), 7.11(m, 6H), 2.72(m, 2H), 1.51(m, 18H), 1.47(m, 2H), 1.44(m, 18H), 1.40(m, 2H), 1.26(m, 18H), 0.94 (m, 3H), 0.93 (m, 27H)

### Synthesis Example 23 (Formula 2-23)

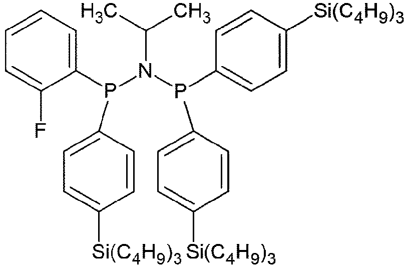

### N-(bis(4-(tributylsilyl)phenyl)phosphaneyl)-1-(2-fluorophenyl)-N-isopropyl-1-(4-(tributylsilyl)phenyl)phosphanamine

¹H NMR(500 MHz, CDCl₃): δ 7.47(m, 6H), 7.33(m, 1H), 7.23(m, 1H), 7.15(m, 7H), 7.10(m, 1H), 2.92(m, 1H), 1.48(m, 18H), 1.39(m, 18H), 1.32(m, 18H), 1.07(m, 6H), 0.94 (m, 27H)

### Synthesis Example 24 (Formula 2-24)

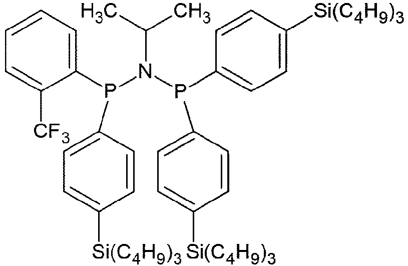

### N-(bis(4-(tributylsilyl)phenyl)phosphaneyl)-N-isopropyl-1-(4-(tributylsilyl)phenyl)-1-(2-(trifluoromethyl)phenyl)phosphanamine

¹H NMR (500 MHz, CDCl₃): δ 7.54(m, 1H), 7.52(m, 1H), 7.45(m, 6H), 7.26(m, 1H), 7.13(m, 6H), 7.09(m, 1H), 2.89(m, 1H), 1.44(m, 36H), 1.31(m, 18H), 1.13(m, 6H), 0.96(m, 27H)

### Synthesis Example 25 (Formula 2-25)

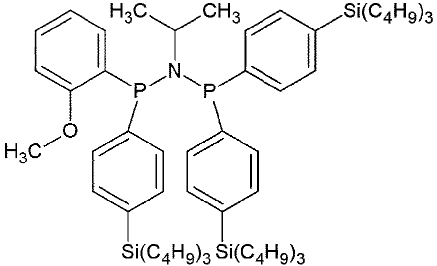

### N-(bis(4-(tributylsilyl)phenyl)phosphaneyl)-N-isopropyl-1-(2-methoxyphenyl)-1-(4-(tributylsilyl)phenyl)phosphanamine

¹H NMR(500 MHz, CDCl₃): δ 7.48(m, 6H), 7.40(m, 1H), 7.20(m, 1H), 7.18(m, 7H), 7.03(m, 1H), 3.99(m, 3H), 2.91(m, 1H), 1.49(m, 18H), 1.44(m, 18H), 1.28(m, 18H), 1.16(m, 6H), 1.00(m, 27H)

### Synthesis Example 26 (Formula 2-26)

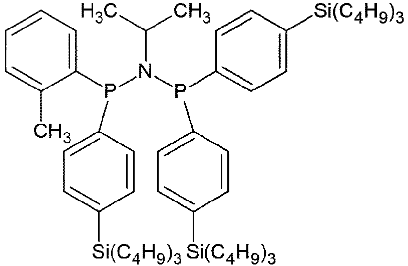

### N-(bis(4-(tributylsilyl)phenyl)phosphaneyl)-N-isopropyl-1-(o-tolyl)-1-(4-(tributylsilyl)phenyl)phosphanamine

¹H NMR(500 MHz, CDCl₃): δ 7.40(m, 6H), 7.37 (m, 1H), 7.20(m, 1H), 7.19(m, 1H), 7.14(m, 6H), 7.07(m, 1H), 2.87(m, 1H), 2.36(m, 3H), 1.44(m, 36H), 1.30(m, 18H), 1.14 (m, 6H), 0.96(m, 27H)

### Synthesis Example 27 (Formula 2-27)

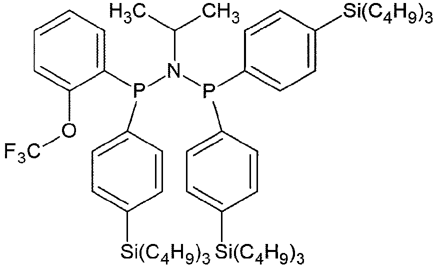

### N-(bis(4-(tributylsilyl)phenyl)phosphaneyl)-N-isopropyl-1-(4-(tributylsilyl)phenyl)-1-(2-(trifluoromethoxy)phenyl)phosphanamine

¹H NMR (500 MHz, CDCl₃): δ 7.45 (m, 1H), 7.43 (m, 6H), 7.20(m, 2H), 7.14(m, 6H), 7.02(m, 1H), 2.92(m, 1H), 1.46(m, 18H), 1.42(m, 18H), 1.30(m, 18H), 1.14(m, 6H), 0.93 (m, 27H)

### Synthesis Example 28 (Formula 2-28)

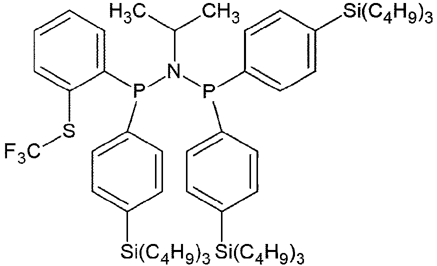

### N-(bis(4-(tributylsilyl)phenyl)phosphaneyl)-N-isopropyl-1-(4-(tributylsilyl)phenyl)-1-(2-((trifluoromethyl)thio)phenyl)phosphanamine

¹H NMR(500 MHz, CDCl₃): δ 7.42(m, 6H), 7.19(m, 2H), 7.17(m, 1H), 7.16(m, 6H), 7.06(m, 1H), 2.84(m, 1H), 1.49(m, 18H), 1.40(m, 18H), 1.25(m, 18H), 1.11(m, 6H), 0.99(m, 27H)

### Synthesis Example 29 (Formula 2-29)

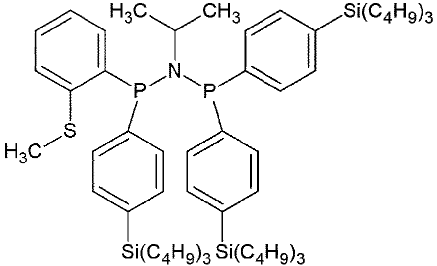

### N-(bis(4-(tributylsilyl)phenyl)phosphaneyl)-N-isopropyl-1-(2-(methylthio)phenyl)-1-(4-(tributylsilyl)phenyl)phosphanamine

¹H NMR(500 MHz, CDCl₃): δ 7.49(m, 6H), 7.22(m, 2H), 7.18(m, 1H), 7.15(m, 6H), 7.11(m, 1H), 2.88(m, 1H), 2.48(m, 3H), 1.44(m, 18H), 1.42(m, 18H), 1.30(m, 18H), 1.14(m, 6H), 0.98 (m, 27H)

### Synthesis Example 30 (Formula 2-30)

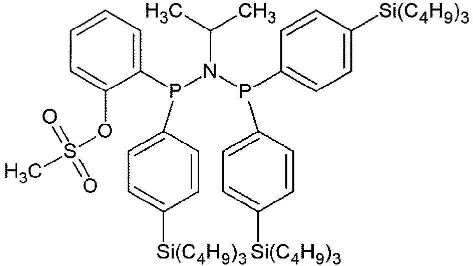

### 2-(((bis(4-(tributylsilyl)phenyl)phosphaneyl)(isopropyl)amino)(4-tributylsilyl)phenyl)phosphaneyl)phenyl methanesulfonate

¹H NMR (500 MHz, CDCl₃): δ 7.45(m, 1H), 7.42 (m, 7H), 7.34(m, 1H), 7.22(m, 1H), 7.15(m, 6H), 3.54(m, 3H), 2.92(m, 1H), 1.51(m, 18H), 1.39(m, 18H), 1.27(m, 18H), 1.15(m, 6H), 0.97(m, 27H)

### Synthesis Example 31 (Formula 2-31)

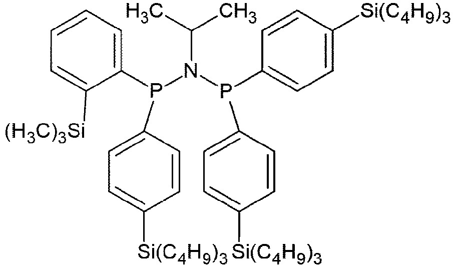

### N-(bis(4-(tributylsilyl)phenyl)phosphaneyl)-1-(4-(tributylsilyl)phenyl)-1-(2-(trimethylsilyl)phenyl)phosphanamine

¹H NMR(500 MHz, CDCl₃): δ 7.44 (m, 8H), 7.27 (m, 1H), 7.10(m, 7H), 2.92(m, 1H), 1.49(m, 18H), 1.43 (m, 18H), 1.26(m, 18H), 1.12(m, 6H), 0.96(m, 27H), 0.29(m, 9H)

### Synthesis Example 32 (Formula 2-32)

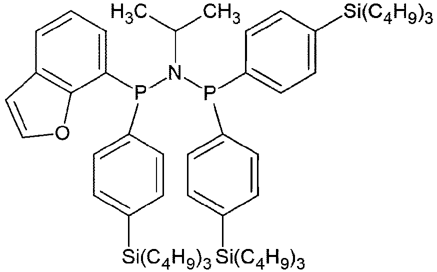

### 1-(benzofuran-7-yl)-N-(bis(4-(tributylsilyl)phenyl)phosphaneyl)-N-isopropyl-1-(4-(tributylsilyl)phenyl)phosphanamine

¹H NMR(500 MHz, CDCl₃): δ 7.67(m, 1H), 7.62(m, 1H), 7.44(m, 6H), 7.35(m, 1H), 7.23(m, 1H), 7.17(m, 6H), 6.82(m, 1H), 2.84(m, 1H), 1.45(m, 18H), 1.44(m, 18H), 1.28(m, 18H), 1.15(m, 6H), 1.00(m, 27H)

### Synthesis Example 33 (Formula 2-33)

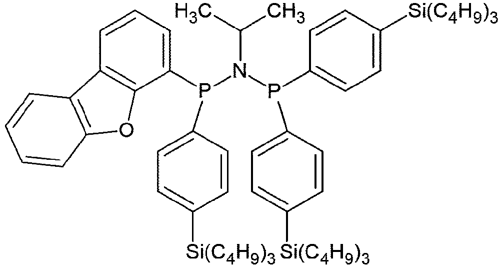

### N-(bis(4-(tributylsilyl)phenyl)phosphaneyl)-1-(dibenzo[b,d]furan-4-yl)-N-isopropyl-1-(4-(tributylsilyl)phenyl)phosphanamine

¹H NMR (500 MHz, CDCl₃): δ 8.03(m, 2H), 7.64(m,H), 7.46(m, 1H), 7.44(m, 6H), 7.38(m, 1H), 7.37(m, 4H), 7.23(m, 1H), 7.12(m, 6H), 2.88(m, 1H), 1.48(m, 18H), 1.40(m, 18H), 1.28 (m, 18H), 1.14(m, 6H), 0.95(m, 27H)

### Synthesis Example 34 (Formula 2-34)

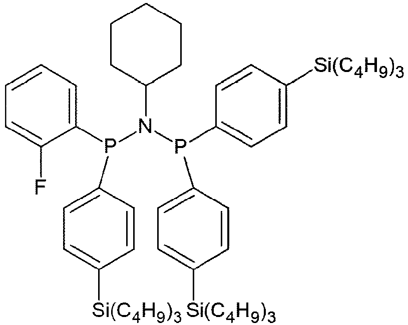

### N-(bis(4-(tributylsilyl)phenyl)phosphaneyl)-N-cyclohexyl-1-(2-fluorophenyl)-1-(4-(tributylsilyl)phenyl)phosphanamine

¹H NMR(500 MHz, CDCl₃): δ 7.50(m, 6H), 7.35(m, 1H), 7.27(m, 1H), 7.17(m, 7H), 7.05(m, 1H), 2.60(m, 4H), 1.80 (m, 2H), 1.54(m, 2H), 1.49(m, 1H), 1.48(m, 18H), 1.47(m, 1H), 1.40(m, 18H), 1.32 (m, 18H), 1.28(m, 2H), 1.18 (m, 2H), 0.99(m, 27H)

### Synthesis Example 35 (Formula 2-35)

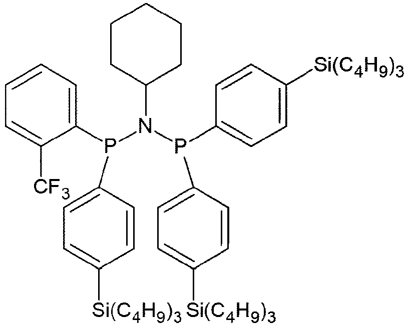

### N-(bis(4-(tributylsilyl)phenyl)phosphaneyl)-N-cyclohexyl-1-(4-(tributylsilyl)phenyl)-1-(2-(trifluoromethyl)phenyl)phosphanamine

¹H NMR(500 MHz, CDCl₃): δ 7.47(m, 6H), 7.45(m, 1H), 7.44(m, 1H), 7.25(m, 1H), 7.14(m, 6H), 7.11 (m, 1H), 2.62(m, 1H), 1.79(m, 2H), 1.50(m, 2H), 1.47(m, 19H), 1.45(m, 19H), 1.28(m, 2H), 1.26(m, 18H), 1.21(m, 2H), 0.99(m, 27H)

### Synthesis Example 36 (Formula 2-36)

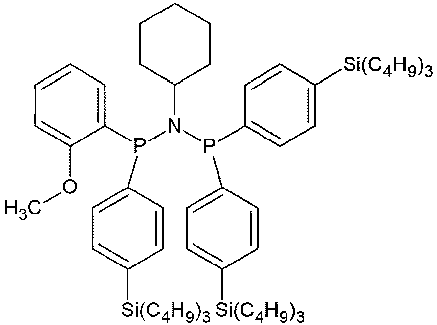

### N-(bis(4-(tributylsilyl)phenyl)phosphaneyl)-N-cyclohexyl-1-(2-methoxyphenyl)-1-(4-(tributylsilyl)phenyl)phosphanamine

¹H NMR(500 MHz, CDCl₃): δ 7.42(m, 6H), 7.37 (m, 1H), 7.12(m, 7H), 7.10(m, 1H), 7.03(m, 1H), 3.98(m, 3H), 2.62(m, 1H), 1.76(m, 2H), 1.56(m, 2H), 1.49(m, 1H), 1.48(m, 19H), 1.46(m, 18H), 1.32(m, 18H), 1.28 (m, 2H), 1.17(m, 2H), 0.98 (m, 27H)

### Synthesis Example 37 (Formula 2-37)

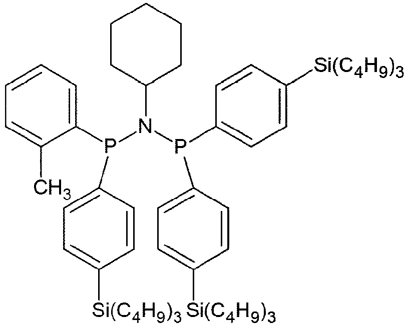

### N-(bis(4-(tributylsilyl)phenyl)phosphaneyl)-N-cyclohexyl-1-(o-tolyl)-1-(4-(tributylsilyl)phenyl)phosphanamine

¹H NMR(500 MHz, CDCl₃): δ 7.40(m, 6H), 7.38 (m, 1H), 7.22(m, 1H), 7.14(m, 1H), 7.13(m, 1H), 7.11(m, 6H), 2.66(m, 1H), 2.38(m, 3H), 1.83(m, 2H), 1.52(m, 2H), 1.51(m, 19H), 1.49(m, 1H), 1.38 (m, 18H), 1.30(m, 18H), 1.28 (m, 2H), 1.21 (m, 2H), 0.96(m, 27H)

### Synthesis Example 38 (Formula 2-38)

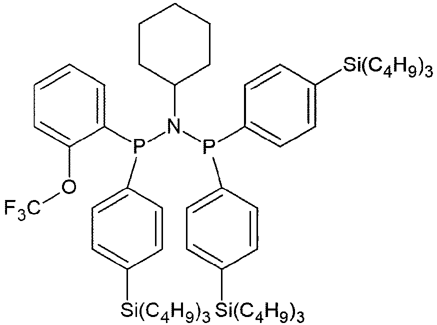

### N-(bis(4-(tributylsilyl)phenyl)phosphaneyl)-N-cyclohexyl-1-(4-(tributylsilyl)phenyl)-1-(2-(trifluoromethoxy)phenyl)phosphanamine

¹H NMR(500 MHz, CDCl₃): δ 7.50(m, 6H), 7.45(m, 1H), 7.14(m, 1H), 7.13(m, 6H), 7.12(m, 1H), 7.01 (m, 1H), 2.66(m, 1H), 1.80(m, 2H), 1.53(m, 2H), 1.52(m, 19H), 1.44(m, 1H), 1.42(m, 18H), 1.25(m, 18H), 1.24(m, 2H), 1.15(m, 2H), 0.94 (m, 27H)

### Synthesis Example 39 (Formula 2-39)

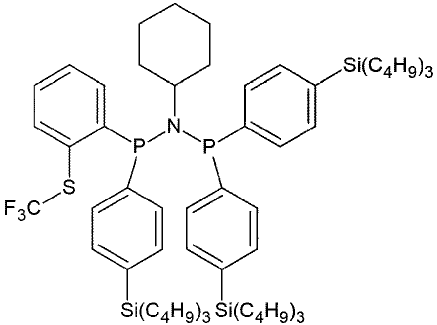

### N-(bis(4-(tributylsilyl)phenyl)phosphaneyl)-N-cyclohexyl-1-(4-(tributylsilyl)phenyl)-1-(2-((trifluoromethyl)thio)phenyl)phosphanamine

¹H NMR (500 MHz, CDCl₃): δ 7.49(m, 6H), 7.20(m, 2H), 7.17(m, 1H), 7.12(m, 6H), 7.02(m, 1H), 2.62(m, 1H), 1.75(m, 2H), 1.57(m, 2H), 1.54(m, 1H), 1.47(m, 18H), 1.45(m, 1H), 1.43(m, 18H), 1.28 (m, 2H), 1.25(m, 18H), 1.12(m, 2H), 0.99(m, 27H)

### Synthesis Example 40 (Formula 2-40)

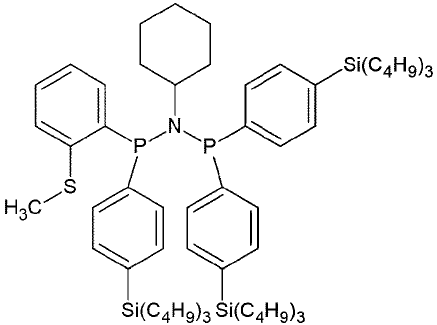

### N-(bis(4-(tributylsilyl)phenyl)phosphaneyl)-N-cyclohexyl-1-(2-(methylthio)phenyl)-1-(4-(tributylsilyl)phenyl)phosphanamine

¹H NMR(500 MHz, CDCl₃): δ 7.45(m, 6H), 7.26(m, 2H), 7.17 (m, 1H), 7.15(m, 6H), 7.02(m, 1H), 2.59(m, 1H), 2.53 (m, 3H), 1.75(m, 2H), 1.50(m, 1H), 1.49(m, 3H), 1.45(m, 18H), 1.36(m, 18H), 1.26(m, 2H), 1.24(m, 18H), 1.19(m, 2H), 0.96(m, 27H)

### Synthesis Example 41 (Formula 2-41)

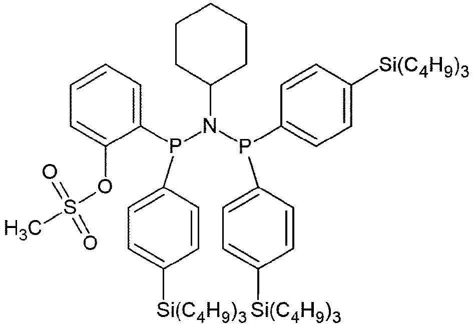

### 2-(((bis(4-(tributylsilyl)phenyl)phosphaneyl)(cyclohexyl)amino)(4-tributylsilyl)phenyl)phosphaneyl)phenyl methanesulfonate

¹H NMR(500 MHz, CDCl₃): δ 7.54 (m, 1H), 7.48 (m, 6H), 7.46(m, 1H), 7.38(m, 1H), 7.26(m, 1H), 7.12(m, 6H), 3.63(m, 3H), 2.63(m, 1H), 1.76(m, 2H), 1.53(m, 1H), 1.50(m, 18H), 1.49(m, 2H), 1.48(m, 1H), 1.44(m, 18H), 1.23(m, 20H), 1.19(m, 2H), 0.94(m, 27H)

### Synthesis Example 42 (Formula 2-42)

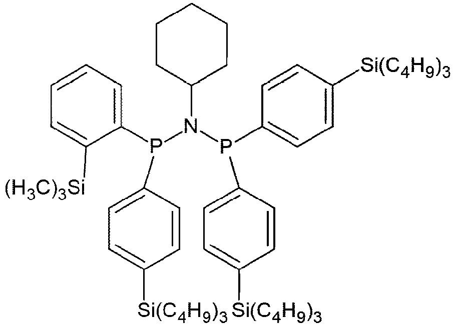

### N-(bis(4-(tributylsilyl)phenyl)phosphaneyl)-N-cyclohexyl-1-(4-(tributylsilyl)phenyl)-1-(2-(trimethylsilyl)phenyl)phosphanamine

¹H NMR (500 MHz, CDCl₃): δ 7.48(m, 8H), 7.32(m, 1H), 7.17(m, 7H), 2.57(m, 1H), 1.79(m, 2H), 1.55(m, 1H), 1.51(m, 2H), 1.49(m, 1H), 1.46(m, 18H), 1.44(m, 18H), 1.32(m, 18H), 1.30(m, 2H), 1.12(m, 2H), 0.99(m, 27H), 0.30(m, 9H)

### Synthesis Example 43 (Formula 2-43)

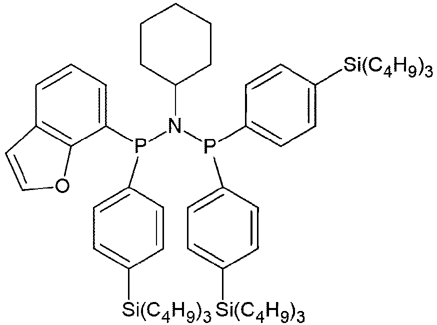

### 1-(benzofuran-7-yl)-N-(bis(4-(tributylsilyl)phenyl)phosphaneyl)-N-cyclohexyl-1-(4-(tributylsilyl)phenyl)phosphanamine

¹H NMR(500 MHz, CDCl₃): δ 7.70(m, 1H), 7.68 (m, 1H), 7.47(m, 6H), 7.38(m, 1H), 7.28(m, 1H), 7.17(m, 6H), 6.82(m, 1H), 2.60(m, 1H), 1.79(m, 2H), 1.56(m, 2H), 1.51(m, 18H), 1.47(m, 2H), 1.38(m, 18H), 1.29(m, 18H), 1.23 (m, 2H), 1.20 (m, 2H), 0.94(m, 27H)

### Synthesis Example 44 (Formula 2-44)

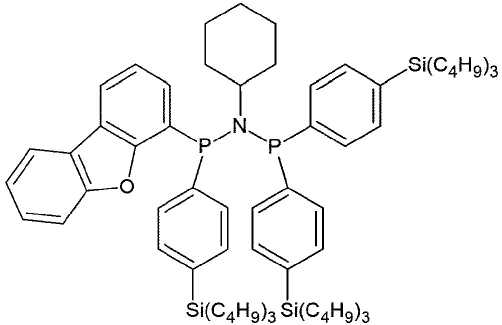

### N-(bis(4-(tributylsilyl)phenyl)phosphaneyl)-N-cyclohexyl-1-(dibenzo[b,d]furan-4-yl)-1-(4-(tributylsilyl)phenyl)phosphanamine

¹H NMR(500 MHz, CDCl₃): δ 8.05(m, 2H), 7.59(m, 1H), 7.47(m, 1H), 7.41(m, 6H), 7.37(m, 1H), 7.31 (m, 1H), 7.28(m, 1H), 7.14(m, 6H), 2.64(m, 1H), 1.81 (m, 2H), 1.54(m, 1H), 1.52 (m, 2H), 1.50(m, 18H), 1.48(m, 1H), 1.46(m, 18H), 1.30(m, 18H), 1.25(m, 2H), 1.21(m, 2H), 0.99(m, 27H)

### Synthesis Example 45 (Formula 2-45)

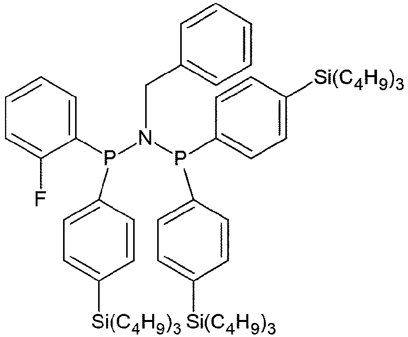

### N-benzyl-N-(bis(4-(tributylsilyl)phenyl)phosphaneyl)-1-(2-fluorophenyl)-1-(4-(tributylsilyl)phenyl)phosphanamine

¹H NMR(500 MHz, CDCl₃): δ 7.48(m, 6H), 7.39(m, 1H), 7.38(m, 1H), 7.34(m, 4H), 7.25(m, 1H), 7.16(m, 7H), 7.04(m, 1H), 4.01(m, 2H), 1.47(m, 18H), 1.41 (m, 18H), 1.30(m, 18H), 0.97(m, 27H)

### Synthesis Example 46 (Formula 2-46)

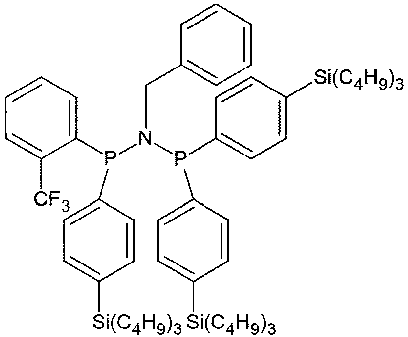

### N-benzyl-N-(bis(4-(tributylsilyl)phenyl)phosphaneyl)-1-(4-(tributylsilyl)phenyl)-1-(2-(trifluoromethyl)phenyl)phosphanamine

¹H NMR(500 MHz, CDCl₃): δ 7.48 (m, 1H), 7.47 (m, 6H), 7.46(m, 1H), 7.40(m, 4H), 7.32(m, 1H), 7.22(m, 1H), 7.13(m, 6H), 7.12(m, 1H), 3.95(m, 2H), 1.50(m, 18H), 1.45(m, 18H), 1.24 (m, 18H), 1.00(m, 27H)

### Synthesis Example 47 (Formula 2-47)

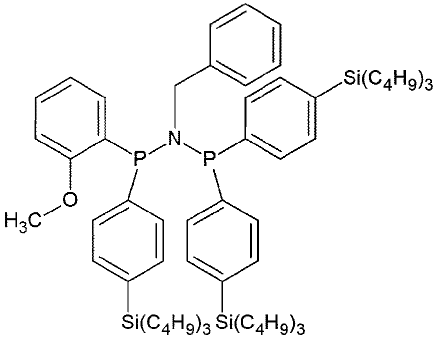

### N-benzyl-N-(bis(4-(tributylsilyl)phenyl)phosphaneyl)-1-(2-methoxyphenyl)-1-(4-(tributylsilyl)phenyl)phosphanamine

¹H NMR(500 MHz, CDCl₃): δ 7.49(m, 6H), 7.38 (m, 5H), 7.37(m, 1H), 7.19(m, 1H), 7.13(m, 1H), 7.11(m, 6H), 6.99(m, 1H), 4.01(m, 5H), 1.45(m, 18H), 1.44(m, 18H), 1.31(m, 18H), 0.93(m, 27H)

### Synthesis Example 48 (Formula 2-48)

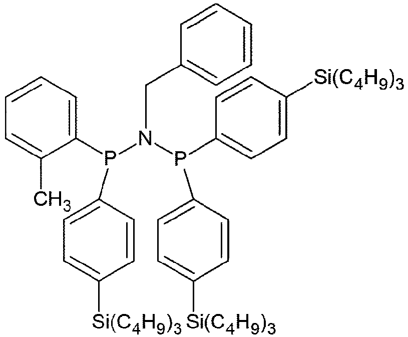

### N-benzyl-N-(bis(4-(tributylsilyl)phenyl)phosphaneyl)-1-(o-tolyl)-1-(4-(tributylsilyl)phenyl)phosphanamine

¹H NMR(500 MHz, CDCl₃): δ 7.40(m, 6H), 7.37 (m, 1H), 7.36(m, 5H), 7.21(m, 2H), 7.14(m, 6H), 7.11(m, 1H), 3.94 (m, 2H), 2.32(m, 3H), 1.51(m, 18H), 1.42(m, 18H), 1.24(m, 18H), 0.95(m, 27H

### Synthesis Example 49 (Formula 2-49)

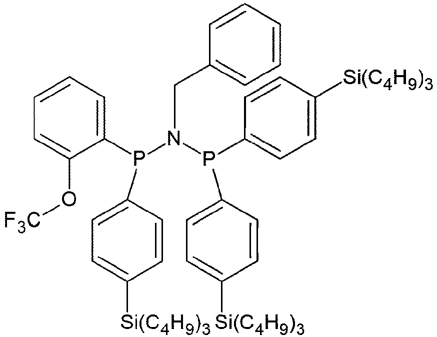

### N-benzyl-N-(bis(4-(tributylsilyl)phenyl)phosphaneyl)-1-(4-(tributylsilyl)phenyl)-1-(2-(trifluoromethoxy)phenyl)phosphanamine

¹H NMR(500 MHz, CDCl₃): δ 7.46(m, 7H), 7.34 (m, 1H), 7.31 (m, 4H), 7.20(m, 1H), 7.17(m, 1H), 7.13(m, 6H), 7.00 (m, 1H), 3.99(m, 2H), 1.46(m, 18H), 1.39(m, 18H), 1.28 (m, 18H), 0.99(m, 27H)

### Synthesis Example 50 (Formula 2-50)

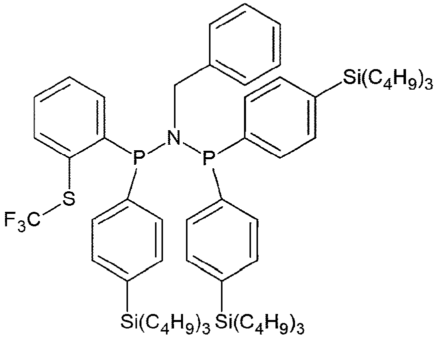

### N-benzyl-N-(bis(4-(tributylsilyl)phenyl)phosphaneyl)-1-(4-(tributylsilyl)phenyl)-1-(2-((trifluoromethyl)thio)phenyl)phosphanamine

¹H NMR(500 MHz, CDCl₃): δ 7.44 (m, 6H), 7.40(m, 4H), 7.37(m, 1H), 7.24(m, 2H), 7.19(m, 1H), 7.12(m, 6H), 7.10(m, 1H), 3.94(m, 2H), 1.53(m, 18H), 1.42(m, 18H), 1.30(m, 18H), 0.96(m, 27H)

### Synthesis Example 51 (Formula 2-51)

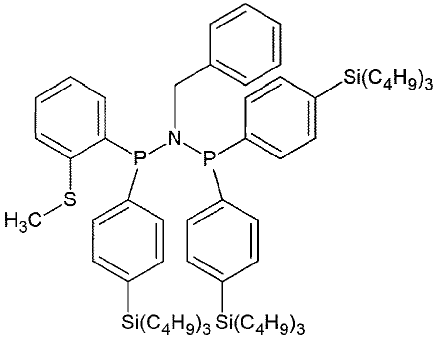

### N-benzyl-N-(bis(4-(tributylsilyl)phenyl)phosphaneyl)-1-(2-(methylthio)phenyl)-1-(4-(tributylsilyl)phenyl)phosphanamine

¹H NMR(500 MHz, CDCl₃): δ 7.43(m, 6H), 7.39(m, 4H), 7.31 (m, 1H), 7.27(m, 2H), 7.23(m, 1H), 7.12(m, 6H), 7.06(m, 1H), 3.99(m, 2H), 2.54(m, 3H), 1.45(m, 36H), 1.24 (m, 18H), 0.98 (m, 27H)

### Synthesis Example 52 (Formula 2-52)

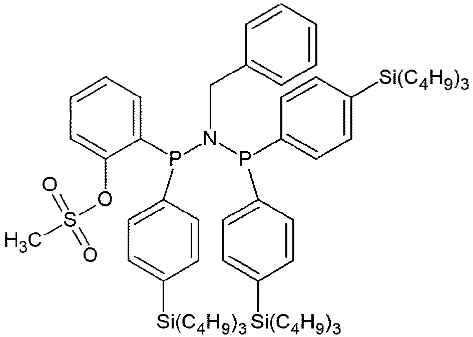

### 2-((benzyl(bis(4-(tributylsilyl)phenyl)phosphaneyl) amino) (4-tributylsilyl)phenyl)phosphaneyl)phenyl methanesulfonate

¹H NMR(500 MHz, CDCl₃): δ 7.46(m, 1H), 7.44(m, 6H), 7.41(m, 1H), 7.40(m, 4H), 7.36(m, 2H), 7.24(m, 1H), 7.17 (m, 6H), 3.92(m, 2H), 3.62(m, 3H), 1.46(m, 18H), 1.41(m, 18H), 1.32(m, 18H), 0.95(m, 27H)

### Synthesis Example 53 (Formula 2-53)

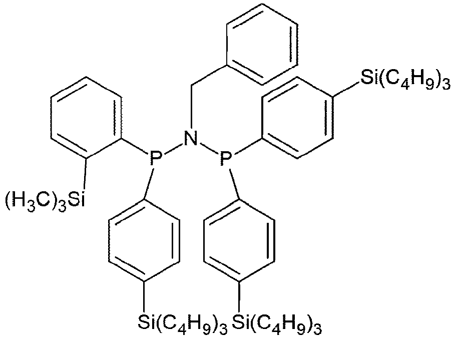

### N-benzyl-N-(bis(4-(tributylsilyl)phenyl)phosphaneyl)-1-(4-(tributylsilyl)phenyl)-1-(2-(trimethylsilyl)phenyl)phosphanamine

¹H NMR(500 MHz, CDCl₃): δ 7.48 (m, 8H), 7.35 (m, 4H), 7.33(m, 2H), 7.19(m, 7H), 3.97(m, 2H), 1.45(m, 18H), 1.43(m, 18H), 1.25(m, 18H), 1.00(m, 27H), 0.32(m, 9H)

### Synthesis Example 54 (Formula 2-54)

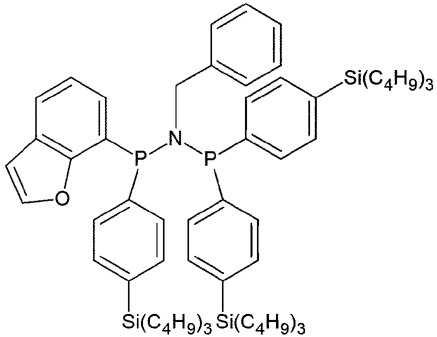

### 1-(benzofuran-7-yl)-N-benzyl-N-(bis(4-(tributylsilyl)phenyl)phosphaneyl)-1-(4-(tributylsilyl)phenyl)phosphanamine

¹H NMR (500 MHz, CDCl₃): δ 7.70(m, 1H), 7.67 (m, 1H), 7.43 (m, 6H), 7.33(m, 2H), 7.32(m, 1H), 7.31 (m, 4H), 7.16(m, 6H), 6.80(m, 1H), 3.97(m, 2H), 1.45(m, 18H), 1.42(m, 18H), 1.26(m, 18H), 0.97 (m, 27H)

### Synthesis Example 55 (Formula 2-55)

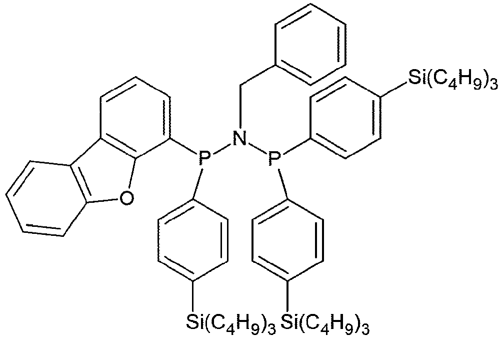

### N-benzyl-N-(bis(4-(tributylsilyl)phenyl)phosphaneyl)-1-(dibenzo[b,d]furan-4-yl)-1-(4-(tributylsilyl)phenyl)phosphanamine

¹H NMR (500 MHz, CDCl₃): δ 8.06(m, 2H), 7.59 (m, 1H), 7.49 (m, 6H), 7.44(m, 1H), 7.40(m, 5H), 7.31 (m, 1H), 7.30(m, 1H), 7.23(m, 1H), 7.19(m, 6H), 4.01(m, 2H), 1.46(m, 18H), 1.38 (m, 18H), 1.33 (m, 18H), 0.95(m, 27H)

### Synthesis Example 56 (Formula 2-56)

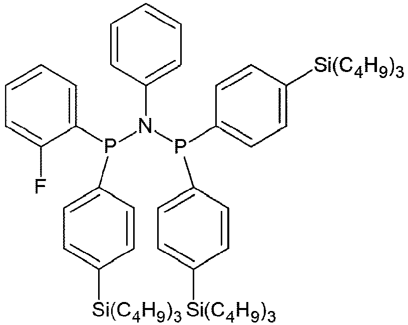

### N-(bis(4-(tributylsilyl)phenyl)phosphaneyl)-1-(2-fluorophenyl)-N-phenyl-1-(4-(tributylsilyl)phenyl)phosphanamine

¹H NMR(500 MHz, CDCl₃): δ 7.47 (m, 6H), 7.39(m, 1H), 7.25(m, 1H), 7.18(m, 7H), 7.13(m, 2H), 7.07(m, 1H), 6.84 (m, 1H), 6.64(m, 2H), 1.49(m, 18H), 1.37(m, 18H), 1.31 (m, 18H), 0.93(m, 27H)

### Synthesis Example 57 (Formula 2-57)

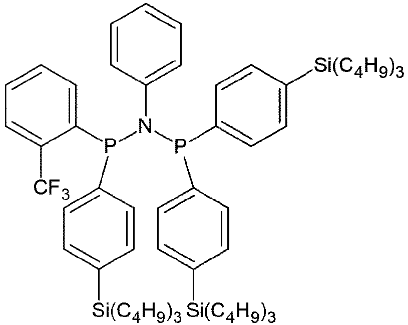

### N-(bis(4-(tributylsilyl)phenyl)phosphaneyl)-N-phenyl-1-(4-(tributylsilyl)phenyl)-1-(2-(trifluoromethyl)phenyl)phosphanamine

¹H NMR (500 MHz, CDCl₃): δ 7.49 (m, 1H), 7.48 (m, 1H), 7.40 (m, 6H), 7.24(m, 1H), 7.13(m, 2H), 7.12(m, 6H), 7.11 (m, 1H), 6.86(m, 1H), 6.62(m, 2H), 1.49(m, 18H), 1.43(m, 18H), 1.24 (m, 18H), 0.92 (m, 27H)

### Synthesis Example 58 (Formula 2-58)

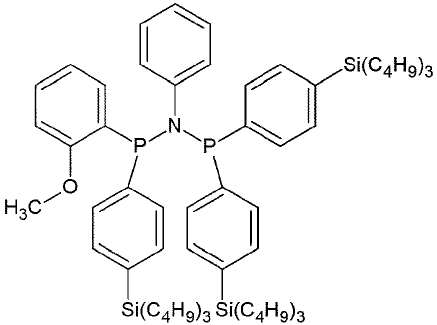

### N-(bis(4-(tributylsilyl)phenyl)phosphaneyl)-1-(2-methoxyphenyl)-N-phenyl-1-(4-(tributylsilyl)phenyl)phosphanamine

¹H NMR(500 MHz, CDCl₃): δ 7.49(m, 6H), 7.42(m, 1H), 7.18(m, 3H), 7.12(m, 7H), 6.96(m, 1H), 6.85(m, 1H), 6.62(m, 2H), 3.99(m, 3H), 1.44(m, 36H), 1.32(m, 18H), 1.01(m, 27H)

### Synthesis Example 59 (Formula 2-59)

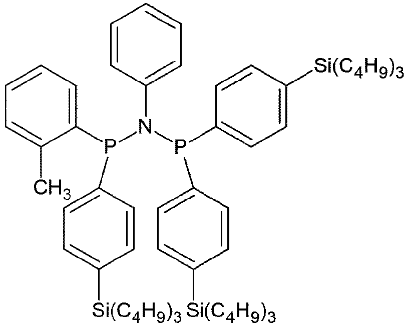

### N-(bis(4-(tributylsilyl)phenyl)phosphaneyl)-N-phenyl-1-(o-tolyl)-1-(4-(tributylsilyl)phenyl)phosphanamine

¹H NMR(500 MHz, CDCl₃) : δ 7.47(m, 2H), 7.35 (m, 1H), 7.24(m, 6H), 7.18(m, 1H), 7.17(m, 1H), 7.16(m, 1H), 7.11 (m, 1H), 6.80(m, 1H), 6.65(m, 6H), 2.32(m, 2H), 1.43 (m, 18H), 1.41(m, 18H), 1.29(m, 18H), 0.98(m, 27H)

### Synthesis Example 60 (Formula 2-60)

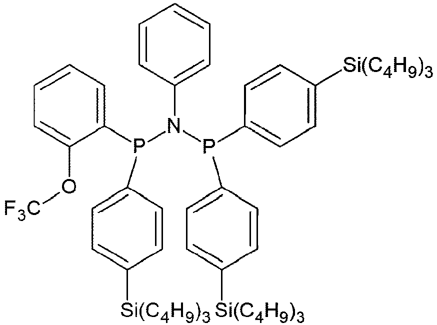

### N-(bis(4-(tributylsilyl)phenyl)phosphaneyl)-N-phenyl-1-(4-(tributylsilyl)phenyl)-1-(2-(trifluoromethoxy)phenyl)phosphanamine

¹H NMR(500 MHz, CDCl₃): δ 7.45(m, 1H), 7.44 (m, 6H), 7.17(m, 1H), 7.16(m, 2H), 7.15(m, 6H), 7.11(m, 1H), 7.06(m, 1H), 6.80(m, 1H), 6.67(m, 2H), 1.50(m, 18H), 1.44(m, 18H), 1.31(m, 18H), 0.94(m, 27H)

### Synthesis Example 61 (Formula 2-61)

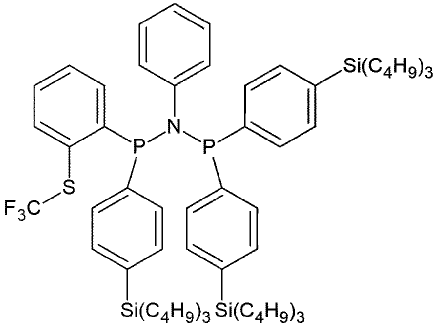

### N-(bis(4-(tributylsilyl)phenyl)phosphaneyl)-N-phenyl-1-(4-(tributylsilyl)phenyl)-1-(2-((trifluoromethyl)thio)phenyl)phosphanamine

¹H NMR(500 MHz, CDCl₃): δ 7.44 (m, 6H), 7.26(m, 2H), 7.18(m, 1H), 7.17(m, 2H), 7.14(m, 6H), 7.11(m, 1H), 6.81(m, 1H), 6.66(m, 2H), 1.45(m, 36H), 1.27(m, 18H), 1.00(m, 27H)

### Synthesis Example 62 (Formula 2-62)

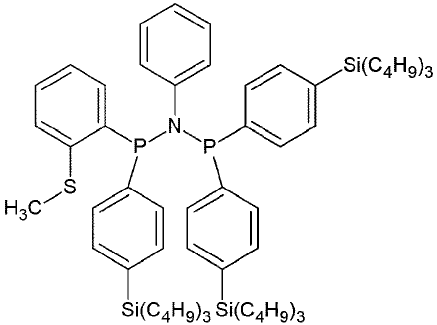

### N-(bis(4-(tributylsilyl)phenyl)phosphaneyl)-1-(2-(methylthio)phenyl)-N-phenyl-1-(4-(tributylsilyl)phenyl)phosphanamine

¹H NMR(500 MHz, CDCl₃): δ 7.48(m, 6H), 7.23(m, 1H), 7.22(m, 2H), 7.16(m, 6H), 7.11(m, 2H), 7.02(m, 1H), 6.79(m, 1H), 6.68(m, 2H),2.52(m, 3H), 1.49(m, 18H), 1.46(m, 18H), 1.32(m, 18H), 0.93(m, 27H)

### Synthesis Example 63 (Formula 2-63)

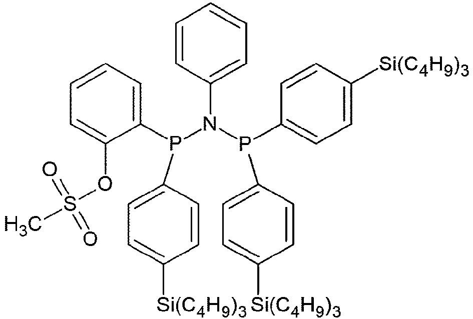

### 2-(((bis(4-(tributylsilyl) phenyl) phosphaneyl) (phenyl) amino) (4-tributylsilyl)phenyl)phosphaneyl)phenyl methanesulfonate

¹H NMR(500 MHz, CDCl₃): δ 7.54 (m, 1H), 7.47(m, 7H), 7.34(m, 1H), 7.28(m, 1H), 7.10(m, 8H), 6.80(m, 1H), 6.64 (m, 2H), 3.54(m, 3H), 1.45(m, 18H), 1.44 (m, 18H), 1.29(m, 18H), 1.01(m, 27H)

### Synthesis Example 64 (Formula 2-64)

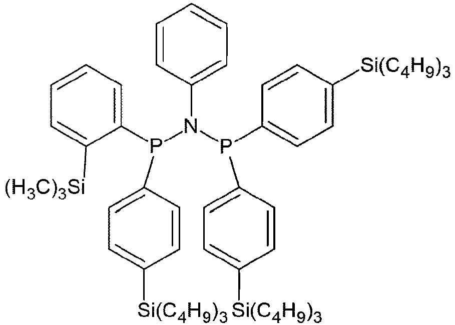

### N-(bis(4-(tributylsilyl)phenyl)phosphaneyl)-N-phenyl-1-(4-(tributylsilyl)phenyl)-1-(2-(trimethylsilyl)phenyl)phosphanamine

¹H NMR (500 MHz, CDCl₃) : δ 7.50(m, 8H), 7.31 (m, 1H), 7.17(m, 9H), 6.87(m, 1H), 6.62(m, 2H), 1.50(m, 18H), 1.39(m, 18H), 1.29(m, 18H), 0.93(m, 27H), 0.27(m, 9H)

### Synthesis Example 65 (Formula 2-65)

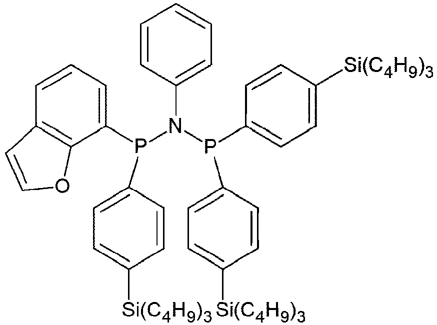

### 1-(benzofuran-7-yl)-N-(bis(4-(tributylsilyl)phenyl)phosphaneyl)-N-phenyl-1-(4-(tributylsilyl)phenyl)phosphanamine

¹H NMR (500 MHz, CDCl₃): δ 7.69(m, 1H), 7.65(m, 1H), 7.48(m, 6H), 7.30(m, 1H), 7.29(m, 1H), 7.19(m, 6H), 7.15(m, 2H), 6.81(m, 1H), 6.80(m, 1H), 6.70(m, 2H), 1.47(m, 18H), 1.42(m, 18H), 1.29(m, 18H), 0.99(m, 27H)

### Synthesis Example 66 (Formula 2-66)

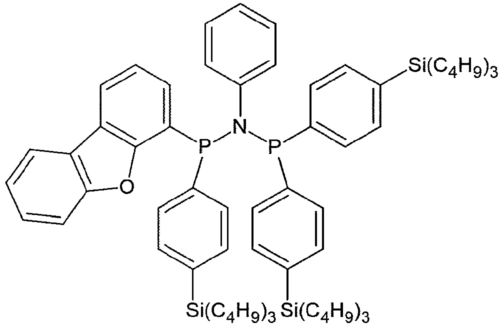

### N-(bis(4-(tributylsilyl)phenyl)phosphaneyl)-1-(dibenzo[b,d]furan-4-yl)-N-phenyl-1-(4-(tributylsilyl)phenyl)phosphanamine

¹H NMR (500 MHz, CDCl₃): δ 8.01(m, 2H), 7.64(m, 1H), 7.46 (m, 6H), 7.43(m, 1H), 7.41(m, 1H), 7.33 (m, 1H), 7.30(m, 1H), 7.13(m, 2H), 7.09(m, 6H), 6.84(m, 1H), 6.63(m, 2H), 1.52(m, 18H), 1.43 (m, 18H), 1.24(m, 18H), 0.97 (m, 27H)

### Synthesis Example 67 to 86: Synthesis of ligand compound represented by Formula 9-1 to Formula 9-20

20 mmol (2 eq) of m-bromo-(tri-n-butylsilyl)-C₆H₄ was dissolved in 20 ml of tetrahydrofuran, and then cooled to - 78 °C. While maintaining the temperature, 20 mmol (2 eq) of n-butyllithium was added dropwise thereto, and then stirred for 3 hours. Subsequently, 10 mmol (1 eq) of dichloro(diethylamino)phosphine dissolved in 10 mL of tetrahydrofuran was added dropwise thereto, and then the temperature was raised to room temperature, and the mixture was stirred overnight. Thereafter, the solvent was removed using vacuum, and the obtained intermediate was dissolved in 30 mL of hexane, and then HCl (in ether, 2 eq) was added thereto, stirred for 15 minutes, and filtered. The filtrate was dried in a vacuum state to obtain (m-(tri-n-butylsilyl)-C₆H₄)₂PCl. 11 mmol (2.2 eq) of R⁵-NH₂ and 10 ml of dichloromethane were introduced into a well-dried flask under a nitrogen atmosphere and stirred. 5 mmol (1 eq) of (m-(tri-n-butylsilyl)-C₆H₄)₂PCl was diluted in 10 ml of dichloromethane and then slowly introduced into the flask. After the reaction was completed by stirring for 4 hours, the precipitated solids were removed through a filter, the solvent was removed under reduced pressure, and then (m-(tri-n-butylsilyl)-C₆H₄)₂P-NHR⁵ was obtained through column chromatography.

5 mmol (1 eq) of (m-(tri-n-butylsilyl)-C₆H₄)PCl₂ was dissolved in 10 ml of ether, and then cooled to 0 °C. Subsequently, 10 mmol (2 eq) of HNEt₂ dissolved in 10 ml of ether was slowly introduced thereto, and stirred overnight. The solids were removed through a filter, and 1 eq of ArMgBr prepared from Ar and Mg pieces (Mg turning) was slowly introduced thereto, and then stirred at room temperature overnight. Thereafter, HCl (in ether, 2 eq) was introduced thereto and stirred for 30 minutes at room temperature. The solids were removed through a filter and the pressure was reduced to obtain (m-(tri-n-butylsilyl)-C₆H₄)ArPCl. 5 mmol (1 eq) of (m-(tri-n-butylsilyl)-C₆H₄)ArPCl was dissolved in 10 ml of dichloromethane, and then 15 mmol (3 eq) of triethylamine was added thereto. Subsequently, 5 mmol (1 eq) of (m-(tri-n-butylsilyl)-C₆H₄)₂P-NHR⁵ was dissolved in dichloromethane and then slowly introduced thereto, and the mixture was stirred at room temperature overnight. The solvent was removed using vacuum, dissolved in hexane, and ligand compounds represented by Formula 9-1 to Formula 9-20 were obtained by column chromatography.

R⁵ and Ar, which are substituents of the reactants used in Synthesis Example 67 to Synthesis Example 86, are each shown in Table 2 below, and the structure of the ligand compound prepared in each Synthetic Example and ¹H NMR data are described below.

**[Table 2]**

| Classific ation | R⁵ | Ar(*binding position) |
|---|---|---|
| Synthesis Example 67 | Methyl (-CH₃) | 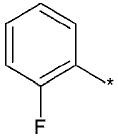 |
| Synthesis Example 68 | Methyl (-CH₃) | 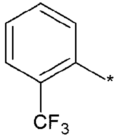 |
| Synthesis Example 69 | Methyl (-CH₃) | 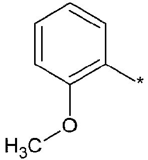 |
| Synthesis Example 70 | Methyl (-CH₃) | 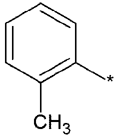 |
| Synthesis Example 71 | Methyl (-CH₃) | 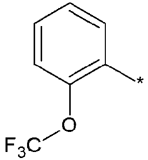 |
| Synthesis Example 72 | n-butyl(-C₄H₉) | 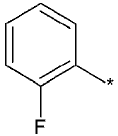 |
| Synthesis Example 73 | n-butyl (-C₄H₉) | 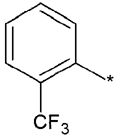 |
| Synthesis Example 74 | n-butyl (-C₄H₉) | 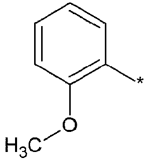 |
| Synthesis Example 75 | n-butyl (-C₄H₉) | 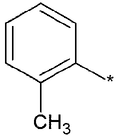 |
| Synthesis Example 76 | n-butyl (-C₄H₉) | 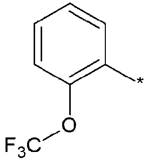 |
| Synthesis Example 77 | iso-propyl(-CH(CH₃)₂) | 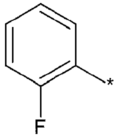 |
| Synthesis Example 78 | iso-propyl(-CH(CH₃)₂) | 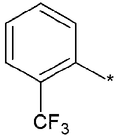 |
| Synthesis Example 79 | iso-propyl(-CH(CH₃)₂) | 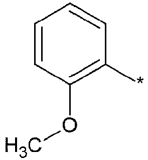 |
| Synthesis Example 80 | iso-propyl(-CH (CH₃)₂) | 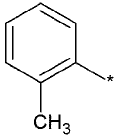 |
| Synthesis Example 81 | iso-propyl(-CH (CH₃)₂) | 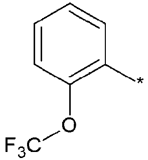 |
| Synthesis Example 82 | cyclohexyl (-C₆H₁₁) | 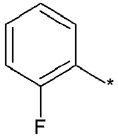 |
| Synthesis Example 83 | cyclohexyl (-C₆H₁₁) | 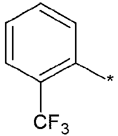 |
| Synthesis Example 84 | cyclohexyl (-C₆H₁₁) | 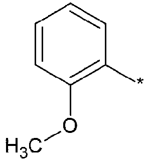 |
| Synthesis Example 85 | cyclohexyl (-C₆H₁₁) | 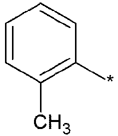 |
| Synthesis Example 86 | cyclohexyl (-C₆H₁₁) | 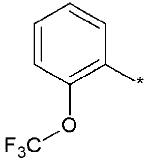 |

### Synthesis Example 67 (Formula 9-1)

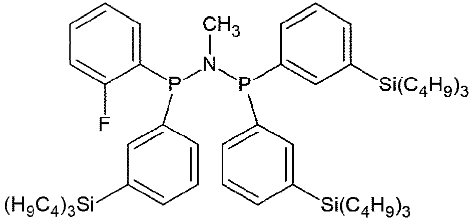

### N-(bis(3-(tributylsilyl)phenyl)phosphaneyl)-1-(2-fluorophenyl)-N-methyl-1-(3-(tributylsilyl)phenyl)phosphanamine

¹H NMR(500 MHz, CDCl₃): δ 7.63(m, 3H), 7.44(m, 3H), 7.40(m, 3H), 7.31(m, 1H), 7.23(m, 1H), 7.16(m, 4H), 7.08(m, 1H), 2.51(m, 3H), 1.51(m, 18H), 1.42(m, 18H), 1.33(m, 18H), 0.94(m, 27H)

### Synthesis Example 68 (Formula 9-2)

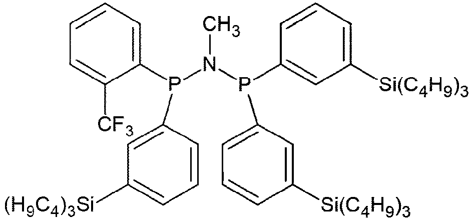

### N-(bis(3-(tributylsilyl)phenyl)phosphaneyl)-N-methyl-1-(3-(tributylsilyl)phenyl)-1-(2-(trifluoromethyl)phenyl)phosphanamine

¹H NMR(500 MHz, CDCl₃): δ 7.62 (m, 3H), 7.52 (m, 1H), 7.45(m, 1H), 7.44(m, 3H), 7.37(m, 3H), 7.24(m, 1H), 7.18(m, 3H), 7.04(m, 1H), 2.49(m, 3H), 1.44(m, 18H), 1.41(m, 18H), 1.33(m, 18H), 0.99(m, 27H)

### Synthesis Example 69 (Formula 9-3)

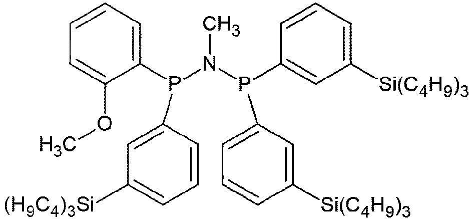

### N-(bis(3-(tributylsilyl)phenyl)phosphaneyl)-1-(2-methoxyphenyl)-N-methyl-1-(3-(tributylsilyl)phenyl)phosphanamine

¹H NMR(500 MHz, CDCl₃): δ 7.60(m, 3H), 7.47 (m, 3H), 7.38(m, 1H), 7.32(m, 3H), 7.16(m, 1H), 7.15(m, 1H), 7.11(m, 3H), 7.00(m, 1H), 3.97(m, 3H), 2.51(m, 3H), 1.48(m, 18H), 1.43 (m, 18H), 1.31 (m, 18H), 0.94(m, 27H)

### Synthesis Example 70 (Formula 9-4)

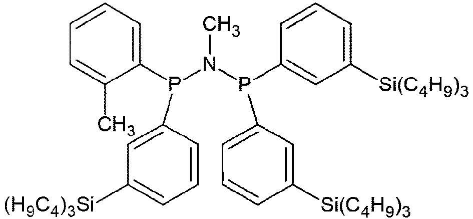

### N-(bis(3-(tributylsilyl)phenyl)phosphaneyl)-N-methyl-1-(o-tolyl)-1-(3-(tributylsilyl)phenyl)phosphanamine

¹H NMR (500 MHz, CDCl₃): δ 7.61(m, 3H), 7.44 (m, 3H), 7.37(m, 1H), 7.33(m, 3H), 7.28(m, 1H), 7.17(m, 1H), 7.16(m, 1H), 7.14(m, 3H), 2.57(m, 3H), 2.31(m, 3H), 1.44(m, 18H), 1.42(m, 18H), 1.28(m, 18H), 0.94(m, 27H)

### Synthesis Example 71 (Formula 9-5)

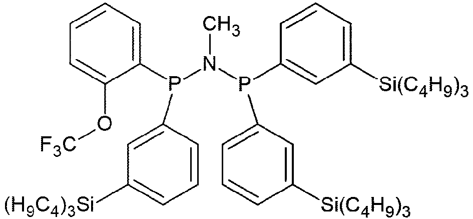

### N-(bis(3-(tributylsilyl)phenyl)phosphaneyl)-N-methyl-1-(3-(tributylsilyl)phenyl)-1-(2-(trifluoromethoxy)phenyl)phosphanamine

¹H NMR(500 MHz, CDCl₃): δ 7.59(m, 3H), 7.42 (m, 3H), 7.39(m, 1H), 7.37(m, 3H), 7.20(m, 1H), 7.13(m, 1H), 7.10 (m, 3H), 7.02(m, 1H), 2.48(m, 3H), 1.48(m, 18H), 1.43 (m, 18H), 1.28(m, 18H), 0.92(m, 27H)

### Synthesis Example 72 (Formula 9-6)

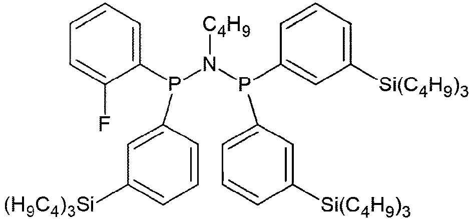

### N-(bis(3-(tributylsilyl)phenyl)phosphaneyl)-N-butyl-1-(2-fluorophenyl)-1-(3-(tributylsilyl)phenyl)phosphanamine

¹H NMR(500 MHz, CDCl₃): δ 7.56 (m, 3H), 7.47 (m, 3H), 7.40 (m, 3H), 7.31(m, 1H), 7.28(m, 1H), 7.12(m, 4H), 7.09(m, 1H), 2.67(m, 2H), 1.49(m, 2H), 1.47(m, 18H), 1.41(m, 18H), 1.40(m, 2H), 1.29(m, 18H), 1.00(m, 3H), 0.95(m, 27H)

### Synthesis Example 73 (Formula 9-7)

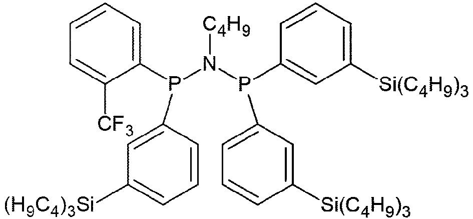

### N-(bis(3-(tributylsilyl)phenyl)phosphaneyl)-N-butyl-1-(3-(tributylsilyl)phenyl)-1-(2-(trifluoromethyl)phenyl)phosphanamine

¹H NMR(500 MHz, CDCl₃): δ 7.64(m, 3H), 7.46(m, 1H), 7.45(m, 3H), 7.42(m, 1H), 7.34(m, 3H), 7.20(m, 1H), 7.19(m, 3H), 7.05(m, 1H), 2.67(m, 2H), 1.52(m, 2H), 1.44(m, 18H), 1.40(m, 18H), 1.37(m, 2H), 1.33(m, 18H), 1.01 (m, 3H), 0.98 (m, 27H)

### Synthesis Example 74 (Formula 9-8)

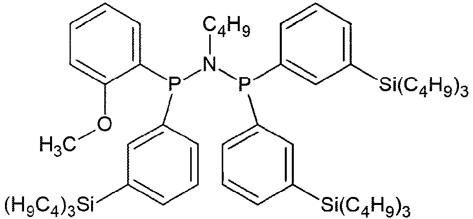

### N-(bis(3-(tributylsilyl)phenyl)phosphaneyl)-N-butyl-1-(2-methoxyphenyl)-1-(3-(tributylsilyl)phenyl)phosphanamine

¹H NMR(500 MHz, CDCl₃): δ 7.62(m, 3H), 7.46(m, 3H), 7.39(m, 1H), 7.32(m, 3H), 7.15(m, 1H), 7.12(m, 3H), 7.10(m, 1H), 7.05(m, 1H), 3.99(m, 3H), 2.63(m, 2H), 1.51(m, 18H), 1.46(m, 2H), 1.37(m, 2H), 1.36(m, 18H), 1.28 (m, 18H), 1.00(m, 27H), 0.97 (m, 3H)

### Synthesis Example 75 (Formula 9-9)

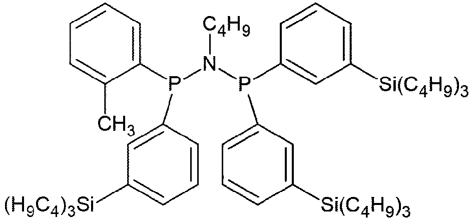

### N-(bis(3-(tributylsilyl)phenyl)phosphaneyl)-N-butyl-1-(o-tolyl)-1-(3-(tributylsilyl)phenyl)phosphanamine

¹H NMR(500 MHz, CDCl₃): δ 7.65(m, 3H), 7.46(m, 3H), 7.35(m, 3H), 7.34(m, 1H), 7.22(m, 1H), 7.16(m, 1H), 7.14(m, 1H), 7.12(m, 3H), 2.64(m, 2H), 2.31(m, 3H), 1.52(m, 18H), 1.46(m, 2H), 1.39(m, 18H), 1.38(m, 2H), 1.27(m, 18H), 1.00(m, 30H)

### Synthesis Example 76 (Formula 9-10)

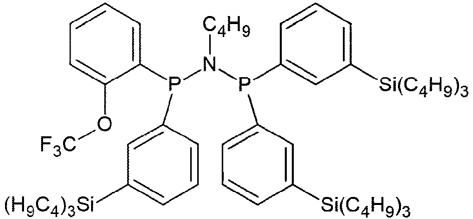

### N-(bis(3-(tributylsilyl)phenyl)phosphaneyl)-N-butyl-1-(3-(tributylsilyl)phenyl)-1-(2-(trifluoromethoxy)phenyl)phosphanamine

¹H NMR(500 MHz, CDCl₃): δ 7.64 (m, 3H), 7.47 (m, 1H), 7.40 (m, 3H), 7.33(m, 3H), 7.18(m, 1H), 7.15(m, 1H), 7.10 (m, 3H), 7.02(m, 1H), 2.70(m, 2H), 1.48(m, 18H), 1.44(m, 20H), 1.40(m, 2H), 1.24(m, 18H), 1.01(m, 27H), 0.98 (m, 3H)

### Synthesis Example 77 (Formula 9-11)

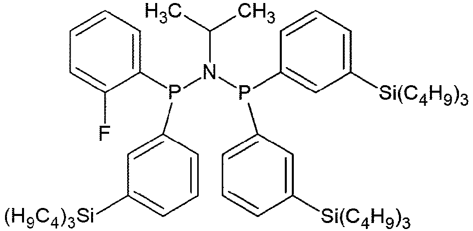

### N-(bis(3-(tributylsilyl)phenyl)phosphaneyl)-1-(2-fluorophenyl)-N-isopropyl-1-(3-(tributylsilyl)phenyl)phosphanamine

¹H NMR (500 MHz, CDCl₃): δ 7.60(m, 3H), 7.43(m, 3H), 7.32(m, 4H), 7.20(m, 1H), 7.17(m, 4H), 7.08(m, 1H), 2.87 (m, 1H), 1.51(m, 18H), 1.44(m, 18H), 1.31(m, 18H), 1.07(m, 6H), 0.95(m, 27H)

### Synthesis Example 78 (Formula 9-12)

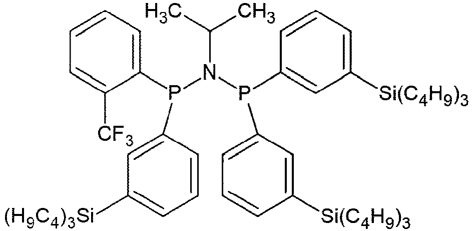

### N-(bis(3-(tributylsilyl)phenyl)phosphaneyl)-N-isopropyl-1-(3-(tributylsilyl)phenyl)-1-(2-(trifluoromethyl)phenyl)phosphanamine

¹H NMR(500 MHz, CDCl₃): δ 7.61(m, 3H), 7.52(m, 1H), 7.50(m, 1H), 7.42(m, 3H), 7.39(m, 3H), 7.23(m, 1H), 7.10(m, 3H), 7.08(m, 1H), 2.83(m, 1H), 1.51(m, 18H), 1.40(m, 18H), 1.27(m, 18H), 1.14(m, 6H), 1.00(m, 27H)

### Synthesis Example 79 (Formula 9-13)

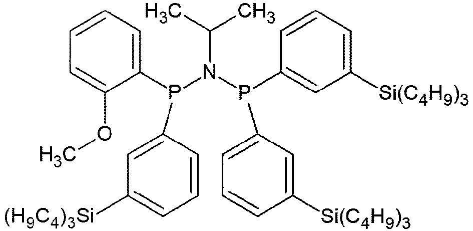

### N-(bis(3-(tributylsilyl)phenyl)phosphaneyl)-N-isopropyl-1-(2-methoxyphenyl)-1-(3-(tributylsilyl)phenyl)phosphanamine

¹H NMR (500 MHz, CDCl₃): δ 7.58 (m, 3H), 7.43 (m, 1H), 7.40 (m, 6H), 7.20(m, 1H), 7.19(m, 1H), 7.13 (m, 3H), 6.97(m, 1H), 3.96(m, 3H), 2.89(m, 1H), 1.46(m, 18H), 1.36(m, 18H), 1.24 (m, 18H), 1.16(m, 6H), 0.94(m, 27H)

### Synthesis Example 80 (Formula 9-14)

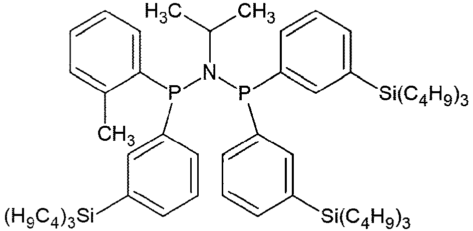

### N-(bis(3-(tributylsilyl)phenyl)phosphaneyl)-N-isopropyl-1-(o-tolyl)-1-(3-(tributylsilyl)phenyl)phosphanamine

¹H NMR(500 MHz, CDCl₃): δ 7.56(m, 3H), 7.48(m, 3H), 7.40(m, 1H), 7.32(m, 3H), 7.27(m, 1H), 7.21(m, 1H), 7.15(m, 3H), 7.09(m, 1H), 2.91(m, 1H), 2.35(m, 3H), 1.47(m, 18H), 1.39(m, 18H), 1.25(m, 18H), 1.13(m, 6H), 0.92(m, 27H)

### Synthesis Example 81 (Formula 9-15)

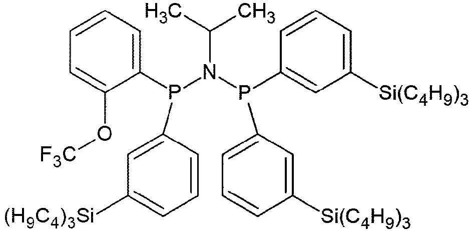

### N-(bis(3-(tributylsilyl)phenyl)phosphaneyl)-N-isopropyl-1-(3-(tributylsilyl)phenyl)-1-(2-(trifluoromethoxy)phenyl)phosphanamine

¹H NMR (500 MHz, CDCl₃): δ 7.60(m, 3H), 7.49 (m, 3H), 7.41(m, 1H), 7.36(m, 3H), 7.17(m, 3H), 7.15(m, 2H), 6.96(m, 1H), 2.87(m, 1H), 1.51(m, 18H), 1.45(m, 18H), 1.27(m, 18H), 1.06(m, 6H), 0.99(m, 27H)

### Synthesis Example 82 (Formula 9-16)

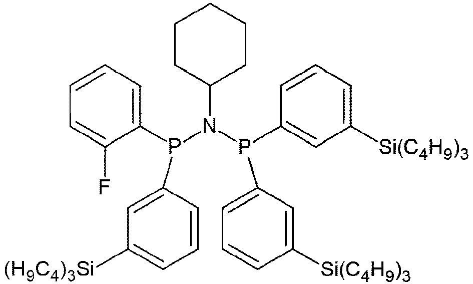

### N-(bis(3-(tributylsilyl)phenyl)phosphaneyl)-N-cyclohexyl-1-(2-fluorophenyl)-1-(3-(tributylsilyl)phenyl)phosphanamine

¹H NMR(500 MHz, CDCl₃): δ 7.62(m, 3H), 7.42 (m, 3H), 7.40 (m, 3H), 7.38(m, 1H), 7.23(m, 1H), 7.17(m, 4H), 7.13(m, 1H), 2.64(m, 1H), 1.75(m, 2H), 1.54(m, 18H), 1.50(m, 2H), 1.49(m, 1H), 1.45(m, 1H), 1.44 (m, 18H), 1.30(m, 18H), 1.22(m, 2H), 1.15(m, 2H), 1.00(m, 27H)

### Synthesis Example 83 (Formula 9-17)

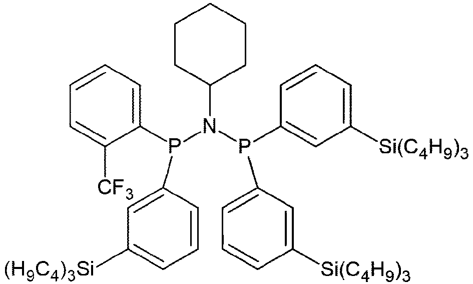

### N-(bis(3-(tributylsilyl)phenyl)phosphaneyl)-N-cyclohexyl-1-(3-(tributylsilyl)phenyl)-1-(2-(trifluoromethyl)phenyl)phosphanamine

¹H NMR(500 MHz, CDCl₃) : δ 7.56(m, 3H), 7.51(m, 1H), 7.44(m, 4H), 7.36(m, 3H), 7.26(m, 1H), 7.13(m, 3H), 7.05(m, 1H), 2.62(m, 1H), 1.80(m, 2H), 1.51(m, 2H), 1.47 (m, 1H), 1.44 (m, 19H), 1.40(m, 18H), 1.29(m, 20H), 1.12(m, 2H), 0.99(m, 27H)

### Synthesis Example 84 (Formula 9-18)

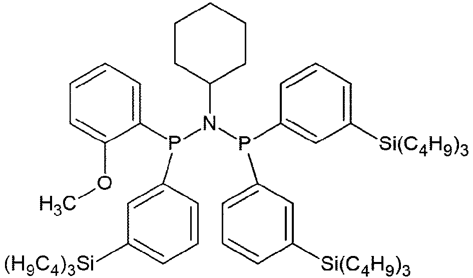

### N-(bis(3-(tributylsilyl)phenyl)phosphaneyl)-N-cyclohexyl-1-(2-methoxyphenyl)-1-(3-(tributylsilyl)phenyl)phosphanamine

¹H NMR (500 MHz, CDCl₃): δ 7.65 (m, 3H), 7.48 (m, 3H), 7.45(m, 1H), 7.32(m, 3H), 7.19(m, 1H), 7.16(m, 3H), 7.14(m, 1H), 7.01(m, 1H), 3.98(m, 3H), 2.66(m, 1H), 1.77(m, 2H), 1.55(m, 2H), 1.52(m, 1H), 1.48(m, 1H), 1.45(m, 18H), 1.44(m, 18H), 1.25(m, 18H), 1.21(m, 2H), 1.17(m, 2H), 0.96(m, 27H)

### Synthesis Example 85 (Formula 9-19)

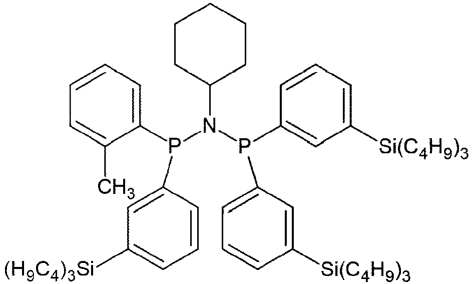

### N-(bis(3-(tributylsilyl)phenyl)phosphaneyl)-N-cyclohexyl-1-(o-tolyl)-1-(3-(tributylsilyl)phenyl)phosphanamine

¹H NMR(500 MHz, CDCl₃): δ 7.60 (m, 3H), 7.43 (m, 3H), 7.40(m, 3H), 7.36 (m, 1H), 7.26 (m, 1H), 7.17 (m, 1H), 7.16 (m, 3H), 7.15(m, 1H), 2.60(m, 1H), 2.39 (m, 3H), 1.80(m, 2H), 1.53(m, 3H), 1.48(m, 18H), 1.47(m, 1H), 1.42 (m, 18H), 1.30(m, 2H), 1.24(m, 18H), 1.24(m, 2H), 0.99(m, 27H)

### Synthesis Example 86 (Formula 9-20)

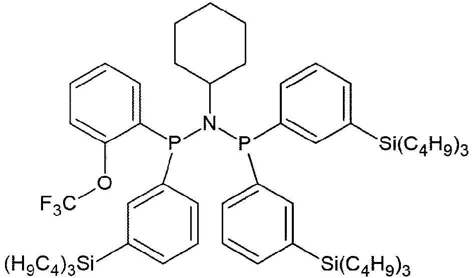

### N-(bis(3-(tributylsilyl)phenyl)phosphaneyl)-N-cyclohexyl-1-(3-(tributylsilyl)phenyl)-1-(2-(trifluoromethoxy)phenyl)phosphanamine

¹H NMR(500 MHz, CDCl₃) : δ 7.62(m, 3H), 7.45(m, 1H), 7.42 (m, 3H), 7.37(m, 3H), 7.19(m, 1H), 7.13(m, 4H), 7.11(m, 3H), 7.05(m, 1H), 2.67 (m, 1H), 1.79(m, 2H), 1.57 (m, 2H), 1.48 (m, 2H), 1.46(m, 18H), 1.40(m, 18H), 1.31(m, 18H), 1.28(m, 2H), 1.18 (m, 2H), 0.96(m, 27H)

### Synthesis Example 87 to 116: Synthesis of ligand compound represented by Formula 2-67 to Formula 2-96

20 mmol (2 eq) of p-bromo-(tri-n-butylsilyl)-C₆H₄ was dissolved in 20 ml of tetrahydrofuran, and then cooled to - 78 °C. While maintaining the temperature, 20 mmol (2 eq) of n-butyllithium was added dropwise thereto, and then stirred for 3 hours. Subsequently, 10 mmol (1 eq) of dichloro(diethylamino)phosphine dissolved in 10 mL of tetrahydrofuran was added dropwise thereto, and then the temperature was raised to room temperature, and the mixture was stirred overnight. Thereafter, the solvent was removed using vacuum, and the obtained intermediate was dissolved in 30 mL of hexane, and then HCl (in ether, 2 eq) was added thereto, stirred for 15 minutes, and filtered. The filtrate was dried in a vacuum state to obtain (p-(tri-n-butylsilyl)-C₆H₄)₂PCl. 11 mmol (2.2 eq) of R⁵-NH₂ and 10 ml of dichloromethane were introduced into a well-dried flask under a nitrogen atmosphere and stirred. 5 mmol (1 eq) of (p-(tri-n-butylsilyl)-C₆H₄)₂PCl was diluted in 10 ml of dichloromethane and then slowly introduced into the flask. After the reaction was completed by stirring for 4 hours, the precipitated solids were removed through a filter, the solvent was removed under reduced pressure, and then (p-(tri-n-butylsilyl)-C₆H₄)₂P-NHR⁵ was obtained through column chromatography.

5 mmol (1 eq) of PhPCl₂ was dissolved in 10 ml of ether. and then cooled to 0°C. Subsequently, 10 mmol (2 eq) of HNEt₂ dissolved in 10 ml of ether was slowly introduced thereto, and stirred overnight. The solids were removed through a filter, and 1 eq of ArMgBr prepared from Ar and Mg pieces (Mg turning) was slowly introduced thereto, and then stirred at room temperature overnight. Thereafter, HCl (in ether, 2 eq) was introduced thereto and stirred for 30 minutes at room temperature. The solid was removed through a filter and the pressure was reduced to obtain PhArPCl. 5 mmol (1 eq) of PhArPCl was dissolved in 10 ml of dichloromethane, and then 15 mmol (3 eq) of triethylamine was added thereto. Subsequently, 5 mmol (1 eq) of (p-(tri-n-butylsilyl)-C₆H₄)₂P-NHR⁵ was dissolved in dichloromethane and then slowly introduced thereto, and the mixture was stirred at room temperature overnight. The solvent was removed using vacuum, dissolved in hexane, and ligand compounds represented by Formula 2-67 to Formula 2-96 were obtained by column chromatography.

R⁵ and Ar, which are substituents of the reactants used in Synthesis Example 97 to Synthesis Example 116, are each shown in Table 3 below, Ph is a phenyl group, and the structure of the ligand compound prepared in each Synthetic Example and ¹H NMR data are described below.

**[Table 3]**

| Classificat ion | R⁵ | Ar(*binding position) |
|---|---|---|
| Synthesis Example 87 | methyl (-CH₃) | 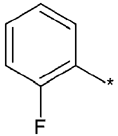 |
| Synthesis Example 88 | methyl (-CH₃) | 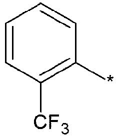 |
| Synthesis Example 89 | methyl (-CH₃) | 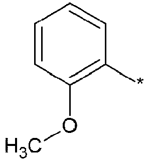 |
| Synthesis Example 90 | methyl (-CH₃) | 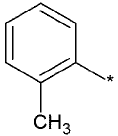 |
| Synthesis Example 91 | methyl (-CH₃) | 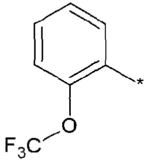 |
| Synthesis Example 92 | n-butyl (-C₄H₉) | 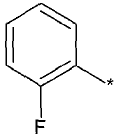 |
| Synthesis Example 93 | n-butyl (-C₄H₉) | 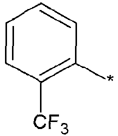 |
| Synthesis Example 94 | n-butyl (-C₄H₉) | 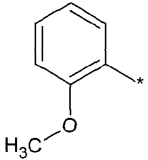 |
| Synthesis Example 95 | n-butyl (-C₄H₉) | 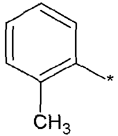 |
| Synthesis Example 96 | n-butyl (-C₄H₉) | 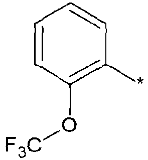 |
| Synthesis Example 97 | iso-propyl(-CH(CH₃)₂) | 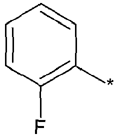 |
| Synthesis Example 98 | iso-propyl(-CH(CH₃)₂) | 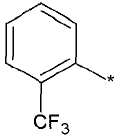 |
| Synthesis Example 99 | iso-propyl(-CH(CH₃)₂) | 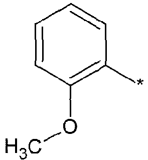 |
| Synthesis Example 100 | iso-propyl (-CH (CH₃)₂) | 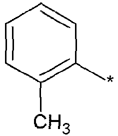 |
| Synthesis Example 101 | iso-propyl(-CH(CH₃)₂) | 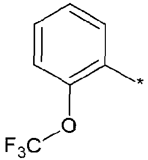 |
| Synthesis Example 102 | cyclohexyl (-C₆H₁₁) | 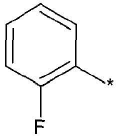 |
| Synthesis Example 103 | cyclohexyl (-C₆H₁₁) | 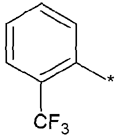 |
| Synthesis Example 104 | cyclohexyl (-C₆H₁₁) | 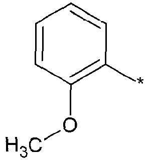 |
| Synthesis Example 105 | cyclohexyl (-C₆H₁₁) | 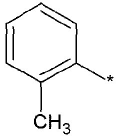 |
| Synthesis Example 106 | cyclohexyl (-C₆H₁₁) | 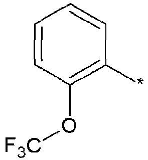 |
| Synthesis Example 107 | benzyl (-CH₂-Ph) | 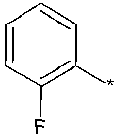 |
| Synthesis Example 108 | benzyl (-CH₂-Ph) | 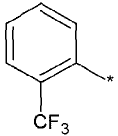 |
| Synthesis Example 109 | benzyl (-CH₂-Ph) | 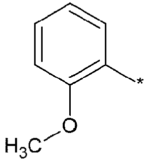 |
| Synthesis Example 110 | benzyl (-CH₂-Ph) | 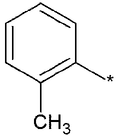 |
| Synthesis Example 111 | benzyl (-CH₂-Ph) | 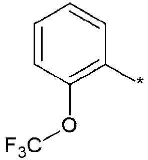 |
| Synthesis Example 112 | phenyl (-Ph) | 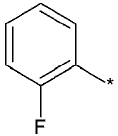 |
| Synthesis Example 113 | phenyl (-Ph) | 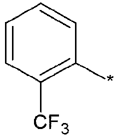 |
| Synthesis Example 114 | phenyl (-Ph) | 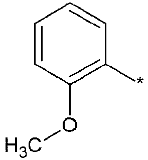 |
| Synthesis Example 115 | phenyl (-Ph) | 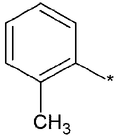 |
| Synthesis Example 116 | phenyl (-Ph) | 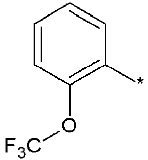 |

### Synthesis Example 87 (Formula 2-67)

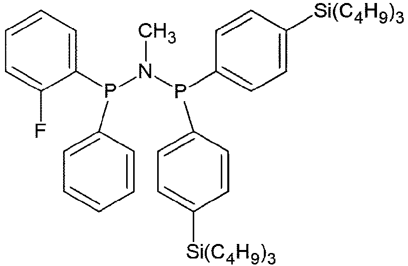

### N-(bis(4-(tributylsilyl)phenyl)phosphaneyl)-1-(2-fluorophenyl)-N-methyl-1-phenylphosphanamine

¹H NMR (500 MHz, CDCl₃): δ 7.49 (m, 3H), 7.45 (m, 4H), 7.36(m, 1H), 7.28(m, 1H), 7.16(m, 2H), 7.13(m, 5H), 7.07(m, 1H), 2.56(m, 3H), 1.47(m, 12H), 1.40(m, 12H), 1.23(m, 12H), 0.94(m, 18H)

### Synthesis Example 88 (Formula 2-68)

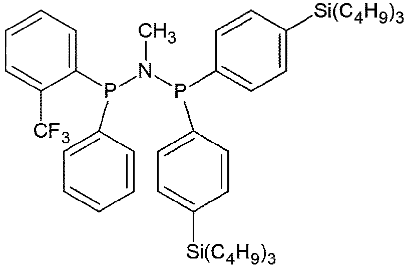

### N-(bis(4-(tributylsilyl)phenyl)phosphaneyl)-N-methyl-1-phenyl-1-(2-(trifluoromethyl)phenyl)phosphanamine

¹H NMR (500 MHz, CDCl₃): δ 7.47 (m, 1H), 7.45 (m, 4H), 7.42(m, 4H), 7.22(m, 1H), 7.12(m, 6H), 7.07(m, 1H), 2.55(m, 3H), 1.45(m, 12H), 1.40(m, 12H), 1.30(m, 12H), 0.96(m, 18H)

### Synthesis Example 89 (Formula 2-69)

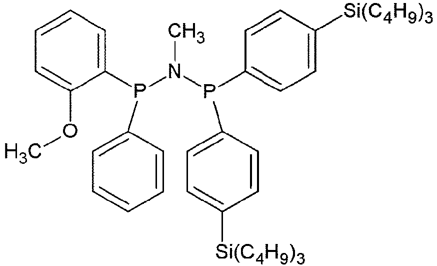

### N-(bis(4-(tributylsilyl)phenyl)phosphaneyl)-1-(2-methoxyphenyl)-N-methyl-1-phenylphosphanamine

¹H NMR (500 MHz, CDCl₃): δ 7.48 (m, 3H), 7.44 (m, 1H), 7.41(m, 4H), 7.18(m, 3H), 7.15(m, 4H), 7.14(m, 1H), 7.02(m, 1H), 3.95(m, 3H), 2.55(m, 3H), 1.45(m, 12H), 1.39(m, 12H), 1.27(m, 12H), 0.98(m, 18H)

### Synthesis Example 90 (Formula 2-70)

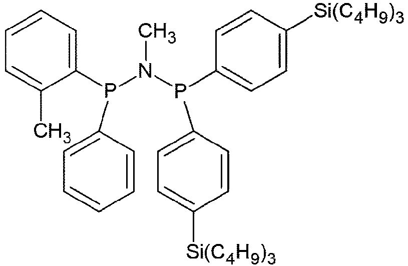

### N-(bis(4-(tributylsilyl)phenyl)phosphaneyl)-N-methyl-1-phenyl-1-(o-tolyl)phosphanamine

¹H NMR (500 MHz, CDCl₃): δ 7.47 (m, 4H), 7.42 (m, 3H), 7.37(m, 1H), 7.26(m, 1H), 7.21(m, 2H), 7.13(m, 1H), 7.12(m, 5H), 2.56(m, 3H), 2.37(m, 3H), 1.45(m, 12H), 1.43(m, 12H), 1.30(m, 12H), 0.98 (m, 18H)

### Synthesis Example 91 (Formula 2-71)

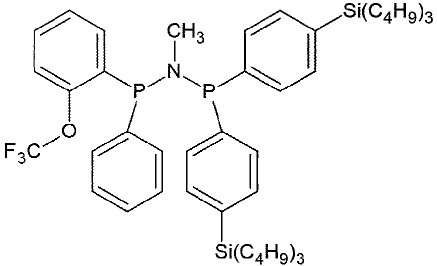

### N-(bis(4-(tributylsilyl)phenyl)phosphaneyl)-N-methyl-1-phenyl-1-(2-(trifluoromethoxy)phenyl)phosphanamine

¹H NMR(500 MHz, CDCl₃): δ 7.46 (m, 3H), 7.42 (m, 4H), 7.41(m, 1H), 7.19(m, 3H), 7.11(m, 1H), 7.10(m, 4H), 7.01(m, 1H), 2.54(m, 3H), 1.51(m, 12H), 1.41(m, 12H), 1.31(m, 12H), 0.97 (m, 18H)

### Synthesis Example 92 (Formula 2-72)

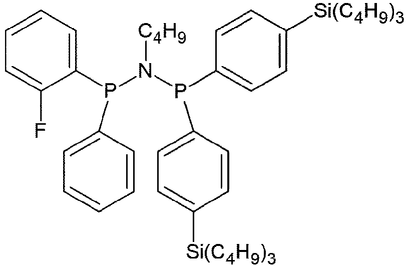

### N-(bis(4-(tributylsilyl)phenyl)phosphaneyl)-N-butyl-1-(2-fluorophenyl)-1-phenylphosphanamine

¹H NMR(500 MHz, CDCl₃): δ 7.49(m, 3H), 7.46(m, 4H), 7.37(m, 1H), 7.24(m, 1H), 7.17(m, 2H), 7.15(m, 5H), 7.14(m, 1H), 2.63(m, 2H), 1.49(m, 12H), 1.45(m, 2H), 1.37(m, 12H), 1.35(m, 2H), 1.30(m, 12H), 0.97(m, 18H), 0.91(m, 3H)

### Synthesis Example 93 (Formula 2-73)

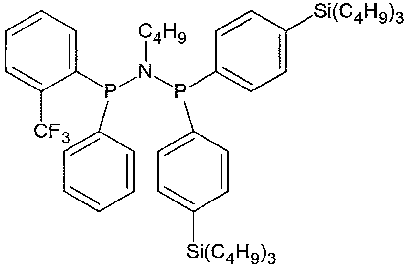

### N-(bis(4-(tributylsilyl)phenyl)phosphaneyl)-N-butyl-1-phenyl-1-(2-(trifluoromethyl)phenyl)phosphanamine

¹H NMR(500 MHz, CDCl₃): δ 7.51 (m, 1H), 7.42 (m, 4H), 7.40(m, 4H), 7.24(m, 1H), 7.19(m, 4H), 7.15(m, 2H), 7.07(m, 1H), 2.68(m, 2H), 1.44(m, 14H), 1.40(m, 12H), 1.32(m, 2H), 1.29(m, 12H), 0.98(m, 3H), 0.95(m, 18H)

### Synthesis Example 94 (Formula 2-74)

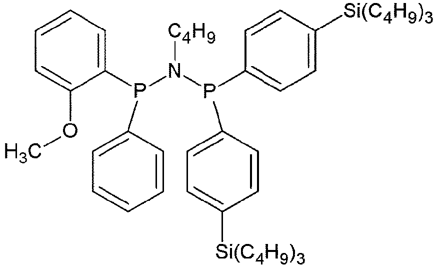

### N-(bis(4-(tributylsilyl)phenyl)phosphaneyl)-N-butyl-1-(2-methoxyphenyl)-1-phenylphosphanamine

¹H NMR (500 MHz, CDCl₃): δ 7.44 (m, 3H), 7.43 (m, 4H), 7.41 (m, 1H), 7.18(m, 4H), 7.15(m, 1H), 7.11(m, 3H), 7.02(m, 1H), 3.95(m, 3H), 2.71(m, 2H), 1.51 (m, 12H), 1.45(m, 2H), 1.41(m, 2H), 1.39(m, 12H), 1.26(m, 12H), 1.02(m, 18H), 0.92 (m, 3H)

### Synthesis Example 95 (Formula 2-75)

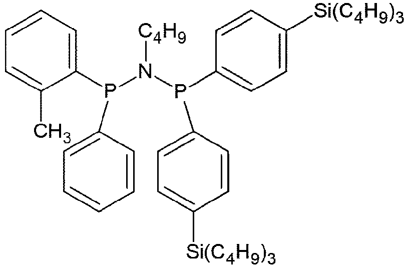

### N-(bis(4-(tributylsilyl)phenyl)phosphaneyl)-N-butyl-1-phenyl-1-(o-tolyl)phosphanamine

¹H NMR (500 MHz, CDCl₃): δ 7.48 (m, 4H), 7.45 (m, 3H), 7.33(m, 1H), 7.21(m, 1H), 7.20(m, 3H), 7.17(m, 4H), 7.14 (m, 1H), 2.64(m, 2H), 2.38(m, 3H), 1.51(m, 12H), 1.46(m, 2H), 1.39(m, 12H), 1.33(m, 2H), 1.30(m, 12H), 0.97 (m, 3H), 0.92 (m, 18H)

### Synthesis Example 96 (Formula 2-76)

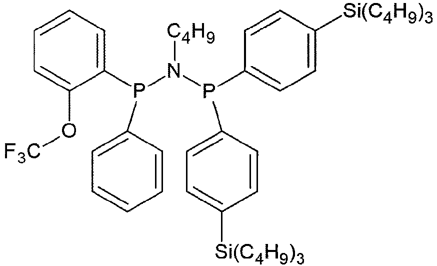

### N-(bis(4-(tributylsilyl)phenyl)phosphaneyl)-N-butyl-1-phenyl-1-(2-(trifluoromethoxy)phenyl)phosphanamine

¹H NMR (500 MHz, CDCl₃): δ 7.46 (m, 3H), 7.45 (m, 4H), 7.39(m, 1H), 7.18(m, 3H), 7.13(m, 1H), 7.12(m, 4H), 7.02 (m, 1H), 2.66(m, 2H), 1.50(m, 12H), 1.47(m, 2H), 1.41(m, 12H), 1.39(m, 2H), 1.26(m, 12H), 1.02(m, 18H), 0.91(m, 3H)

### Synthesis Example 97 (Formula 2-77)

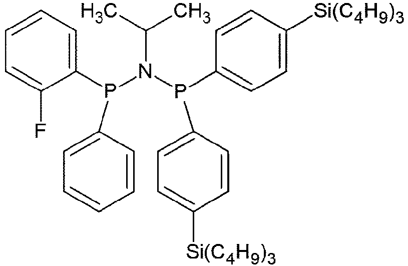

### N-(bis(4-(tributylsilyl)phenyl)phosphaneyl)-1-(2-fluorophenyl)-N-isopropyl-1-phenylphosphanamine

¹H NMR(500 MHz, CDCl₃): δ 7.47 (m, 4H), 7.46(m, 3H), 7.34(m, 1H), 7.20(m, 1H), 7.17(m, 5H), 7.12(m, 2H), 7.10(m, 1H), 2.86(m, 1H), 1.45(m, 12H), 1.37(m, 12H), 1.28(m, 12H), 1.08(m, 6H), 1.02(m, 18H)

### Synthesis Example 98 (Formula 2-78)

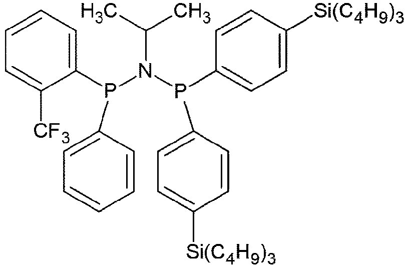

### N-(bis(4-(tributylsilyl)phenyl)phosphaneyl)-N-isopropyl-1-phenyl-1-(2-(trifluoromethyl)phenyl)phosphanamine

¹H NMR (500 MHz, CDCl₃): δ 7.46(m, 1H), 7.43 (m, 8H), 7.19(m, 1H), 7.14(m, 2H), 7.11(m, 4H), 7.07(m, 1H), 2.85(m, 1H), 1.50(m, 12H), 1.44(m, 12H), 1.28(m, 12H), 1.08(m, 6H), 1.00(m, 18H)

### Synthesis Example 99 (Formula 2-79)

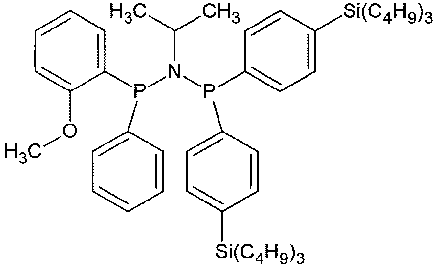

### N-(bis(4-(tributylsilyl)phenyl)phosphaneyl)-N-isopropyl-1-(2-methoxyphenyl)-1-phenylphosphanamine

¹H NMR (500 MHz, CDCl₃): δ 7.51 (m, 3H), 7.49 (m, 4H), 7.45(m, 1H), 7.18(m, 4H), 7.15(m, 3H), 7.13(m, 1H), 6.99(m, 1H), 4.00(m, 3H), 2.88(m, 1H), 1.52(m, 12H), 1.43(m, 12H), 1.31(m, 12H), 1.08(m, 6H), 0.94(m, 18H)

### Synthesis Example 100 (Formula 2-80)

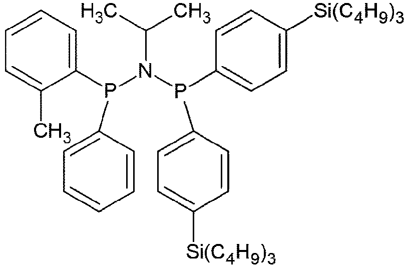

### N-(bis(4-(tributylsilyl)phenyl)phosphaneyl)-N-isopropyl-1-phenyl-1-(o-tolyl)phosphanamine

¹H NMR (500 MHz, CDCl₃): δ 7.45 (m, 4H), 7.44 (m, 3H), 7.40 (m, 1H), 7.22(m, 1H), 7.17(m, 1H), 7.16(m, 2H), 7.13(m, 1H), 7.11(m, 4H), 2.87(m, 1H), 2.33(m, 3H), 1.44 (m, 12H), 1.41 (m, 12H), 1.28 (m, 12H), 1.12(m, 6H), 0.93(m, 18H)

### Synthesis Example 101 (Formula 2-81)

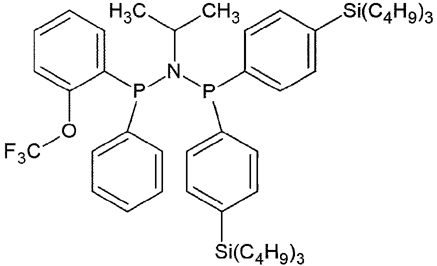

### N-(bis(4-(tributylsilyl)phenyl)phosphaneyl)-N-isopropyl-1-phenyl-1-(2-(trifluoromethoxy)phenyl)phosphanamine

¹H NMR(500 MHz, CDCl₃): δ 7.44 (m, 3H), 7.43 (m, 4H), 7.37(m, 1H), 7.16(m, 1H), 7.14(m, 3H), 7.12(m, 4H), 7.01(m, 1H), 2.89(m, 4H), 1.52(m, 12H), 1.37(m, 12H), 1.32(m, 12H), 1.07(m, 6H), 0.92(m, 18H)

### Synthesis Example 102 (Formula 2-82)

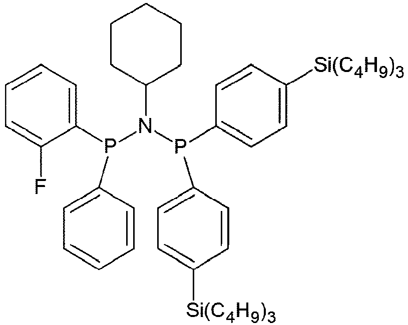

### N-(bis(4-(tributylsilyl)phenyl)phosphaneyl)-N-cyclohexyl-1-(2-fluorophenyl)-1-phenylphosphanamine

¹H NMR(500 MHz, CDCl₃): δ 7.49(m, 4H), 7.45(m, 3H), 7.40(m, 1H), 7.22(m, 1H), 7.21(m, 2H), 7.13(m, 1H), 7.10(m, 5H), 2.61(m, 1H), 1.80(m, 2H), 1.56(m, 2H), 1.53(m, 12H), 1.49(m, 1H), 1.46(m, 1H), 1.41(m, 12H), 1.27(m, 12H), 1.21(m, 2H), 1.14(m, 2H), 0.92(m, 18H)

### Synthesis Example 103 (Formula 2-83)

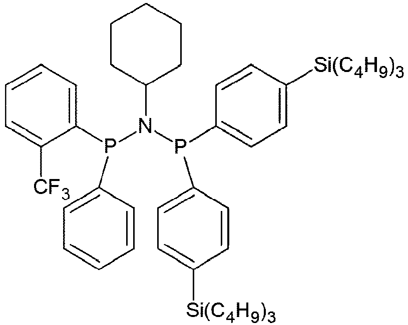

### N-(bis(4-(tributylsilyl)phenyl)phosphaneyl)-N-cyclohexyl-1-phenyl-1-(2-(trifluoromethyl)phenyl)phosphanamine

¹H NMR(500 MHz, CDCl₃): δ 7.50 (m, 4H), 7.47 (m, 4H), 7.46(m, 1H), 7.22(m, 1H), 7.16(m, 2H), 7.12(m, 4H), 7.06(m, 1H), 2.61 (m, 1H), 1.76(m, 2H), 1.52(m, 2H), 1.51(m, 1H), 1.50(m, 13H), 1.39(m, 12H), 1.25(m, 12H), 1.24(m, 2H), 1.14(m, 2H), 0.92(m, 18H)

### Synthesis Example 104 (Formula 2-84)

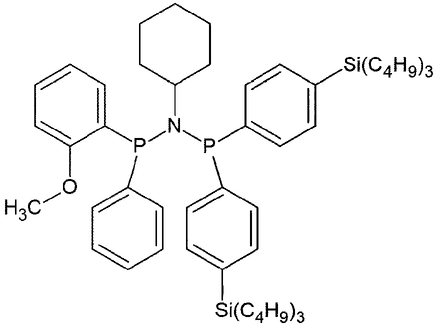

### N-(bis(4-(tributylsilyl)phenyl)phosphaneyl)-N-cyclohexyl-1-(2-methoxyphenyl)-1-phenylphosphanamine

¹H NMR(500 MHz, CDCl₃): δ 7.48 (m, 3H), 7.44 (m, 1H), 7.43 (m, 4H), 7.18(m, 3H), 7.12(m, 4H), 7.11(m, 1H), 6.96(m, 1H), 4.00(m, 3H), 2.61(m, 1H), 1.78(m, 2H), 1.52(m, 2H), 1.49(m, 1H), 1.48(m, 1H), 1.45(m, 12H), 1.41(m, 12H), 1.32(m, 12H), 1.27(m, 2H), 1.13(m, 2H), 0.99(m, 18H)

### Synthesis Example 105 (Formula 2-85)

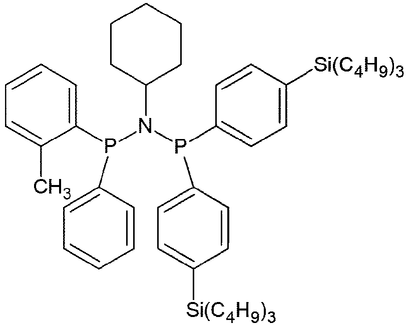

### N-(bis(4-(tributylsilyl)phenyl)phosphaneyl)-N-cyclohexyl-1-phenyl-1-(o-tolyl)phosphanamine

¹H NMR(500 MHz, CDCl₃): δ 7.49(m, 7H), 7.37(m, 1H), 7.22(m, 1H), 7.21(m, 1H), 7.16(m, 4H), 7.15(m, 1H), 7.11(m, 2H), 2.64(m, 1H), 2.41(m, 3H), 1.83(m, 2H), 1.51(m, 12H), 1.50(m, 2H), 1.49(m, 1H), 1.48(m, 1H), 1.39(m, 12H), 1.28(m, 12H), 1.27(m, 2H), 1.18(m, 2H), 0.94(m, 18H)

### Synthesis Example 106 (Formula 2-86)

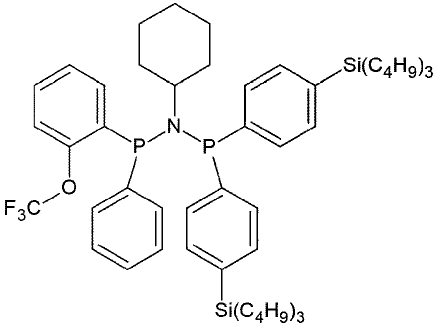

### N-(bis(4-(tributylsilyl)phenyl)phosphaneyl)-N-cyclohexyl-1-phenyl-1-(2-(trifluoromethoxy)phenyl)phosphanamine

¹H NMR(500 MHz, CDCl₃): δ 7.50(m, 3H), 7.46 (m, 4H), 7.40 (m, 1H), 7.18(m, 4H), 7.17(m, 3H), 7.12(m, 1H), 7.03(m, 1H), 2.64 (m, 1H), 1.81(m, 2H), 1.58(m, 2H), 1.52(m, 1H), 1.47(m, 13H), 1.41(m, 12H), 1.29(m, 2H), 1.24(m, 12H), 1.16(m, 2H), 1.01(m, 18H)

### Synthesis Example 107 (Formula 2-87)

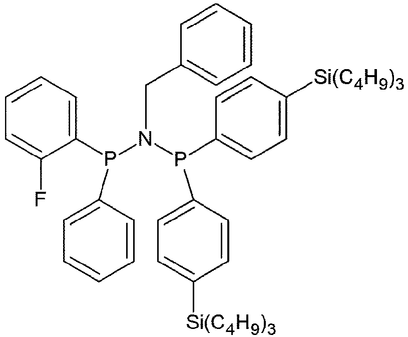

### N-benzyl-N-(bis(4-(tributylsilyl)phenyl)phosphaneyl)-1-(2-fluorophenyl)-1-phenylphosphanamine

¹H NMR (500 MHz, CDCl₃): δ 7.50 (m, 3H), 7.41 (m, 4H), 7.35(m, 4H), 7.33(m, 2H), 7.26(m, 1H), 7.17(m, 2H), 7.14(m, 1H), 7.13(m, 5H), 3.94(m, 2H), 1.51(m, 12H), 1.44 (m, 12H), 1.24(mm, 12H), 0.96(m, 18H)

### Synthesis Example 108 (Formula 2-88)

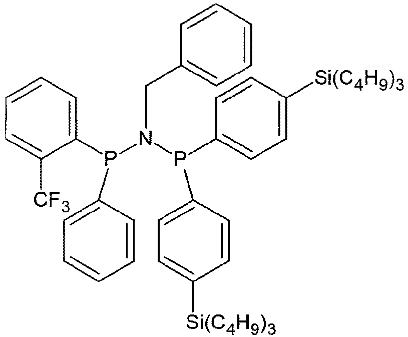

### N-benzyl-N-(bis(4-(tributylsilyl)phenyl)phosphaneyl)-1-phenyl-1-(2-(trifluoromethyl)phenyl)phosphanamine

¹H NMR(500 MHz, CDCl₃): δ 7.50 (m, 4H), 7.47(m, 1H), 7.45(m, 4H), 7.35(m, 4H), 7.33(m, 1H), 7.20(m, 2H), 7.19(m, 1H), 7.10(m, 4H), 7.04(m, 1H), 3.94(m, 2H), 1.51(m, 12H), 1.43 (m, 12H), 1.31(m, 12H), 0.95(m, 18H)

### Synthesis Example 109 (Formula 2-89)

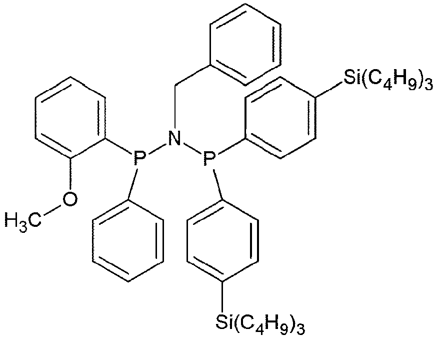

### N-benzyl-N-(bis(4-(tributylsilyl)phenyl)phosphaneyl)-1-(2-methoxyphenyl)-1-phenylphosphanamine

¹H NMR(500 MHz, CDCl₃): δ 7.48 (m, 4H), 7.45 (m, 3H), 7.41(m, 1H), 7.33(m, 5H), 7.15(m, 3H), 7.14(m, 4H), 7.11(m, 1H), 7.05(m, 1H), 3.95(m, 5H), 1.50(m, 12H), 1.46(m, 12H), 1.27(m, 12H), 0.92(m, 18H)

### Synthesis Example 110 (Formula 2-90)

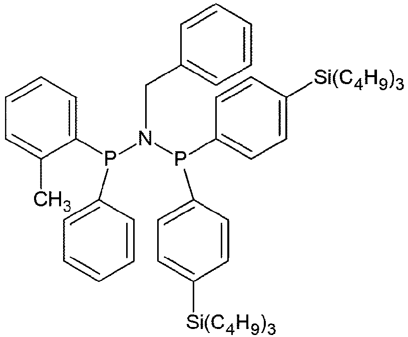

### N-benzyl-N-(bis(4-(tributylsilyl)phenyl)phosphaneyl)-1-phenyl-1-(o-tolyl)phosphanamine

¹H NMR (500 MHz, CDCl₃): δ 7.43 (m, 3H), 7.42 (m, 4H), 7.37(m, 1H), 7.35(m, 5H), 7.20(m, 1H), 7.16(m, 1H), 7.14 (m, 2H), 7.12 (m, 4H), 7.09(m, 1H), 3.99(m, 2H), 2.34(m, 3H), 1.46(m, 12H), 1.42 (m, 12H), 1.32(m, 12H), 0.92(m, 18H)

### Synthesis Example 111 (Formula 2-91)

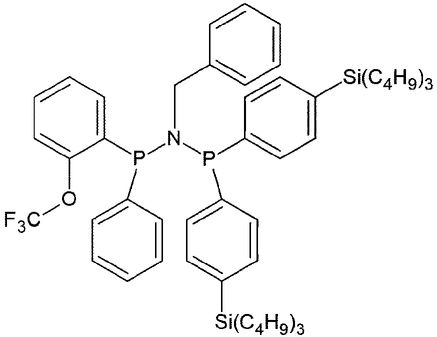

### N-benzyl-N-(bis(4-(tributylsilyl)phenyl)phosphaneyl)-1-phenyl-1-(2-(trifluoromethoxy)phenyl)phosphanamine

¹H NMR(500 MHz, CDCl₃): δ 7.51(m, 3H), 7.47 (m, 4H), 7.43 (m, 1H), 7.32(m, 1H), 7.31(m, 4H), 7.20(m, 1H), 7.17(m, 3H), 7.13(m, 4H), 6.97(m, 1H), 3.98(m, 2H), 1.52(m, 12H), 1.39(m, 12H), 1.30(m, 12H), 0.96(m, 18H)

### Synthesis Example 112 (Formula 2-92)

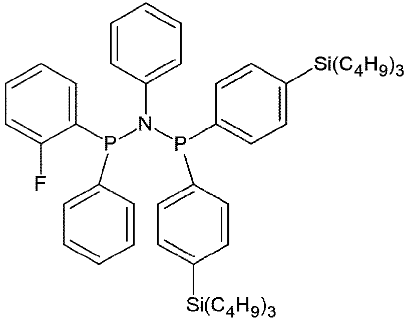

### N-(bis(4-(tributylsilyl)phenyl)phosphaneyl)-1-(2-fluorophenyl)-N,1-diphenyl-phosphanamine

¹H NMR (500 MHz, CDCl₃): δ 7.44 (m, 3H), 7.42 (m, 4H), 7.31(m, 1H), 7.23(m, 1H), 7.19(m, 2H), 7.16(m, 5H), 7.12(m, 2H), 7.09(m, 1H), 6.83(m, 1H), 6.68(m, 2H), 1.50(m, 12H), 1.45(m, 12H), 1.23(m, 12H), 0.94(m, 18H)

### Synthesis Example 113 (Formula 2-93)

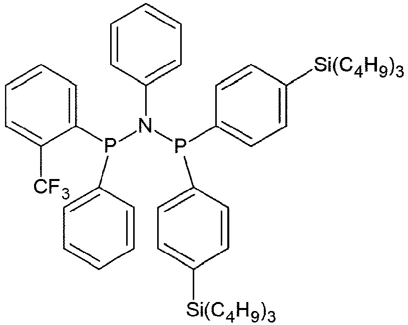

### N-(bis(4-(tributylsilyl)phenyl)phosphaneyl)-N,1-diphenyl-1-(2-(trifluoromethyl)phenyl)phosphanamine

¹H NMR (500 MHz, CDCl₃): δ 7.49(m, 1H), 7.46(m, 4H), 7.44(m, 4H), 7.20(m, 1H), 7.17(m, 4H), 7.14(m, 1H), 7.13(m, 4H), 6.83(m, 1H), 6.62 (m, 2H), 1.48 (m, 12H), 1.36(m, 12H), 1.31(m, 12H), 1.00(m, 18H)

### Synthesis Example 114 (Formula 2-94)

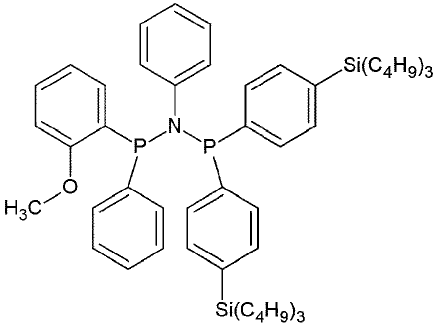

### N-(bis(4-(tributylsilyl)phenyl)phosphaneyl)-1-(2-methoxyphenyl)-N,1-diphenylphosphanamine

¹H NMR(500 MHz, CDCl₃): δ 7.50(m, 3H), 7.44 (m, 1H), 7.43 (m, 4H), 7.15(m, 1H), 7.12(m, 6H), 7.11(m, 3H), 7.03(m, 1H), 6.87(m, 1H), 6.68(m, 2H), 3.93(m, 3H), 1.51(m, 12H), 1.37(m, 12H), 1.23(m, 12H), 0.99(m, 18H)

### Synthesis Example 115 (Formula 2-95)

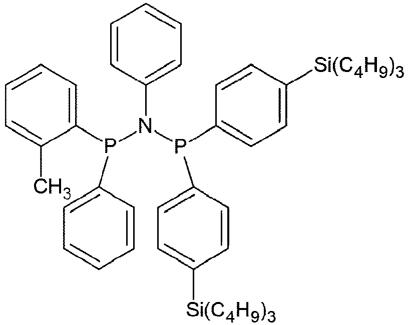

### N-(bis(4-(tributylsilyl)phenyl)phosphaneyl)-N,1-diphenyl-1-(o-tolyl)phosphanamine

¹H NMR(500 MHz, CDCl₃): δ 7.45(m, 3H), 7.41(m, 4H), 7.37(m, 1H), 7.20(m, 1H), 7.18(m, 1H), 7.17 (m, 4H), 7.14(m, 3H), 7.11(m, 2H), 6.86(m, 1H), 6.68(m, 2H), 2.32(m, 3H), 1.48(m, 12H), 1.42(m, 12H), 1.25(m, 12H), 0.93(m, 18H)

### Synthesis Example 116 (Formula 2-96)

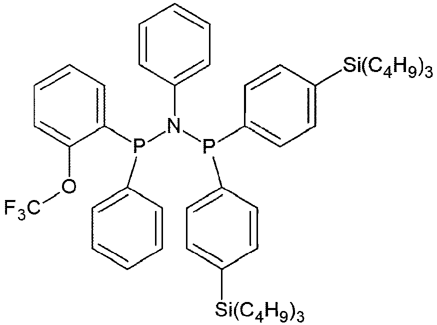

### N-(bis(4-(tributylsilyl)phenyl)phosphaneyl)-N,1-diphenyl-1-(2-(trifluoromethoxy)phenyl)phosphanamine

¹H NMR(500 MHz, CDCl₃): δ 7.46(m, 4H), 7.45 (m, 3H), 7.43(m, 1H), 7.16(m, 4H), 7.14(m, 3H), 7.13(m, 1H), 7.10(m, 2H), 6.99(m, 1H), 6.77(m, 1H), 6.63(m, 2H), 1.46(m, 12H), 1.44(m, 12H), 1.30(m, 12H), 0.94(m, 18H)

### Comparative Synthesis Example 1: Synthesis of ligand compound represented by Formula 14

5.5 mmol (0.5 eq) of 3-methyl-2-butanamine, 22 mmol (2 eq) of triethylamine, and 10 ml of dichloromethane were introduced into a dried flask under a nitrogen atmosphere, and stirred. Subsequently, the mixture was cooled to 0 °C, and then 11 mmol (1 eq) of chlorobis(4-(tributylsilyl)phenyl)phosphane was slowly introduced thereto. After the reaction was completed, the precipitated solids were removed through a filter, the solvent was removed under reduced pressure, and then a compound represented by Formula 14 was obtained through column chromatography.

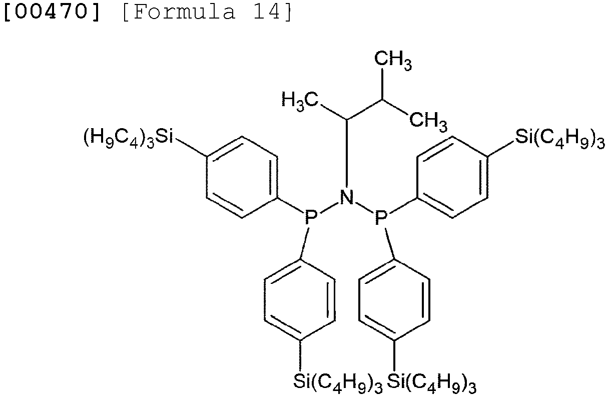

### N-(bis(4-(tributylsilyl)phenyl)phosphaneyl)-N-(3-methylbutan-2-yl)-1,1-bis(4-(tributylsilyl)phenyl)phosphanamine

¹H NMR(500 MHz, C₆D₆): δ 7.96-7.34(m, 16H), 3.64-3.53(m, 1H), 1.81-1.72(m, 1H), 1.38-1.29(m, 48H), 1.05(d, 3H), 0.91-0.84(m, 39H), 0.82-0.76(m, 24H), 0.55(d, 3H)

### Comparative Synthesis Example 2: Synthesis of ligand compound represented by Formula 15

5.5 mmol (0.5 eq) of cyclohexylamine, 22 mmol (2 eq) of triethylamine, and 10 ml of dichloromethane were introduced into a dried flask under a nitrogen atmosphere, and stirred. Subsequently, the mixture was cooled to 0 °C, and then 11 mmol (1 eq) of chlorobis (4-cyclohexylphenyl) phosphane was slowly introduced thereto. After the reaction was completed, the precipitated solids were removed through a filter, the solvent was removed under reduced pressure, and then a compound represented by Formula 15 was obtained through column chromatography.

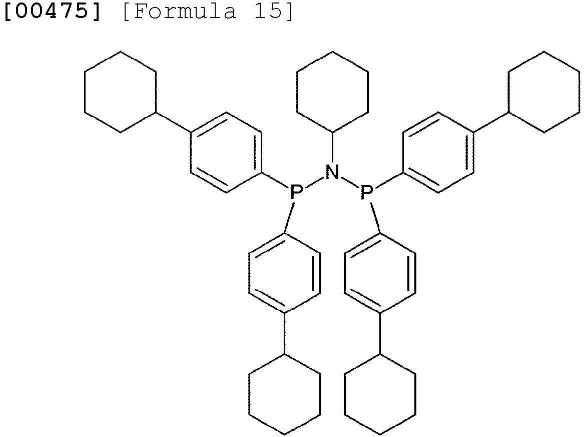

### N-(bis(4-cyclohexylphenyl)phosphaneyl)-N-cyclohexyl-1,1-bis(4-cyclohexylphenyl)phosphanamine

¹H NMR(500 MHz, C₆D₆): δ 7.58 (br.s, 8H), 7.08 (d, 8H), 3.47(pent, 1H), 2.35(t, 4H), 2.21-2.11(m, 2H), 1.80(d, 8H), 1.71-1.56(m, 16H), 1.44-1.11(m, 24H)

### Comparative Synthesis Example 3: Synthesis of ligand compound represented by Formula 16

5.5 mmol (0.5 eq) of cyclohexylamine, 22 mmol (2 eq) of triethylamine, and 10 ml of dichloromethane were introduced into a dried flask under a nitrogen atmosphere, and stirred. Subsequently, the mixture was cooled to 0 °C, and then 11 mmol (1 eq) of chlorobis(4-phenylphenyl)phosphane was slowly introduced thereto. After the reaction was completed, the precipitated solids were removed through a filter, the solvent was removed under reduced pressure, and then a compound represented by Formula 16 was obtained through column chromatography.

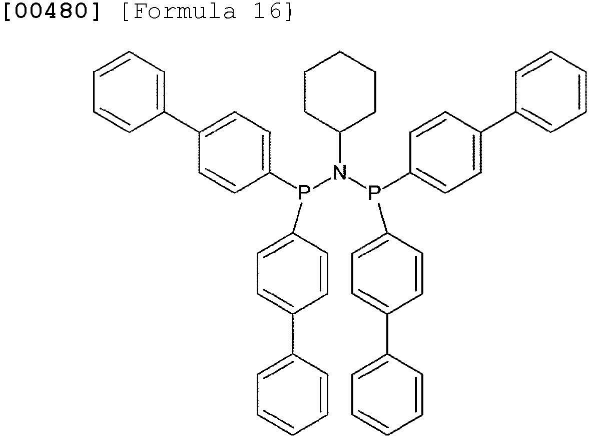

### 1,1-di([1,1'-biphenyl]-4-yl)-N-cyclohexyl-N-(di([1,1'-biphenyl]-4-yl)phosphaneyl)phosphanamine

¹H NMR(500 MHz, C₆D₆): δ 7.92-7.42 (m, 24H), 7.17(t, 8H), 7.10(t, 4H), 3.58(pent, 1H), 2.28-2.17(m, 2H), 1.84-1.77(m, 2H), 1.67-1.60(m, 2H), 1.48-1.42(m, 1H), 1.18-1.07(m, 3H)

### Comparative Synthesis Example 4: Synthesis of ligand compound represented by Formula 17

5.5 mmol (0.5 eq) of cyclohexylamine, 22 mmol (2 eq) of triethylamine, and 10 ml of dichloromethane were introduced into a dried flask under a nitrogen atmosphere, and stirred. Subsequently, the mixture was cooled to 0 °C, and then 11 mmol (1 eq) of chlorobis(3,5-dimethylphenyl)phosphane was slowly introduced thereto. After the reaction was completed, the precipitated solids were removed through a filter, the solvent was removed under reduced pressure, and then a compound represented by Formula 17 was obtained through column chromatography.

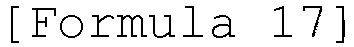

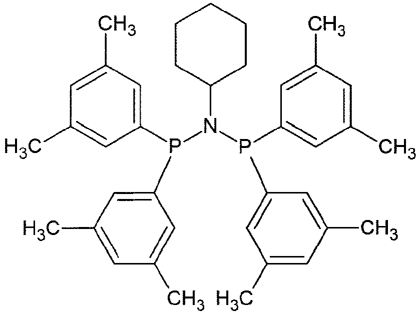

### N-(bis(3,5-dimethylphenyl)phosphaneyl)-N-cyclohexyl-1,1-bis(3,5-dimethylphenyl)phosphanamine

¹H NMR(500 MHz, C₆D₆): δ 7.38(br.s, 8H), 6.79(s, 4H), 3.63(pent, 1H), 2.24-2.13(m, 2H), 2.10(s, 24H), 1.77(d, 2H), 1.60(d, 2H), 1.45-1.39(m, 1H), 1.19-1.00(m, 3H)

### Examples and Comparative Examples

### Example 1

Under an argon gas atmosphere, 0.5 mmol of chromium(III) chloride tetrahydrofuran (Cr (THF) ₃Cl₃), 0.5 mmol of a ligand compound represented by Formula 2-1 according to Synthesis Example 1, and as a co-catalyst, 0.5 mmol of N,N-dimethylanilinium tetrakis(pentafluorophenyl)borate (AB) were put into a flask, and then 30 ml of dichloromethane was introduced thereto, and stirred for 1 hour, and the solvent was removed using vacuum. Thereafter, the mixture was dissolved in methylcyclohexane and filtered, and then the solvent was removed again using vacuum, and dissolved in methylcyclohexane to prepare 0.15 mM (based on Cr) of a catalyst solution.

A Parr reactor with a capacity of 600 ml was prepared and vacuumed at 120 °C for 2 hours, and then the inside of the reactor was substituted with argon and the temperature was lowered to 70 °C. Thereafter, 180 ml of methylcyclohexane, and as 2 ml of an activator, 725 µmol of diisobutyl aluminum hydride were injected thereto, and 5 ml (0.75 µmol Cr) of the catalyst solution was injected thereto. After two minutes of stirring at 1,000 rpm, the valve of an ethylene line which was set to 40 bar was opened to fill the reactor with ethylene, and then stirring was performed for 60 minutes at 1,000 rpm. The ethylene line valve was closed, and the reactor was cooled to 0 °C using a dry ice/acetone bath, and then unreacted ethylene was slowly vented, and 0.5 ml of nonane (GC internal standard) was added thereto. After stirring was performed for 10 seconds, 2 ml of a liquid portion of the reactor was taken and quenched with water, and an obtained organic portion was filtered with a PTFE syringe filter to prepare a GC-FID sample. Then, the distribution of a liquid product was analyzed by GC (Agilent Co., 6890N, Alltech AT-5 (30 m X 0.32 mm ID X 0.25 µm; series no. 12446)). In addition, 400 ml of ethanol/HCl (10 vol% of aqueous 12 M HCl solution) was added to the remaining reaction solution, stirred, and filtered to analyze the amount of a solid product. The obtained polymer was dried in a 80 °C vacuum oven overnight.

### Examples 2 to 116 and Comparative Examples 1 to 4

Examples 2 to 116 and Comparative Examples 1 to 4 were executed in the same manner as in Example 1-1 above, except that the catalyst type was changed as shown in Table 4 below.

**[Table 4]**

| Classificati on | Ligand compound | Co-catalyst | **Chromium** source |
|---|---|---|---|
| Example 1 | Synthesis Example 1 (Formula 2-1) | AB | Cr (THF)₃Cl₃ |
| Example 2 | Synthesis Example 2 (Formula 2-2) | AB | Cr (THF)₃Cl₃ |
| Example 3 | Synthesis Example 3 (Formula 2-3) | AB | Cr (THF)₃Cl₃ |
| Example 4 | Synthesis Example 4 (Formula 2-4) | AB | Cr (THF)₃Cl₃ |
| Example 5 | Synthesis Example 5 (Formula 2-5) | AB | Cr (THF)₃Cl₃ |
| Example 6 | Synthesis Example 6 (Formula 2-6) | AB | Cr (THF)₃Cl₃ |
| Example 7 | Synthesis Example 7 (Formula 2-7) | AB | Cr (THF)₃Cl₃ |
| Example 8 | Synthesis Example 8 (Formula 2-8) | AB | Cr (THF)₃Cl₃ |
| Example 9 | Synthesis Example 9 (Formula 2-9) | AB | Cr (THF)₃Cl₃ |
| Example 10 | Synthesis Example 10 (Formula 2-10) | AB | Cr (THF)₃Cl₃ |
| Example 11 | Synthesis Example 11 (Formula 2-11) | AB | Cr (THF)₃Cl₃ |
| Example 12 | Synthesis Example 12 (Formula 2-12) | AB | Cr (THF)₃Cl₃ |
| Example 13 | Synthesis Example 13 (Formula 2-13) | AB | Cr (THF)₃Cl₃ |
| Example 14 | Synthesis Example 14 (Formula 2-14) | AB | Cr (THF)₃Cl₃ |
| Example 15 | Synthesis Example 15 (Formula 2-15) | AB | Cr (THF)₃Cl₃ |
| Example 16 | Synthesis Example 16 (Formula 2-16) | AB | Cr (THF)₃Cl₃ |
| Example 17 | Synthesis Example 17 (Formula 2-17) | AB | Cr (THF)₃Cl₃ |
| Example 18 | Synthesis Example 18 (Formula 2-18) | AB | Cr (THF)₃Cl₃ |
| Example 19 | Synthesis Example 19 (Formula 2-19) | AB | Cr (THF)₃Cl₃ |
| Example 20 | Synthesis Example 20 (Formula 2-20) | AB | Cr (THF)₃Cl₃ |
| Example 21 | Synthesis Example 21 (Formula 2-21) | AB | Cr (THF)₃Cl₃ |
| Example 22 | Synthesis Example 22 (Formula 2-22) | AB | Cr (THF)₃Cl₃ |
| Example 23 | Synthesis Example 23 (Formula 2-23) | AB | Cr (THF)₃Cl₃ |
| Example 24 | Synthesis Example 24 (Formula 2-24) | AB | Cr (THF)₃Cl₃ |
| Example 25 | Synthesis Example 25 (Formula 2-25) | AB | Cr (THF)₃Cl₃ |
| Example 26 | Synthesis Example 26 (Formula 2-26) | AB | Cr (THF)₃Cl₃ |
| Example 27 | Synthesis Example 27 (Formula 2-27) | AB | Cr (THF)₃Cl₃ |
| Example 28 | Synthesis Example 28 (Formula 2-28) | AB | Cr (THF)₃Cl₃ |
| Example 29 | Synthesis Example 29 (Formula 2-29) | AB | Cr (THF)₃Cl₃ |
| Example 30 | Synthesis Example 30 (Formula 2-30) | AB | Cr (THF)₃Cl₃ |
| Example 31 | Synthesis Example 31 (Formula 2-31) | AB | Cr (THF)₃Cl₃ |
| Example 32 | Synthesis Example 32 (Formula 2-32) | AB | Cr (THF)₃Cl₃ |
| Example 33 | Synthesis Example 33 (Formula 2-33) | AB | Cr (THF)₃Cl₃ |
| Example 34 | Synthesis Example 34 (Formula 2-34) | AB | Cr (THF)₃Cl₃ |
| Example 35 | Synthesis Example 35 (Formula 2-35) | AB | Cr (THF)₃Cl₃ |
| Example 36 | Synthesis Example 36 (Formula 2-36) | AB | Cr (THF)₃Cl₃ |
| Example 37 | Synthesis Example 37 (Formula 2-37) | AB | Cr (THF)₃Cl₃ |
| Example 38 | Synthesis Example 38 (Formula 2-38) | AB | Cr (THF)₃Cl₃ |
| Example 39 | Synthesis Example 39 (Formula 2-39) | AB | Cr (THF)₃Cl₃ |
| Example 40 | Synthesis Example 40 (Formula 2-40) | AB | Cr (THF)₃Cl₃ |
| Example 41 | Synthesis Example 41 (Formula 2-41) | AB | Cr (THF)₃Cl₃ |
| Example 42 | Synthesis Example 42 (Formula 2-42) | AB | Cr (THF)₃Cl₃ |
| Example 43 | Synthesis Example 43 (Formula 2-43) | AB | Cr (THF)₃Cl₃ |
| Example 44 | Synthesis Example 44 (Formula 2-44) | AB | Cr (THF)₃Cl₃ |
| Example 45 | Synthesis Example 45 (Formula 2-45) | AB | Cr (THF)₃Cl₃ |
| Example 46 | Synthesis Example 46 (Formula 2-46) | AB | Cr (THF)₃Cl₃ |
| Example 47 | Synthesis Example 47 (Formula 2-47) | AB | Cr (THF)₃Cl₃ |
| Example 48 | Synthesis Example 48 (Formula 2-48) | AB | Cr (THF)₃Cl₃ |
| Example 49 | Synthesis Example 49 (Formula 2-49) | AB | Cr (THF)₃Cl₃ |
| Example 50 | Synthesis Example 50 (Formula 2-50) | AB | Cr (THF)₃Cl₃ |
| Example 51 | Synthesis Example 51 (Formula 2-51) | AB | Cr (THF)₃Cl₃ |
| Example 52 | Synthesis Example 52 (Formula 2-52) | AB | Cr (THF)₃Cl₃ |
| Example 53 | Synthesis Example 53 (Formula 2-53) | AB | Cr (THF)₃Cl₃ |
| Example 54 | Synthesis Example 54 (Formula 2-54) | AB | Cr (THF)₃Cl₃ |
| Example 55 | Synthesis Example 55 (Formula 2-55) | AB | Cr (THF)₃Cl₃ |
| Example 56 | Synthesis Example 56 (Formula 2-56) | AB | Cr (THF)₃Cl₃ |
| Example 57 | Synthesis Example 57 (Formula 2-57) | AB | Cr (THF)₃Cl₃ |
| Example 58 | Synthesis Example 58 (Formula 2-58) | AB | Cr (THF)₃Cl₃ |
| Example 59 | Synthesis Example 59 (Formula 2-59) | AB | Cr (THF)₃Cl₃ |
| Example 60 | Synthesis Example 60 (Formula 2-60) | AB | Cr (THF)₃Cl₃ |
| Example 61 | Synthesis Example 61 (Formula 2-61) | AB | Cr (THF)₃Cl₃ |
| Example 62 | Synthesis Example 62 (Formula 2-62) | AB | Cr (THF)₃Cl₃ |
| Example 63 | Synthesis Example 63 (Formula 2-63) | AB | Cr (THF)₃Cl₃ |
| Example 64 | Synthesis Example 64 (Formula 2-64) | AB | Cr (THF)₃Cl₃ |
| Example 65 | Synthesis Example 65 (Formula 2-65) | AB | Cr (THF)₃Cl₃ |
| Example 66 | Synthesis Example 66 (Formula 2-66) | AB | Cr (THF)₃Cl₃ |
| Example 67 | Synthesis Example 67 (Formula 9-1) | AB | Cr (THF)₃Cl₃ |
| Example 68 | Synthesis Example 68 (Formula 9-2) | AB | Cr (THF)₃Cl₃ |
| Example 69 | Synthesis Example 69 (Formula 9-3) | AB | Cr (THF)₃Cl₃ |
| Example 70 | Synthesis Example 70 (Formula 9-4) | AB | Cr (THF)₃Cl₃ |
| Example 71 | Synthesis Example 71 (Formula 9-5) | AB | Cr (THF)₃Cl₃ |
| Example 72 | Synthesis Example 72 (Formula 9-6) | AB | Cr (THF)₃Cl₃ |
| Example 73 | Synthesis Example 73 (Formula 9-7) | AB | Cr (THF)₃Cl₃ |
| Example 74 | Synthesis Example 74 (Formula 9-8) | AB | Cr (THF)₃Cl₃ |
| Example 75 | Synthesis Example 75 (Formula 9-9) | AB | Cr (THF)₃Cl₃ |
| Example 76 | Synthesis Example 76 (Formula 9-10) | AB | Cr (THF)₃Cl₃ |
| Example 77 | Synthesis Example 77 (Formula 9-11) | AB | Cr (THF)₃Cl₃ |
| Example 78 | Synthesis Example 78 (Formula 9-12) | AB | Cr (THF)₃Cl₃ |
| Example 79 | Synthesis Example 79 (Formula 9-13) | AB | Cr (THF)₃Cl₃ |
| Example 80 | Synthesis Example 80 (Formula 9-14) | AB | Cr (THF)₃Cl₃ |
| Example 81 | Synthesis Example 81 (Formula 9-15) | AB | Cr (THF)₃Cl₃ |
| Example 82 | Synthesis Example 82 (Formula 9-16) | AB | Cr (THF)₃Cl₃ |
| Example 83 | Synthesis Example 83 (Formula 9-17) | AB | Cr (THF)₃Cl₃ |
| Example 84 | Synthesis Example 84 (Formula 9-18) | AB | Cr (THF)₃Cl₃ |
| Example 85 | Synthesis Example 85 (Formula 9-19) | AB | Cr (THF)₃Cl₃ |
| Example 86 | Synthesis Example 86 (Formula 9-20) | AB | Cr (THF)₃Cl₃ |
| Example 87 | Synthesis Example 87 (Formula 2-67) | AB | Cr (THF)₃Cl₃ |
| Example 88 | Synthesis Example 88 (Formula 2-68) | AB | Cr (THF)₃Cl₃ |
| Example 89 | Synthesis Example 89 (Formula 2-69) | AB | Cr (THF)₃Cl₃ |
| Example 90 | Synthesis Example 90 (Formula 2-70) | AB | Cr (THF)₃Cl₃ |
| Example 91 | Synthesis Example 91 (Formula 2-71) | AB | Cr (THF)₃Cl₃ |
| Example 92 | Synthesis Example 92 (Formula 2-72) | AB | Cr (THF)₃Cl₃ |
| Example 93 | Synthesis Example 93 (Formula 2-73) | AB | Cr (THF)₃Cl₃ |
| Example 94 | Synthesis Example 94 (Formula 2-74) | AB | Cr (THF)₃Cl₃ |
| Example 95 | Synthesis Example 95 (Formula 2-75) | AB | Cr (THF)₃Cl₃ |
| Example 96 | Synthesis Example 96 (Formula 2-76) | AB | Cr (THF)₃Cl₃ |
| Example 97 | Synthesis Example 97 (Formula 2-77) | AB | Cr (THF)₃Cl₃ |
| Example 98 | Synthesis Example 98 (Formula 2-78) | AB | Cr (THF)₃Cl₃ |
| Example 99 | Synthesis Example 99 (Formula 2-79) | AB | Cr (THF)₃Cl₃ |
| Example 100 | Synthesis Example 100 (Formula 2-80) | AB | Cr (THF)₃Cl₃ |
| Example 101 | Synthesis Example 101 (Formula 2-81) | AB | Cr (THF)₃Cl₃ |
| Example 102 | Synthesis Example 102 (Formula 2-82) | AB | Cr (THF)₃Cl₃ |
| Example 103 | Synthesis Example 103 (Formula 2-83) | AB | Cr (THF)₃Cl₃ |
| Example 104 | Synthesis Example 104 (Formula 2-84) | AB | Cr (THF)₃Cl₃ |
| Example 105 | Synthesis Example 105 (Formula 2-85) | AB | Cr (THF)₃Cl₃ |
| Example 106 | Synthesis Example 106 (Formula 2-86) | AB | Cr (THF)₃Cl₃ |
| Example 107 | Synthesis Example 107 (Formula 2-87) | AB | Cr (THF)₃Cl₃ |
| Example 108 | Synthesis Example 108 (Formula 2-88) | AB | Cr (THF)₃Cl₃ |
| Example 109 | Synthesis Example 109 (Formula 2-89) | AB | Cr (THF)₃Cl₃ |
| Example 110 | Synthesis Example 110 (Formula 2-90) | AB | Cr (THF)₃Cl₃ |
| Example 111 | Synthesis Example 111 (Formula 2-91) | AB | Cr (THF)₃Cl₃ |
| Example 112 | Synthesis Example 112 (Formula 2-92) | AB | Cr (THF)₃Cl₃ |
| Example 113 | Synthesis Example 113 (Formula 2-93) | AB | Cr (THF)₃Cl₃ |
| Example 114 | Synthesis Example 114 (Formula 2-94) | AB | Cr (THF)₃Cl₃ |
| Example 115 | Synthesis Example 115 (Formula 2-95) | AB | Cr (THF)₃Cl₃ |
| Example 116 | Synthesis Example 116 (Formula 2-96) | AB | Cr (THF)₃Cl₃ |
| Comparative Example 1 | Comparative Synthesis Example 1 (Formula 14) | AB | Cr (THF)₃Cl₃ |
| Comparative Example 2 | Comparative Synthesis Example 2 (Formula 15) | AB | Cr (THF)₃Cl₃ |
| Comparative Example 3 | Comparative Synthesis Example 3 (Formula 16) | AB | Cr (THF)₃Cl₃ |
| Comparative Example 4 | Comparative Synthesis Example 4 (Formula 17) | AB | Cr (THF)₃Cl₃ |

### Experimental Examples

The results of the ethylene oligomerization reaction according to Examples and Comparative examples above are shown in Table 5 below.
* Catalytic activity (ton/mol· Cr/hr): From a total product weight (ton) value, which is the sum of the weight (ton) of the obtained liquid product and the weight of the obtained solid product, the catalytic activity was calculated.
* 1-C6 and 1-C8 selectivity (wt%): From the result of analyzing the distribution of the liquid product by GC, the contents of 1-hexene (1-C6) and 1-octene (1-C8) were calculated to calculate the wt% of 1-hexene or 1-octene with respect to the total weight of the product.
* Solid (wt%): The wt% of the solid product with respect to the total weight of the product was calculated. This represents the degree to which polyethylene having a carbon number of about 40 or more was generated as an insoluble solid which was not dissolved in the solvent.

**[Table 5]**

| Classif ication | Catalytic activity | 1-C6 and 1-C8 selectivity | | | Solid |
|---|---|---|---|---|---|
| | | 1-C6 | 1-C8 | Total | |
| | (ton/mol·Cr /hr) | (wt%) | (wt%) | (wt%) | (wt%) |
| Example 1 | 157 | 32.0 | 54.8 | 86.9 | 0.32 |
| Example 2 | 137 | 37.5 | 46.9 | 84.5 | 0.68 |
| Example 3 | 148 | 31.9 | 54.5 | 86.4 | 0.51 |
| Example 4 | 142 | 36.5 | 44.6 | 81.2 | 0.66 |
| Example 5 | 152 | 31.5 | 57.7 | 89.3 | 0.63 |
| Example 6 | 143 | 32.7 | 53.0 | 85.7 | 0.52 |
| Example 7 | 145 | 31.9 | 57.2 | 89.1 | 0.46 |
| Example 8 | 130 | 36.0 | 52.2 | 88.1 | 0.64 |
| Example 9 | 135 | 31.9 | 54.5 | 86.3 | 0.35 |
| Example 10 | 150 | 29.7 | 55.9 | 85.6 | 0.41 |
| Example 11 | 160 | 31.7 | 57.4 | 89.1 | 0.63 |
| Example 12 | 179 | 31.7 | 56.0 | 87.8 | 0.55 |
| Example 13 | 159 | 38.0 | 47.0 | 85.0 | 0.57 |
| Example 14 | 172 | 32.6 | 53.8 | 86.4 | 0.48 |
| Example 15 | 166 | 36.8 | 47.0 | 83.8 | 0.70 |
| Example 16 | 174 | 31.6 | 58.2 | 89.8 | 0.57 |
| Example 17 | 165 | 34.1 | 54.8 | 88.9 | 0.65 |
| Example 18 | 165 | 32.4 | 56.8 | 89.1 | 0.58 |
| Example 19 | 154 | 36.7 | 52.0 | 88.7 | 0.49 |
| Example 20 | 160 | 32.1 | 55.0 | 87.0 | 0.52 |
| Example 21 | 172 | 31.0 | 56.7 | 87.7 | 0.68 |
| Example 22 | 181 | 32.6 | 58.3 | 90.9 | 0.50 |
| Example 23 | 177 | 39.5 | 46.8 | 86.2 | 0.53 |
| Example 24 | 158 | 45.5 | 38.0 | 83.5 | 0.41 |
| Example 25 | 172 | 40.7 | 43.9 | 84.6 | 0.55 |
| Example 26 | 161 | 46.3 | 34.6 | 80.9 | 0.64 |
| Example 27 | 172 | 40.9 | 47.7 | 88.6 | 0.58 |
| Example 28 | 159 | 42.3 | 43.4 | 85.7 | 0.35 |
| Example 29 | 162 | 41.1 | 47.1 | 88.2 | 0.47 |
| Example 30 | 152 | 44.2 | 43.6 | 87.8 | 0.32 |
| Example 31 | 151 | 41.6 | 43.6 | 85.2 | 0.32 |
| Example 32 | 173 | 40.6 | 47.5 | 88.1 | 0.40 |
| Example 33 | 179 | 41.6 | 47.8 | 89.4 | 0.64 |
| Example 34 | 185 | 39.8 | 48.1 | 87.9 | 0.49 |
| Example 35 | 164 | 45.5 | 38.2 | 83.7 | 0.49 |
| Example 36 | 173 | 39.5 | 44.8 | 84.3 | 0.35 |
| Example 37 | 167 | 47.1 | 36.5 | 83.6 | 0.69 |
| Example 38 | 179 | 41.6 | 47.2 | 88.8 | 0.43 |
| Example 39 | 164 | 43.7 | 45.4 | 89.0 | 0.57 |
| Example 40 | 174 | 39.8 | 48.1 | 87.9 | 0.67 |
| Example 41 | 160 | 44.0 | 41.2 | 85.2 | 0.50 |
| Example 42 | 159 | 42.4 | 43.7 | 86.1 | 0.59 |
| Example 43 | 177 | 39.4 | 48.0 | 87.5 | 0.62 |
| Example 44 | 184 | 41.2 | 47.4 | 88.6 | 0.39 |
| Example 45 | 178 | 47.8 | 38.6 | 86.4 | 0.48 |
| Example 46 | 160 | 53.9 | 31.6 | 85.5 | 0.69 |
| Example 47 | 172 | 56.9 | 38.6 | 85.5 | 0.39 |
| Example 48 | 164 | 52.9 | 30.0 | 83.0 | 0.53 |
| Example 49 | 176 | 46.0 | 41.1 | 87.1 | 0.50 |
| Example 50 | 164 | 48.1 | 37.7 | 85.8 | 0.47 |
| Example 51 | 170 | 47.7 | 42.4 | 90.1 | 0.43 |
| Example 52 | 152 | 52.4 | 36.3 | 88.7 | 0.34 |
| Example 53 | 158 | 48.6 | 38.3 | 86.9 | 0.62 |
| Example 54 | 174 | 47.7 | 41.1 | 88.8 | 0.34 |
| Example 55 | 183 | 47.4 | 39.8 | 87.1 | 0.53 |
| Example 56 | 148 | 51.6 | 29.9 | 81.4 | 0.43 |
| Example 57 | 128 | 57.4 | 21.1 | 78.5 | 0.47 |
| Example 58 | 139 | 51.5 | 27.8 | 79.2 | 0.38 |
| Example 59 | 133 | 56.7 | 18.9 | 75.6 | 0.30 |
| Example 60 | 145 | 52.5 | 30.5 | 83.0 | 0.36 |
| Example 61 | 136 | 55.0 | 27.0 | 82.0 | 0.44 |
| Example 62 | 137 | 53.7 | 30.8 | 84.6 | 0.65 |
| Example 63 | 121 | 57.0 | 26.4 | 83.4 | 0.61 |
| Example 64 | 127 | 54.1 | 29.2 | 83.3 | 0.58 |
| Example 65 | 143 | 51.2 | 32.4 | 83.6 | 0.57 |
| Example 66 | 150 | 52.3 | 29.1 | 81.4 | 0.41 |
| Example 67 | 141 | 32.7 | 54.4 | 87.2 | 0.43 |
| Example 68 | 120 | 37.8 | 42.6 | 80.4 | 0.62 |
| Example 69 | 132 | 33.5 | 53.0 | 86.5 | 0.47 |
| Example 70 | 126 | 38.2 | 42.5 | 80.7 | 0.65 |
| Example 71 | 135 | 32.8 | 51.6 | 84.3 | 0.67 |
| Example 72 | 157 | 32.5 | 54.3 | 86.9 | 0.45 |
| Example 73 | 135 | 38.2 | 44.1 | 82.3 | 0.68 |
| Example 74 | 147 | 32.8 | 53.7 | 86.5 | 0.69 |
| Example 75 | 143 | 38.6 | 43.0 | 81.6 | 0.52 |
| Example 76 | 153 | 33.3 | 51.9 | 85.2 | 0.41 |
| Example 77 | 156 | 39.9 | 46.6 | 86.5 | 0.43 |
| Example 78 | 135 | 45.7 | 33.6 | 79.3 | 0.66 |
| Example 79 | 148 | 42.0 | 44.5 | 86.5 | 0.33 |
| Example 80 | 142 | 47.5 | 31.6 | 79.1 | 0.49 |
| Example 81 | 153 | 39.9 | 42.8 | 82.7 | 0.48 |
| Example 82 | 157 | 41.1 | 45.9 | 87.1 | 0.49 |
| Example 83 | 136 | 45.6 | 34.5 | 80.1 | 0.59 |
| Example 84 | 149 | 42.1 | 45.3 | 87.4 | 0.66 |
| Example 85 | 144 | 46.6 | 33.0 | 79.6 | 0.43 |
| Example 86 | 151 | 41.1 | 43.0 | 84.0 | 0.47 |
| Example 87 | 152 | 30.3 | 55.7 | 86.0 | 0.46 |
| Example 88 | 129 | 37.0 | 47.3 | 84.2 | 0.35 |
| Example 89 | 141 | 30.6 | 53.6 | 84.3 | 0.66 |
| Example 90 | 136 | 37.1 | 45.6 | 82.7 | 0.58 |
| Example 91 | 146 | 30.4 | 58.5 | 88.9 | 0.42 |
| Example 92 | 167 | 31.7 | 56.1 | 87.8 | 0.43 |
| Example 93 | 147 | 38.0 | 48.4 | 86.4 | 0.35 |
| Example 94 | 157 | 31.8 | 54.2 | 86.0 | 0.40 |
| Example 95 | 153 | 35.7 | 45.7 | 81.4 | 0.57 |
| Example 96 | 163 | 31.2 | 59.4 | 90.6 | 0.67 |
| Example 97 | 165 | 39.4 | 46.2 | 85.6 | 0.45 |
| Example 98 | 143 | 45.2 | 37.8 | 82.9 | 0.42 |
| Example 99 | 155 | 39.3 | 43.7 | 83.0 | 0.42 |
| Example 100 | 150 | 43.8 | 36.7 | 80.5 | 0.42 |
| Example 101 | 157 | 38.5 | 50.8 | 89.3 | 0.51 |
| Example 102 | 164 | 39.9 | 47.6 | 87.5 | 0.49 |
| Example 103 | 140 | 45.2 | 38.2 | 83.4 | 0.65 |
| Example 104 | 152 | 41.1 | 44.6 | 85.7 | 0.63 |
| Example 105 | 147 | 44.6 | 35.2 | 79.7 | 0.64 |
| Example 106 | 160 | 39.1 | 50.4 | 89.5 | 0.52 |
| Example 107 | 162 | 47.9 | 40.9 | 88.8 | 0.50 |
| Example 108 | 143 | 52.2 | 32.8 | 85.0 | 0.45 |
| Example 109 | 155 | 47.2 | 36.1 | 83.3 | 0.64 |
| Example 110 | 149 | 52.4 | 30.5 | 82.9 | 0.61 |
| Example 111 | 158 | 46.1 | 42.6 | 88.7 | 0.46 |
| Example 112 | 143 | 52.6 | 35.3 | 87.9 | 0.47 |
| Example 113 | 121 | 56.8 | 27.6 | 84.4 | 0.58 |
| Example 114 | 134 | 51.2 | 31.7 | 82.8 | 0.42 |
| Example 115 | 129 | 57.1 | 24.6 | 81.7 | 0.59 |
| Example 116 | 138 | 49.6 | 38.2 | 87.8 | 0.37 |
| Compara tive Example 1 | 113 | 52.4 | 38.4 | 90.8 | 2.70 |
| Compara tive Example 2 | 42 | 45.7 | 43.0 | 88.7 | 1.20 |
| Compara tive Example 3 | 0 | - | - | - | - |
| Compara tive Example 4 | 0 | - | - | - | - |

As shown in Table 5 above, when an ethylene oligomerization reaction is performed using a catalyst composition including the ligand compound according to the present invention, it has been confirmed that phenyl positioned at the end of the diphosphino aminyl moiety has a specific substituent at the ortho position, and has, as a substituent, an alkyl group having a specific carbon number, or a silyl group substituted with the alkyl group having a specific carbon number at each of the meta position and the para position, and also, that catalytic activity, selectivity, and stability were all improved by controlling the steric strain around the P-N-P functional group from the substituent substituted with the nitrogen atom.

From the above results, it can be confirmed that when ethylene oligomerization is performed using an organic chromium compound including the ligand compound of the present invention and the catalyst composition, it is possible to prepare linear alpha-olefins with high 1-hexene and 1-octene selectivity while having excellent productivity due to high catalytic activity.

## Claims

1. A ligand compound represented by any one among Formula 2 to Formula 9 below: 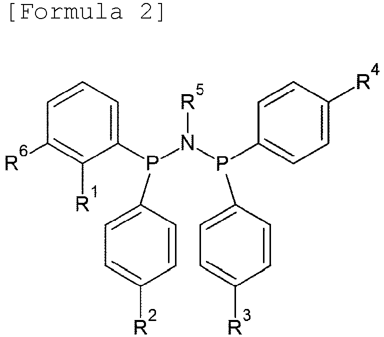 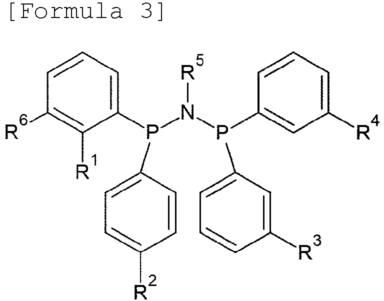 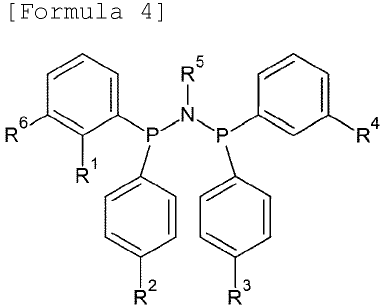 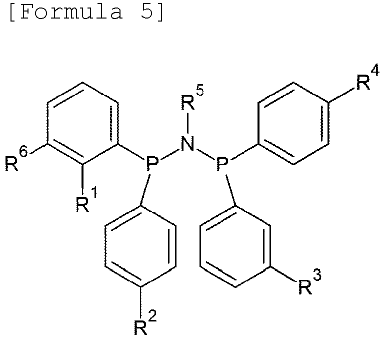 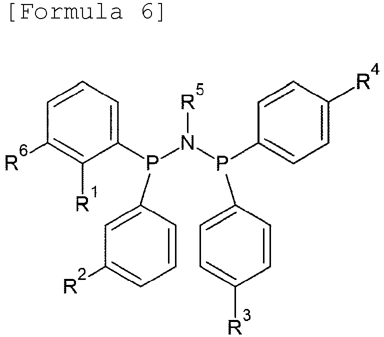 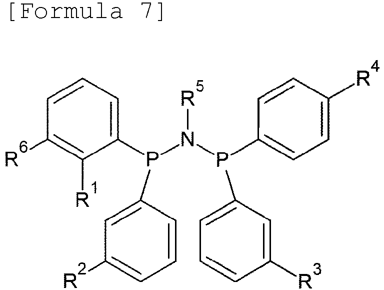 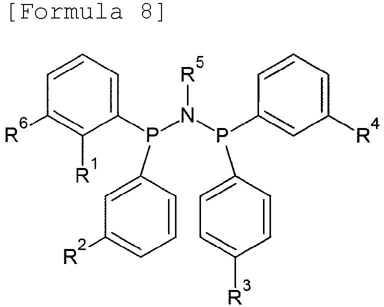 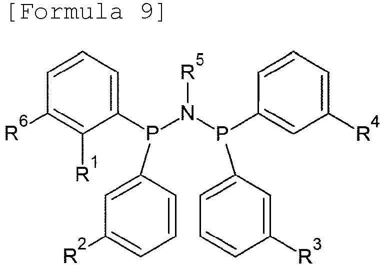 wherein in Formula 2 to Formula 9 above,
R¹ is a halogen group, an alkyl group having 1 to 10 carbon atoms substituted or unsubstituted with a halogen group, an alkoxy group having 1 to 10 carbon atoms substituted or unsubstituted with a halogen group, an alkylthio group having 1 to 10 carbon atoms substituted or unsubstituted with a halogen group, an alkylsulfonate group having 1 to 10 carbon atoms substituted or unsubstituted with a halogen group, or a trialkylsilyl group in which the alkyl groups each independently have 1 to 10 carbon atoms, or R¹ is bonded to R⁶ to form a monocyclic or polycyclic aromatic hydrocarbon ring, or a monocyclic or polycyclic hetero ring, wherein if R⁶ and R¹ are not bonded to each other to form a monocyclic or polycyclic aromatic hydrocarbon ring, or a monocyclic or polycyclic hetero ring, R⁶ is hydrogen;
R² to R⁴ are each independently hydrogen, an alkyl group having 5 to 20 carbon atoms, a cycloalkyl group having 5 to 20 carbon atoms, or a trialkylsilyl group in which the alkyl groups each independently have 1 to 10 carbon atoms, wherein R² to R⁴ are not hydrogen at the same time; and
R⁵ is an alkyl group having 1 to 20 carbon atoms, an alkyl group having 1 to 20 carbon atoms and substituted with an aryl group having 6 to 10 carbon atoms, a cycloalkyl group having 5 to 10 carbon atoms, or a cycloalkyl group having 5 to 10 carbon atoms fused with an aryl group having 6 to 10 carbon atoms.

2. The ligand compound of claim 1, wherein R¹ is fluoro, an alkyl group having 1 to 5 carbon atoms substituted or unsubstituted with fluoro, an alkoxy group having 1 to 5 carbon atoms substituted or unsubstituted with fluoro, an alkylthio group having 1 to 5 carbon atoms substituted or unsubstituted with fluoro, an alkylsulfonate group having 1 to 5 carbon atoms substituted or unsubstituted with fluoro, or a trialkylsilyl group in which the alkyl groups each independently have 1 to 5 carbon atoms, or R¹ is bonded to R⁶ to form a monocyclic or polycyclic hetero ring.

3. The ligand compound of claim 1, wherein R¹ is a fluoro group, a trifluoromethyl group, a methoxy group, a methyl group, a trifluoromethoxy group, a trifluoromethylthio group, a methylthio group, a methylsulfonate group, or a trimethylsilyl group, or R¹ is bonded to R⁶ to form furan or dibenzofuran.

4. The ligand compound of claim 1, wherein R² to R⁴ are each independently hydrogen, an alkyl group having 8 to 12 carbon atoms, a tripropylsilyl group, or a tributylsilyl group, wherein R² to R⁴ are not hydrogen at the same time.

5. The ligand compound of claim 1, wherein R² to R⁴ are each independently an n-decyl group, a tripropylsilyl group, or a tributylsilyl group.

6. The ligand compound of claim 1, wherein R² is hydrogen, and R³ and R⁴ are each independently an n-decyl group, a tripropylsilyl group, or a tributylsilyl group.

7. The ligand compound of claim 1, wherein R⁵ is an alkyl group having 3 to 5 carbon atoms, an alkyl group having 1 to 5 carbon atoms substituted with an aryl group having 6 to 10 carbon atoms, a cycloalkyl group having 5 to 8 carbon atoms, or a cycloalkyl group having 5 to 10 carbon atoms fused with an aryl group having 6 to 10 carbon atoms.

8. The ligand compound of claim 1, which is represented by any one among Formula 2-1 to Formula 2-96 and Formula 9-1 to Formula 9-20 below:

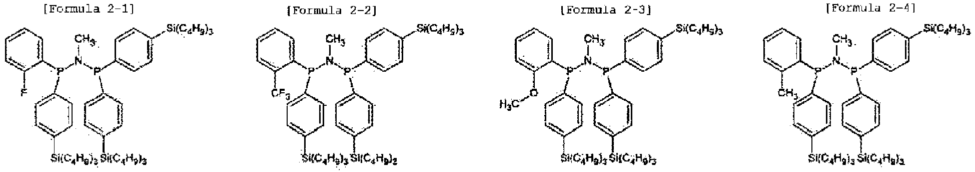

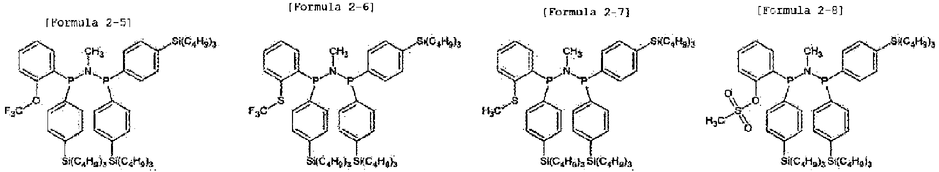

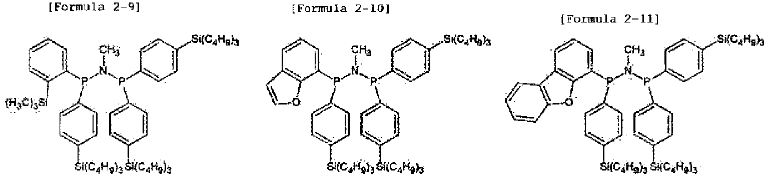

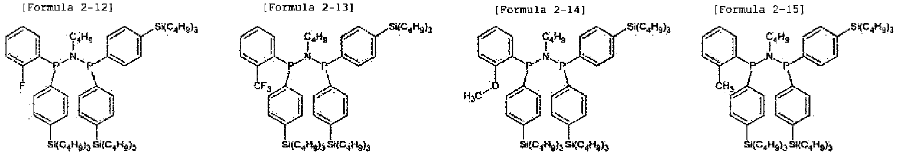

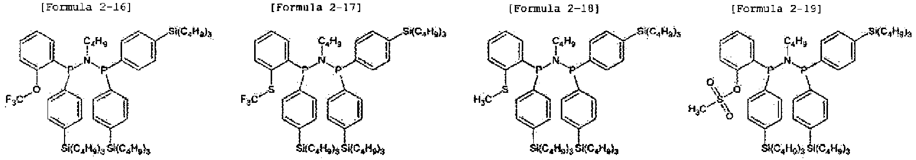

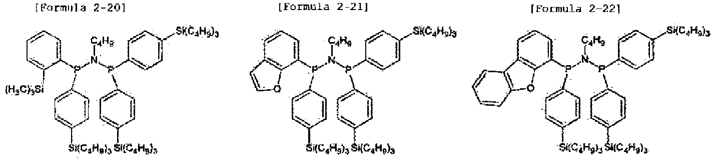

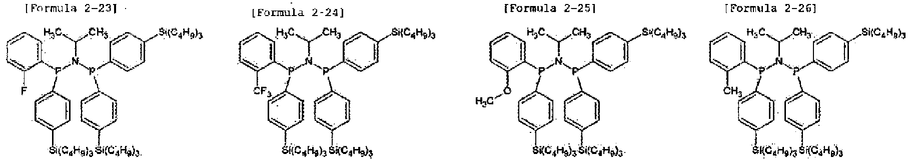

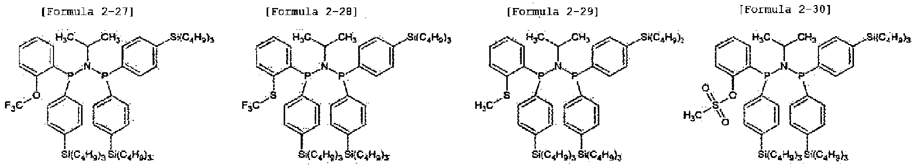

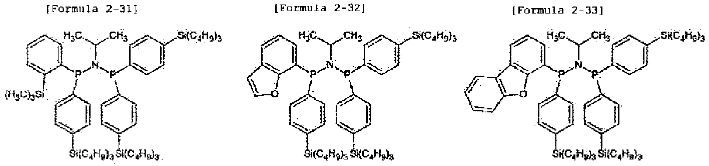

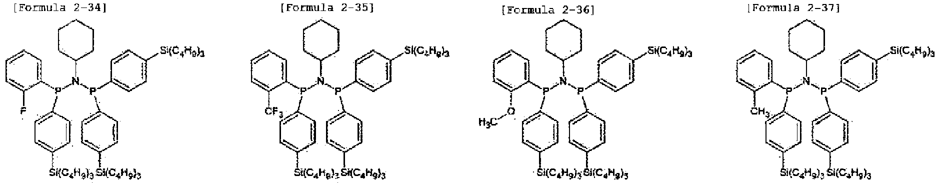

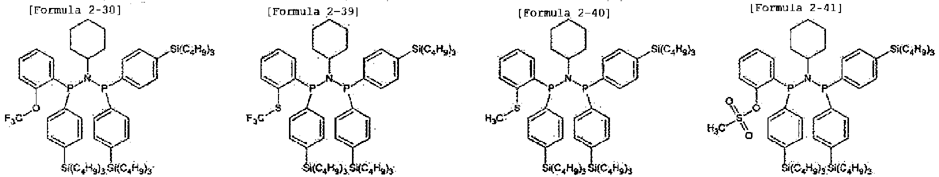

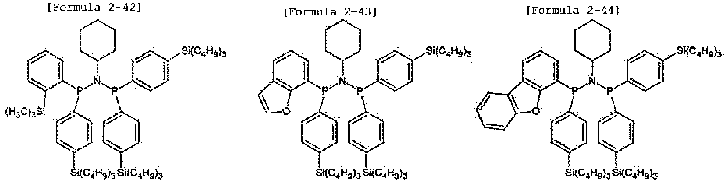

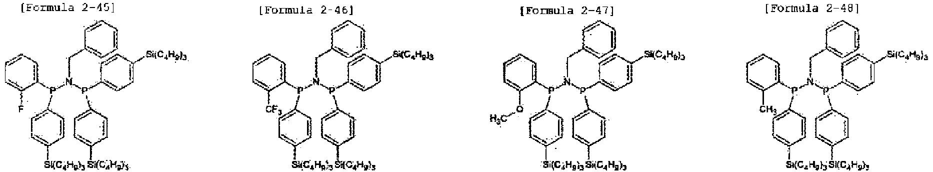

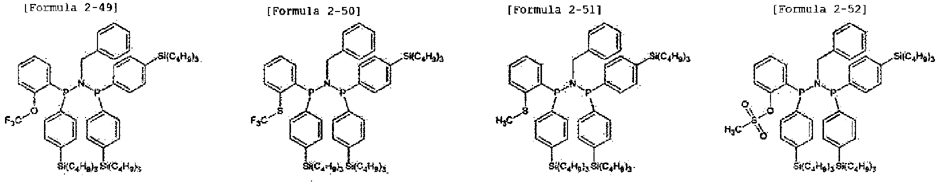

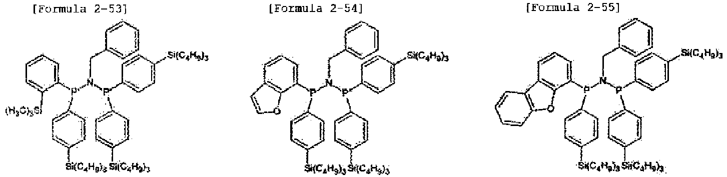

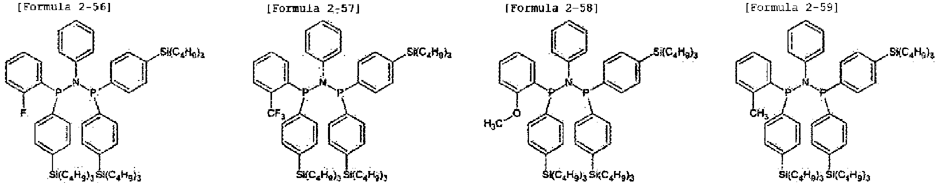

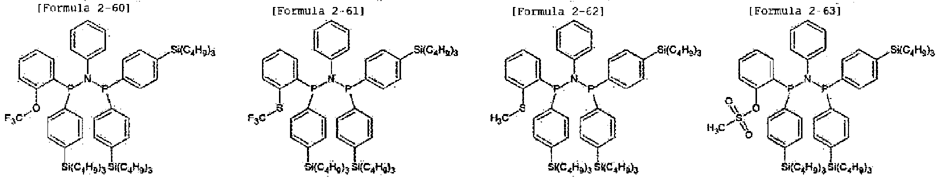

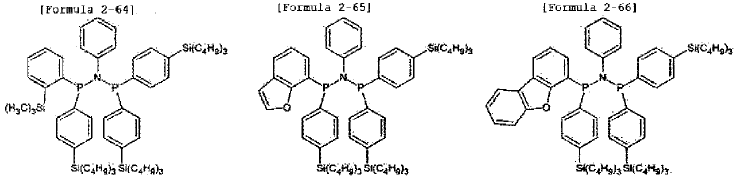

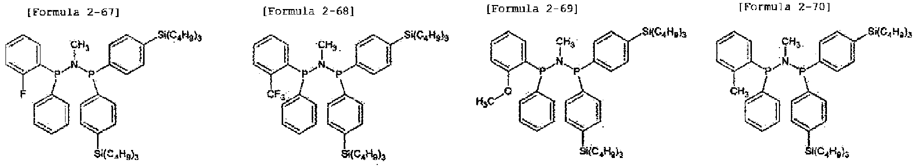

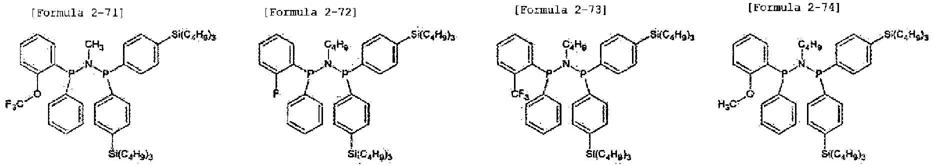

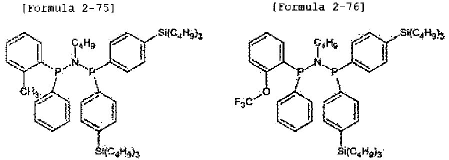

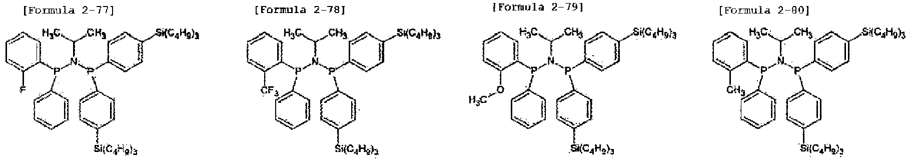

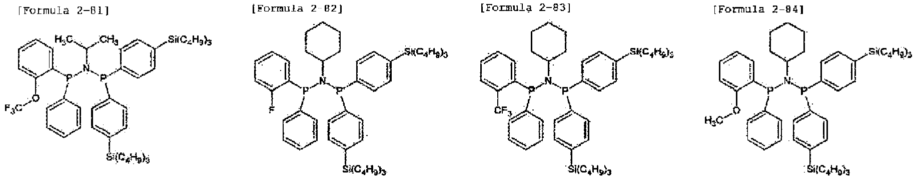

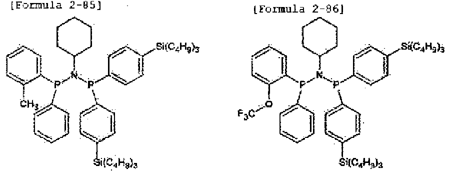

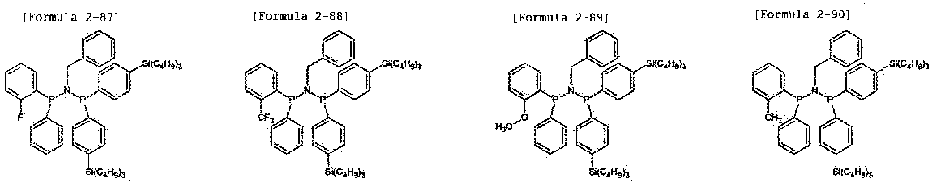

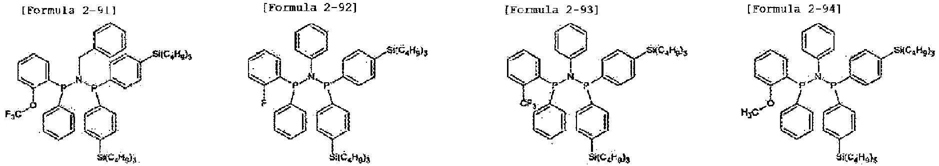

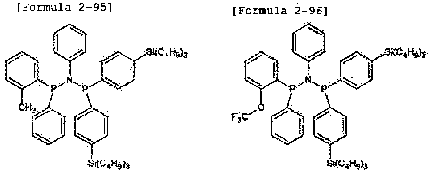

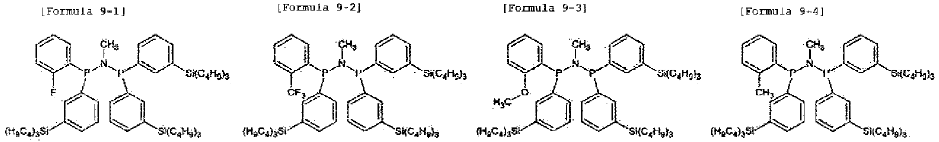

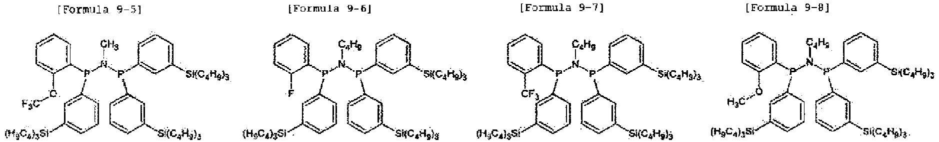

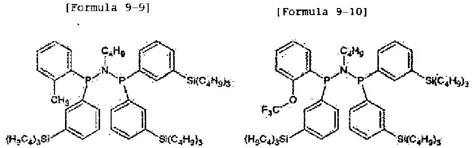

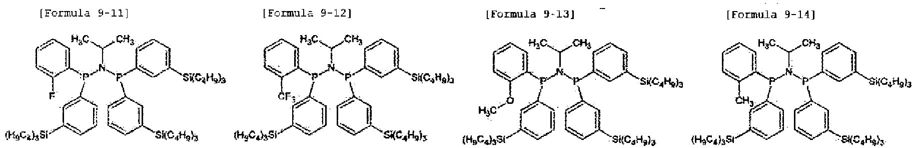

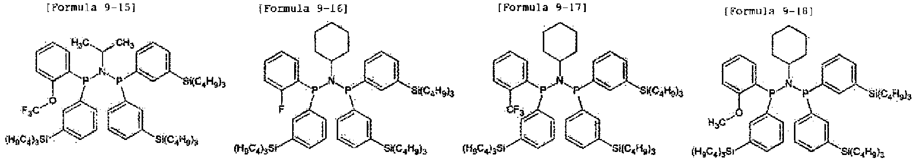

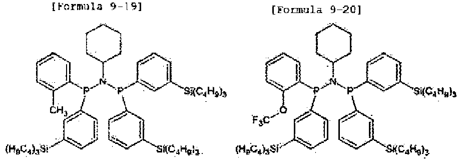

9. An organic chromium compound comprising the ligand compound according to claim 1 and chromium bidentated to the ligand compound.

10. The organic chromium compound of claim 9, wherein an unshared electron pair of N and one or both of the two Ps of the ligand compound is bidentated to the chromium.

11. A catalyst composition comprising the ligand compound according to claim 1, chromium, and a co-catalyst.

12. The catalyst composition of claim 11, wherein the co-catalyst is one or more compounds selected from compounds represented by Formula 10 to Formula 13 below:
[Formula 10] -[Al(R¹³)-O]ₐ-
-wherein in Formula 10 above,
each R¹³ is independently a halogen group, a hydrocarbyl group having 1 to 20 carbon atoms, or a hydrocarbyl group having 1 to 20 carbon atoms substituted with a halogen group, and
a is an integer of 2 or greater;
[Formula 11] E(R¹⁴)₃
wherein in Formula 11 above,
E is aluminum or boron, and
each R¹⁴ is independently hydrogen, a halogen group, a hydrocarbyl group having 1 to 20 carbon atoms, or a hydrocarbyl group having 1 to 20 carbon atoms substituted with a halogen group;
[Formula 12] [L-H]⁺[G(Y)₄]⁻
[Formula 13] [L]⁺[G(Y)₄]⁻
wherein in Formulas 12 and 13 above,
L is a neutral or a cationic Lewis acid,
[L-H]⁺ is a Bronsted acid,
G is a Group 13 element, and
each Y is independently a substituted or unsubstituted alkyl group having 1 to 20 carbon atoms, or a substituted or unsubstituted aryl group having 6 to 20 carbon atoms, wherein if the alkyl group or aryl group is substituted, the substituent is a halogen group, a hydrocarbyl group having 1 to 20 carbon atoms, an alkoxy group having 1 to 20 carbon atoms, or an aryloxy group having 6 to 20 carbon atoms.

13. A method for preparing a linear alpha-olefin, the method comprising (S10) oligomerizing ethylene in the presence of the catalyst composition according to claim 11.

14. The method of claim 13, wherein the linear alpha-olefin is 1-hexene, 1-octene, or a mixture thereof.

## Patentansprüche

1. Ligandenverbindung, dargestellt durch eine der nachstehenden Formel 2 bis Formel 9: 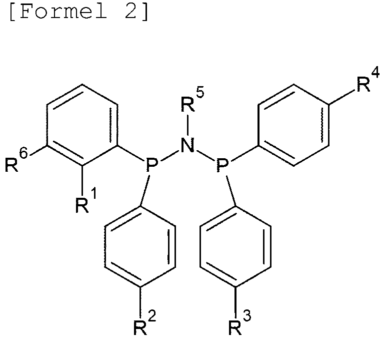 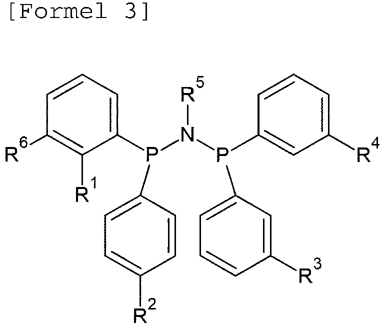 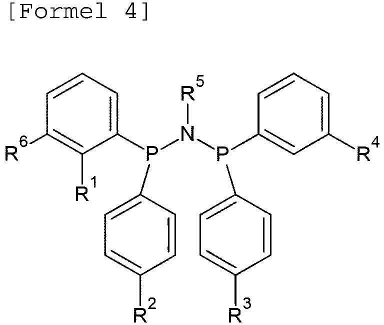 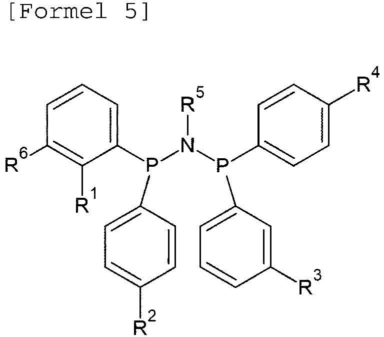 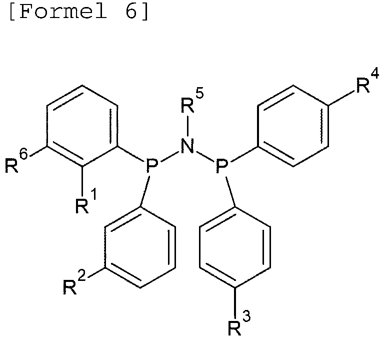 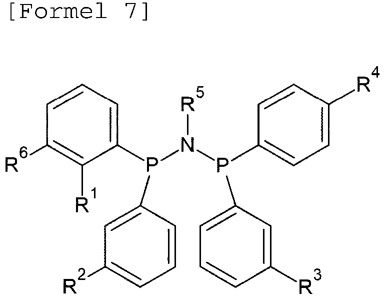 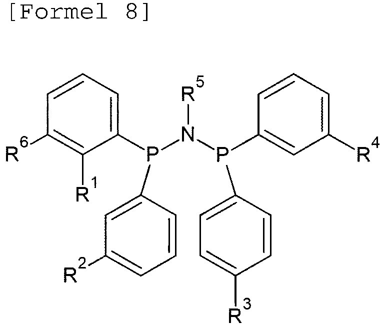 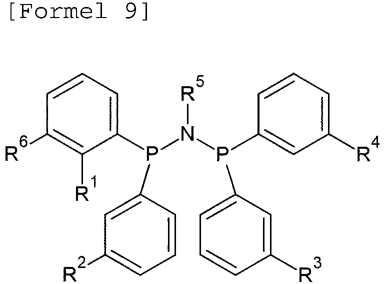 wobei in der obigen Formel 2 bis Formel 9
R¹ ein Halogengruppe, eine Alkylgruppe mit 1 bis 10 Kohlenstoffatomen, die mit einer Halogengruppe substituiert oder unsubstituiert ist, eine Alkoxygruppe mit 1 bis 10 Kohlenstoffatomen, die mit einer Halogengruppe substituiert oder unsubstituiert ist, eine Alkylthiogruppe mit 1 bis 10 Kohlenstoffatomen, die mit einer Halogengruppe substituiert oder unsubstituiert ist, eine Alkylsulfonatgruppe mit 1 bis 10 Kohlenstoffatomen, die mit einer Halogengruppe substituiert oder unsubstituiert ist, oder eine Trialkylsilylgruppe, in der die Alkylgruppen jeweils unabhängig 1 bis 10 Kohlenstoffatome aufweisen, ist oder R¹ an R⁶ gebunden ist, um einen monocyclischen oder polycyclischen aromatischen Kohlenwasserstoffring oder einen monocyclischen oder polycyclischen Heteroring zu bilden, wobei, wenn R⁶ und R¹ nicht aneinander gebunden sind, um einen monocyclischen oder polycyclischen aromatischen Kohlenwasserstoffring oder einen monocyclischen oder polycyclischen Heteroring zu bilden, R⁶ Wasserstoff ist,
R² bis R⁴ jeweils unabhängig Wasserstoff, eine Alkylgruppe mit 5 bis 20 Kohlenstoffatomen, eine Cycloalkylgruppe mit 5 bis 20 Kohlenstoffatomen oder eine Trialkylsilylgruppe, in der die Alkylgruppen jeweils unabhängig 1 bis 10 Kohlenstoffatome aufweisen, ist, wobei R² bis R⁴ nicht gleichzeitig Wasserstoff sind, und
R⁵ eine Alkylgruppe mit 1 bis 20 Kohlenstoffatomen, eine Alkylgruppe, die 1 bis 20 Kohlenstoffatome aufweist und mit einer Arylgruppe mit 6 bis 10 Kohlenstoffatomen substituiert ist, eine Cycloalkylgruppe mit 5 bis 10 Kohlenstoffatomen oder eine Cycloalkylgruppe mit 5 bis 10 Kohlenstoffatomen, kondensiert mit einer Arylgruppe mit 6 bis 10 Kohlenstoffatomen, ist.

2. Ligandenverbindung nach Anspruch 1, wobei R¹ Fluor, eine Alkylgruppe mit 1 bis 5 Kohlenstoffatomen, die mit Fluor substituiert oder unsubstituiert ist, eine Alkoxygruppe mit 1 bis 5 Kohlenstoffatomen, die mit Fluor substituiert oder unsubstituiert ist, eine Alkylthiogruppe mit 1 bis 5 Kohlenstoffatomen, die mit Fluor substituiert oder unsubstituiert ist, eine Alkylsulfonatgruppe mit 1 bis 5 Kohlenstoffatomen, die mit Fluor substituiert oder unsubstituiert ist, oder eine Trialkylsilylgruppe, in der die Alkylgruppen jeweils unabhängig 1 bis 5 Kohlenstoffatome aufweisen, ist oder R¹ an R⁶ gebunden ist, um einen monocyclischen oder polycyclischen Heteroring zu bilden.

3. Ligandenverbindung nach Anspruch 1, wobei R¹ eine Fluorgruppe, eine Trifluormethylgruppe, eine Methoxygruppe, eine Methylgruppe, eine Trifluormethoxygruppe, eine Trifluormethylthiogruppe, eine Methylthiogruppe, eine Methylsulfonatgruppe oder eine Trimethylsilylgruppe ist oder R¹ an R⁶ gebunden ist, um Furan oder Dibenzofuran zu bilden.

4. Ligandenverbindung nach Anspruch 1, wobei R² bis R⁴ jeweils unabhängig Wasserstoff, eine Alkylgruppe mit 8 bis 12 Kohlenstoffatomen, eine Tripropylsilylgruppe oder eine Tributylsilylgruppe sind, wobei R² bis R⁴ nicht gleichzeitig Wasserstoff sind.

5. Ligandenverbindung nach Anspruch 1, wobei R² bis R⁴ jeweils unabhängig eine n-Decylgruppe, eine Tripropylsilylgruppe oder eine Tributylsilylgruppe sind.

6. Ligandenverbindung nach Anspruch 1, wobei R² Wasserstoff ist und R³ und R⁴ jeweils unabhängig eine n-Decylgruppe, eine Tripropylsilylgruppe oder eine Tributylsilylgruppe sind.

7. Ligandenverbindung nach Anspruch 1, wobei R⁵ eine Alkylgruppe mit 3 bis 5 Kohlenstoffatomen, eine Alkylgruppe mit 1 bis 5 Kohlenstoffatomen, die mit einer Arylgruppe mit 6 bis 10 Kohlenstoffatomen substituiert ist, eine Cycloalkylgruppe mit 5 bis 8 Kohlenstoffatomen oder eine Cycloalkylgruppe mit 5 bis 10 Kohlenstoffatomen, kondensiert mit einer Arylgruppe mit 6 bis 10 Kohlenstoffatomen, ist.

8. Ligandenverbindung nach Anspruch 1, die durch eine der nachstehenden Formel 2-1 bis Formel 2-96 und Formel 9-1 bis Formel 9-20 dargestellt ist:

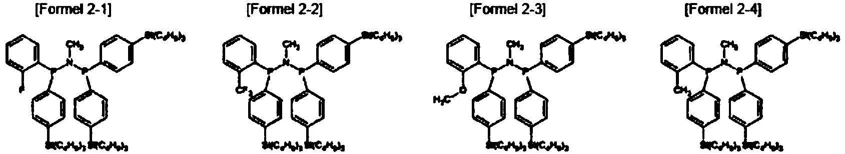

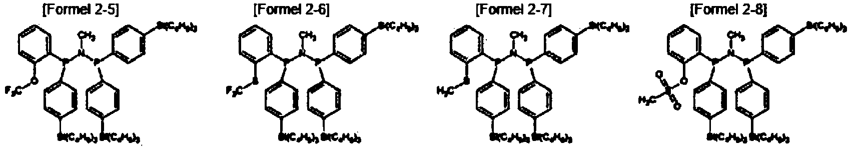

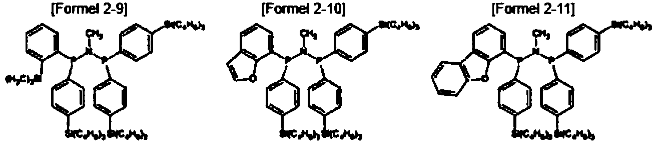

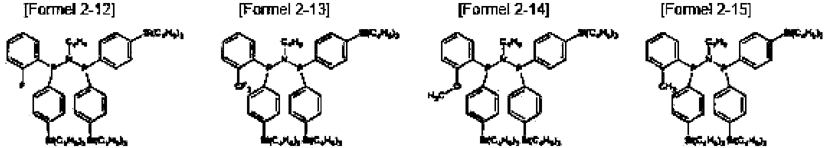

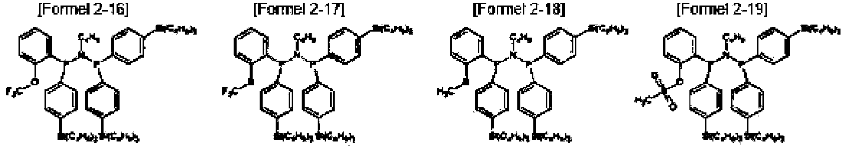

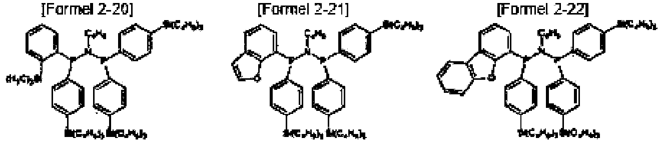

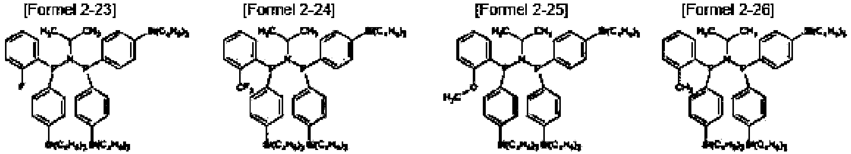

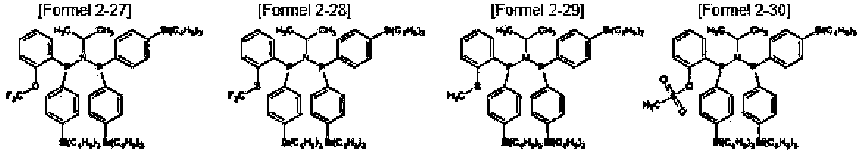

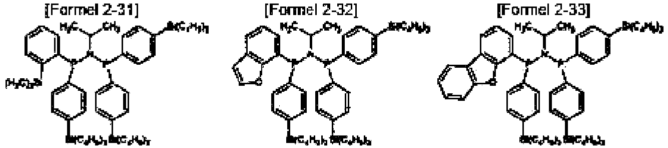

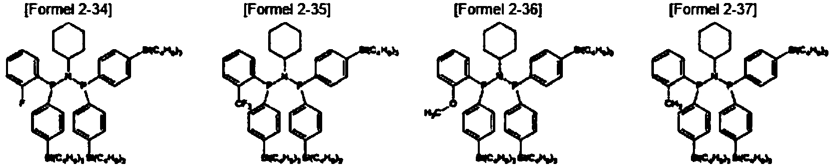

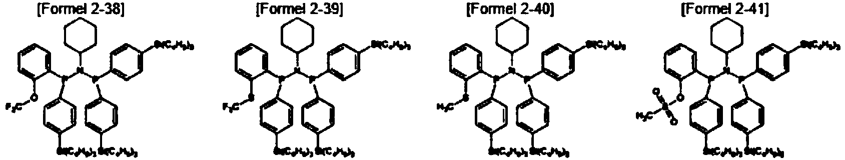

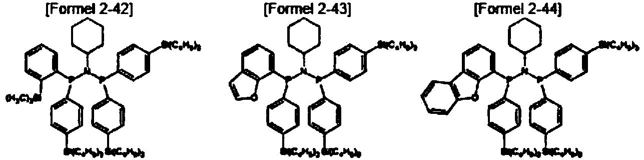

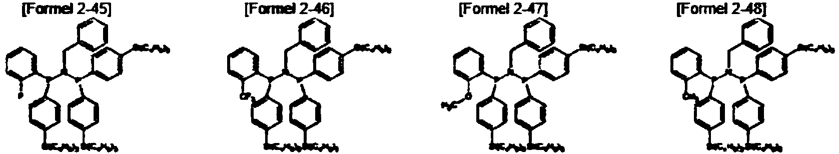

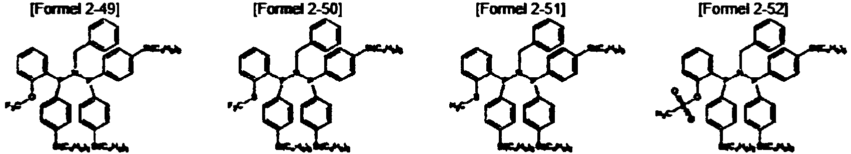

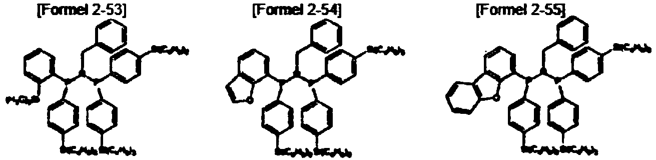

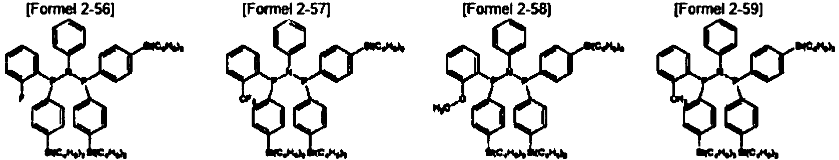

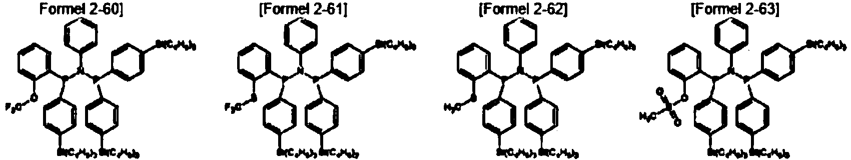

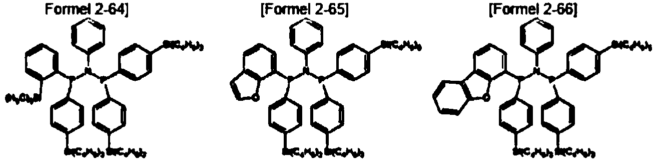

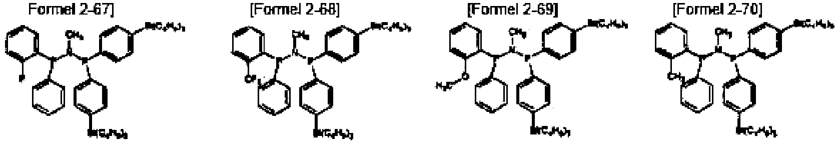

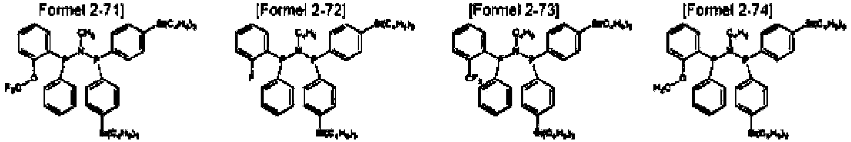

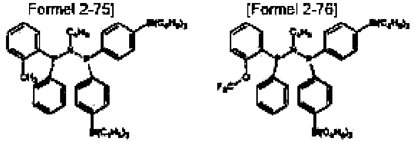

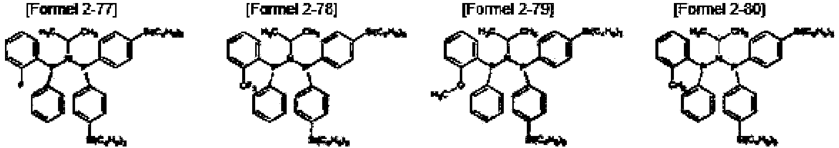

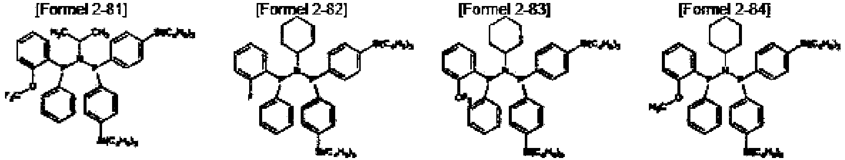

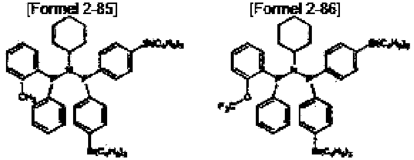

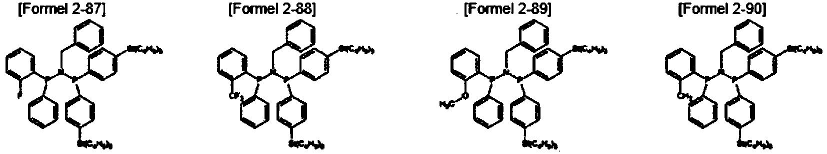

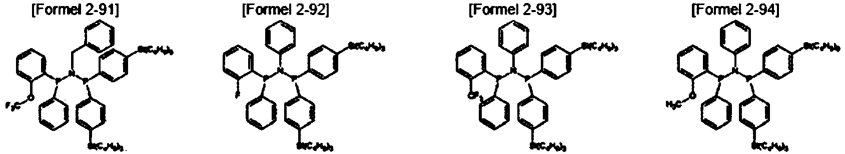

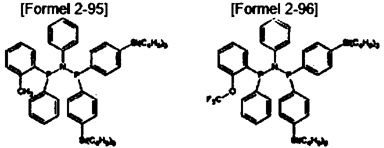

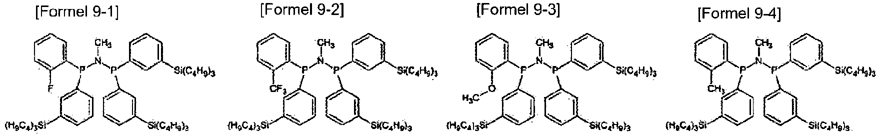

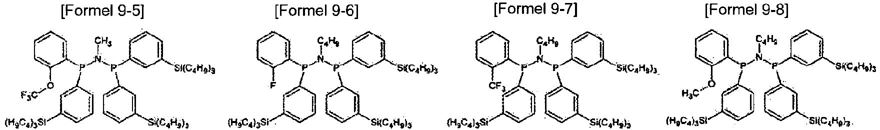

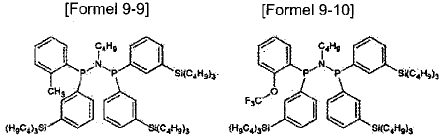

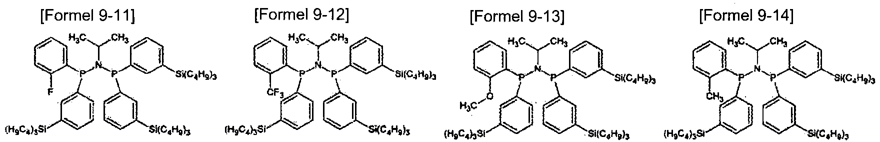

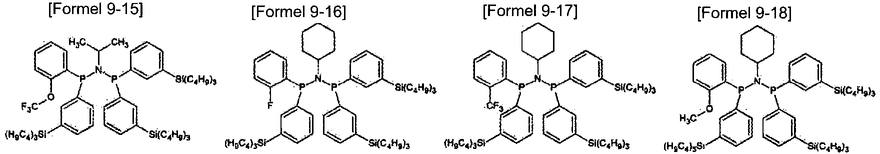

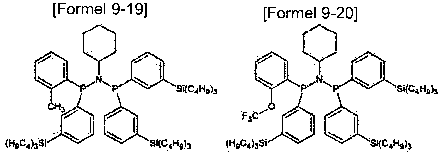

9. Organische Chromverbindung, umfassend die Ligandenverbindung gemäß Anspruch 1 und zweizähnig an die Ligandenverbindung gebundenes Chrom.

10. Organische Chromverbindung nach Anspruch 9, wobei ein ungeteiltes Elektronenpaar von N und eines oder beide der zwei P der Ligandenverbindung zweizähnig an das Chrom gebunden sind.

11. Katalysatorzusammensetzung, umfassend die Ligandenverbindung gemäß Anspruch 1, Chrom und einen Co-Katalysator.

12. Katalysatorzusammensetzung nach Anspruch 11, wobei der Co-Katalysator eine oder mehrere Verbindungen, ausgewählt aus durch die nachstehende Formel 10 bis Formel 13 dargestellten Verbindungen, ist:
[Formel 10] -[Al(R¹³)-O]ₐ
wobei in der obigen Formel 10
jedes R¹³ unabhängig eine Halogengruppe, eine Hydrocarbylgruppe mit 1 bis 20 Kohlenstoffatomen oder eine Hydrocarbylgruppe mit 1 bis 2 Kohlenstoffatomen, die mit einer Halogengruppe substituiert ist, ist und
a eine ganze Zahl von 2 oder größer ist,
[Formel 11] E(R¹⁴)₃
wobei in der obigen Formel 11
E Aluminium oder Bor ist und
jedes R¹⁴ unabhängig Wasserstoff, eine Halogengruppe, eine Hydrocarbylgruppe mit 1 bis 20 Kohlenstoffatomen oder eine Hydrocarbylgruppe mit 1 bis 20 Kohlenstoffatomen, die mit einer Halogengruppe substituiert ist, ist,
[Formel 12] [L-H]⁺[G(Y)₄]⁻
[Formel 13] [L]⁺[G(Y)₄]⁻
wobei in den obigen Formeln 12 und 13
L eine neutrale oder eine kationische Lewis-Säure ist,
[L-H]⁺ eine Brønsted-Säure ist,
G ein Element der Gruppe 13 ist und
jedes Y unabhängig eine substituierte oder unsubstituierte Alkylgruppe mit 1 bis 20 Kohlenstoffatomen oder eine substituierte oder unsubstituierte Arylgruppe mit 6 bis 20 Kohlenstoffatomen ist, wobei, wenn die Alkylgruppe oder Arylgruppe substituiert ist, der Substituent eine Halogengruppe, eine Hydrocarbylgruppe mit 1 bis 20 Kohlenstoffatomen, eine Alkoxygruppe mit 1 bis 20 Kohlenstoffatomen oder eine Aryloxygruppe mit 6 bis 20 Kohlenstoffatomen ist.

13. Verfahren zur Herstellung eines linearen alpha-Olefins, wobei das Verfahren (S10) das Oligomerisieren von Ethylen in Gegenwart der Katalysatorzusammensetzung gemäß Anspruch 11 umfasst.

14. Verfahren nach Anspruch 13, wobei das lineare alpha-Olefin 1-Hexen, 1-Octen oder eine Mischung davon ist.

## Revendications

1. Composé ligand représenté par l'une quelconque des formules 2 à 9 ci-dessous : 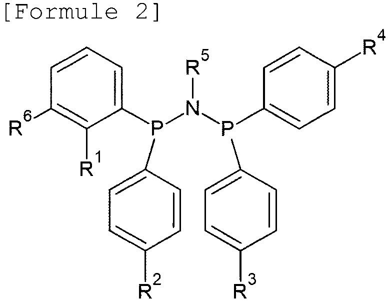 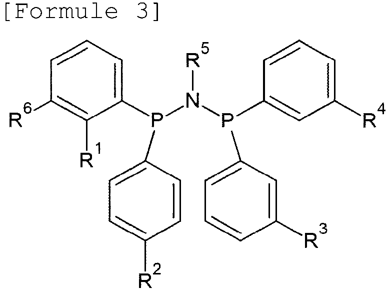 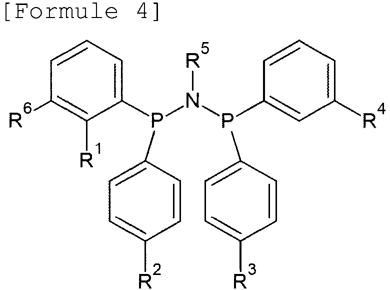 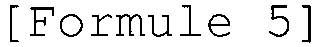 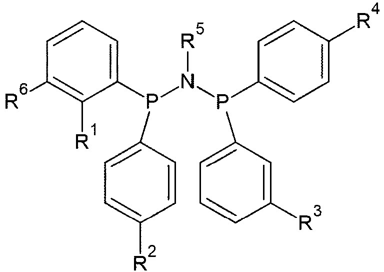 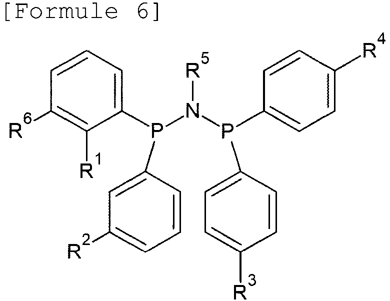 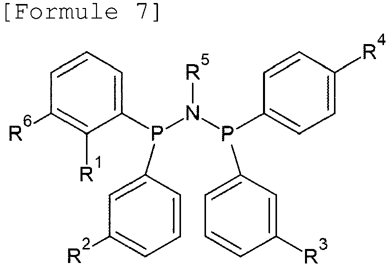 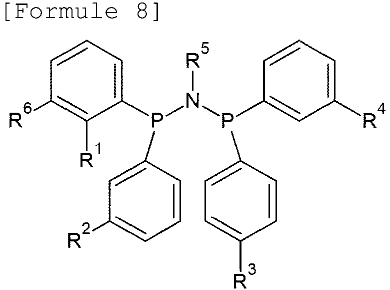 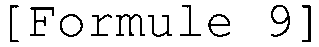 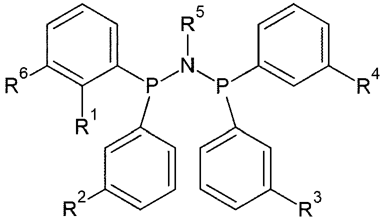 dans lesquelles, dans les formules 2 à 9 ci-dessus,
R¹ représente un atome d'halogène, un groupe alkyle comportant de 1 à 10 atomes de carbone, substitué ou non par un atome d'halogène, un groupe alcoxy comportant de 1 à 10 atomes de carbone, substitué ou non par un atome d'halogène, un groupe alkylthio comportant 1 à 10 atomes de carbone, substitué ou non par un groupe halogène, un groupe alkylsulfonate comportant 1 à 10 atomes de carbone, substitué ou non par un groupe halogène, ou un groupe trialkylsilyle dans lequel les groupes alkyle comportent chacun indépendamment 1 à 10 atomes de carbone, ou R¹ est lié à R⁶ pour former un cycle hydrocarboné aromatique monocyclique ou polycyclique, ou un hétérocycle monocyclique ou polycyclique, dans lequel, si R⁶ et R¹ ne sont pas liés l'un à l'autre pour former un cycle hydrocarboné aromatique monocyclique ou polycyclique, ou un hétérocycle monocyclique ou polycyclique, R⁶ est un atome d'hydrogène ;
R² à R⁴ représentent chacun indépendamment un atome d'hydrogène, un groupe alkyle ayant de 5 à 20 atomes de carbone, un groupe cycloalkyle ayant de 5 à 20 atomes de carbone, ou un groupe trialkylsilyle dans lequel les groupes alkyle ont chacun indépendamment de 1 à 10 atomes de carbone, R² à R⁴ n'étant pas simultanément des atomes d'hydrogène ; et
R⁵ est un groupe alkyle ayant de 1 à 20 atomes de carbone, un groupe alkyle ayant de 1 à 20 atomes de carbone et substitué par un groupe aryle ayant de 6 à 10 atomes de carbone, un groupe cycloalkyle ayant de 5 à 10 atomes de carbone, ou un groupe cycloalkyle ayant de 5 à 10 atomes de carbone condensé avec un groupe aryle ayant de 6 à 10 atomes de carbone.

2. Le composé ligand selon la revendication 1, dans lequel R¹ représente un atome de fluor, un groupe alkyle comportant 1 à 5 atomes de carbone substitué ou non substitué par un atome de fluor, un groupe alcoxy comportant 1 à 5 atomes de carbone substitué ou non substitué par un atome de fluor, un groupe alkylthio comportant 1 à 5 atomes de carbone substitué ou non substitué par un atome de fluor, un groupe alkylsulfonate comportant 1 à 5 atomes de carbone, substitué ou non par du fluor, ou un groupe trialkylsilyle dans lequel les groupes alkyle comportent chacun indépendamment 1 à 5 atomes de carbone, ou R¹ est lié à R⁶ pour former un hétérocycle monocyclique ou polycyclique.

3. Le composé ligand selon la revendication 1, dans lequel R¹ est un groupe fluoro, un groupe trifluorométhyle, un groupe méthoxy, un groupe méthyle, un groupe trifluorométhoxy, un groupe trifluorométhylthio, un groupe méthylthio, un groupe méthylsulfonate ou un groupe triméthylsilyle, ou R¹ est lié à R⁶ pour former un furane ou un dibenzofurane.

4. Le composé ligand selon la revendication 1, dans lequel R² à R⁴ représentent chacun indépendamment un atome d'hydrogène, un groupe alkyle ayant 8 à 12 atomes de carbone, un groupe tripropylsilyle ou un groupe tributylsilyle, R² à R⁴ n'étant pas simultanément des atomes d'hydrogène.

5. Le composé ligand selon la revendication 1, dans lequel R² à R⁴ représentent chacun indépendamment un groupe n-décyle, un groupe tripropylsilyle ou un groupe tributylsilyle.

6. Le composé ligand selon la revendication 1, dans lequel R² représente un atome d'hydrogène, et R³ et R⁴ représentent chacun indépendamment un groupe n-décyle, un groupe tripropylsilyle ou un groupe tributylsilyle.

7. Le composé ligand selon la revendication 1, dans lequel R⁵ est un groupe alkyle ayant de 3 à 5 atomes de carbone, un groupe alkyle ayant de 1 à 5 atomes de carbone substitué par un groupe aryle ayant de 6 à 10 atomes de carbone, un groupe cycloalkyle ayant 5 à 8 atomes de carbone, ou un groupe cycloalkyle ayant 5 à 10 atomes de carbone condensé avec un groupe aryle ayant 6 à 10 atomes de carbone.

8. Le composé ligand selon la revendication 1, qui est représenté par l'une quelconque des formules 2-1 à 2-96 et des formules 9-1 à 9-20 ci-dessous :

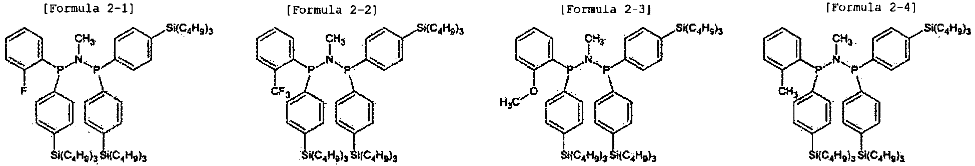

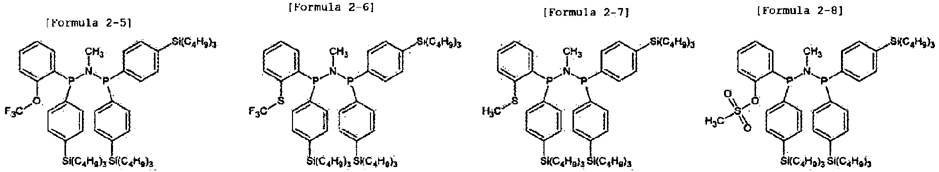

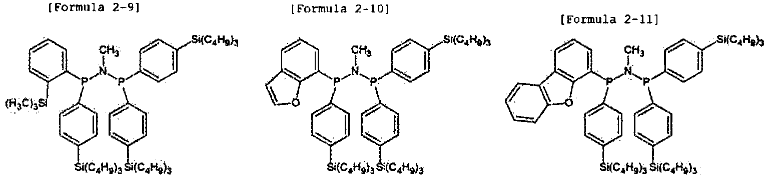

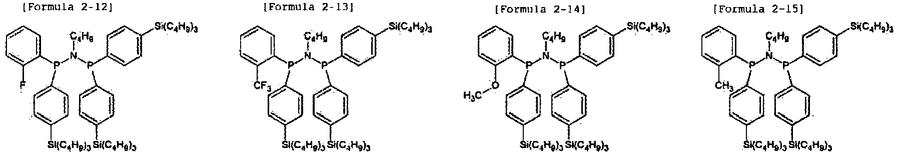

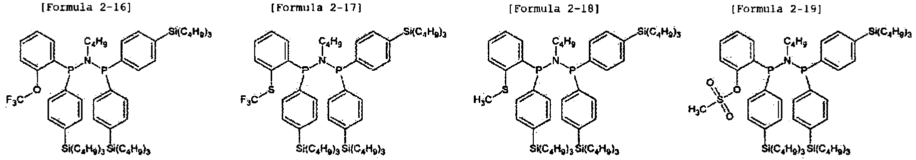

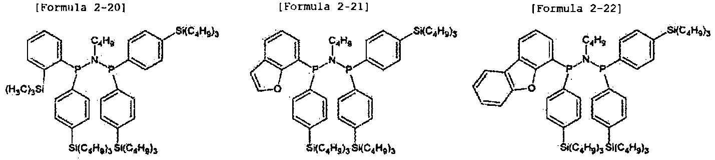

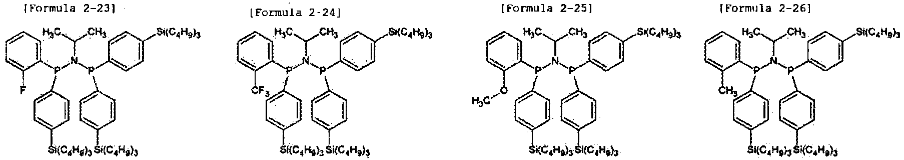

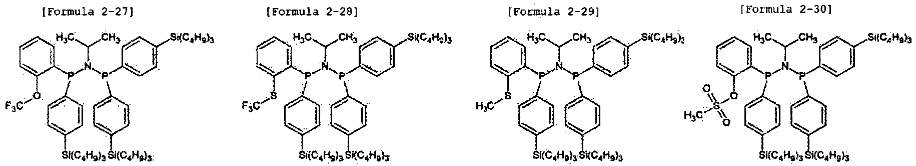

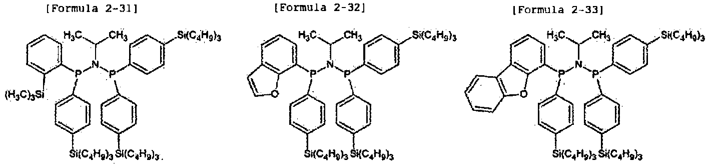

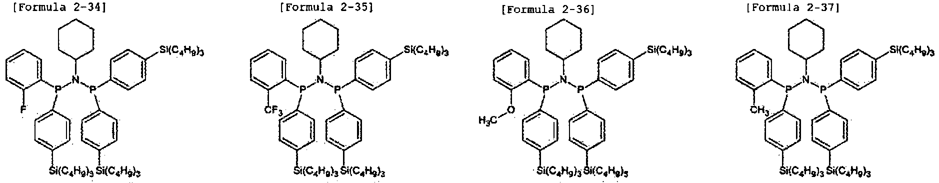

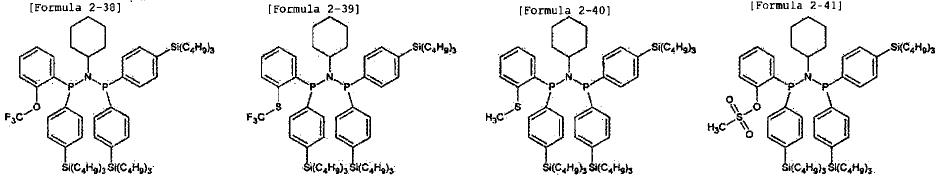

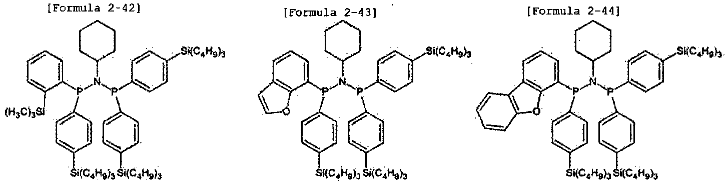

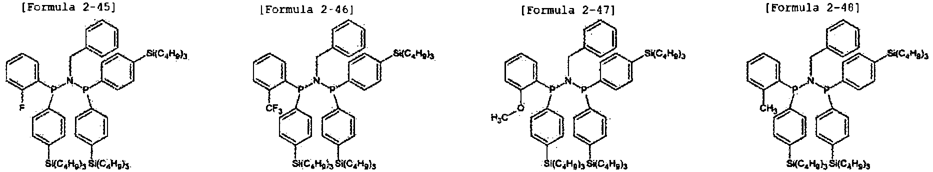

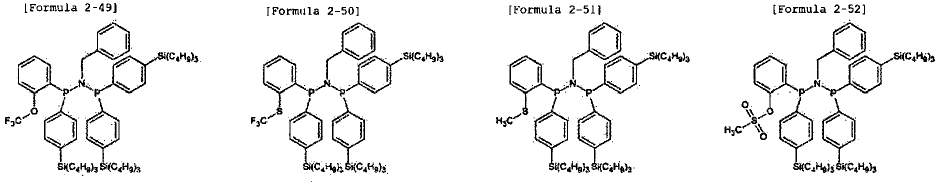

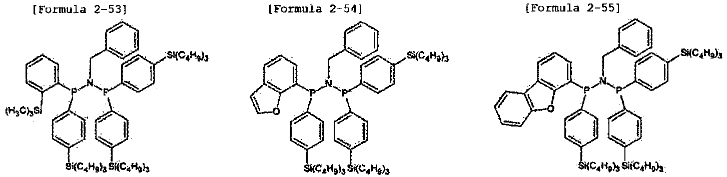

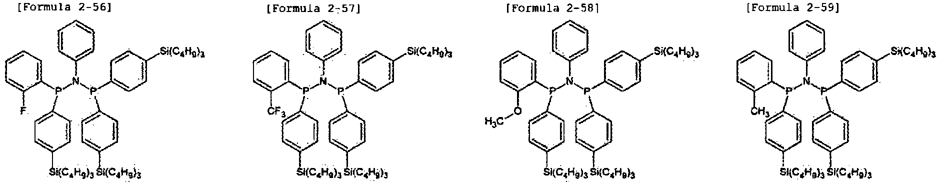

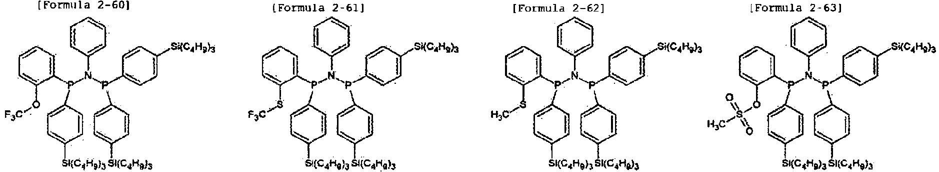

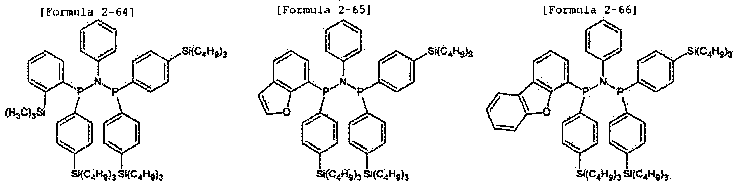

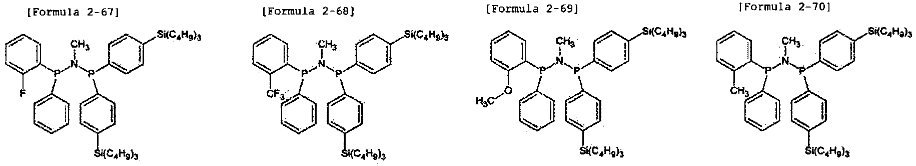

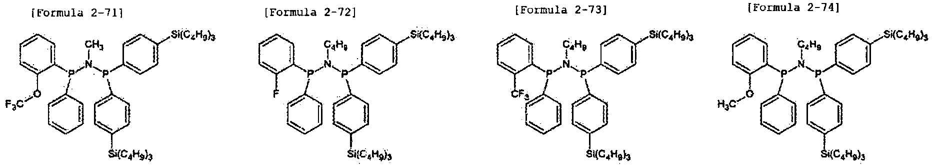

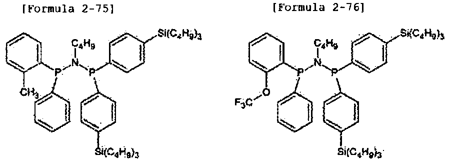

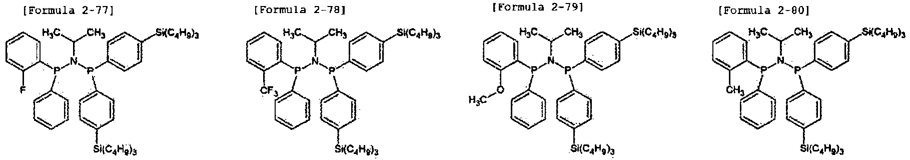

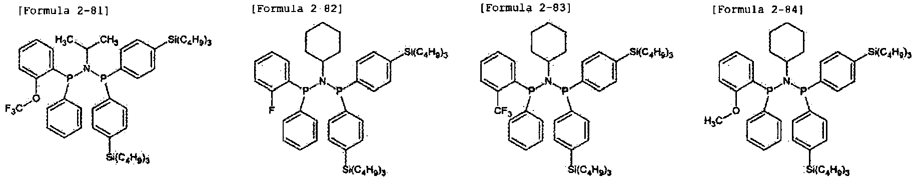

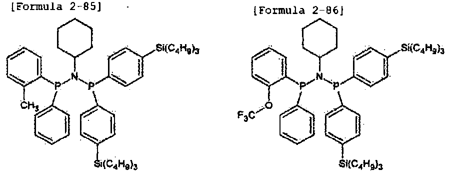

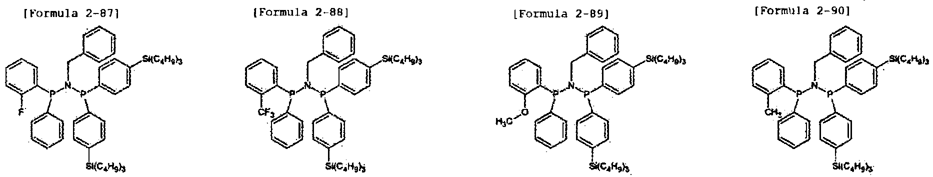

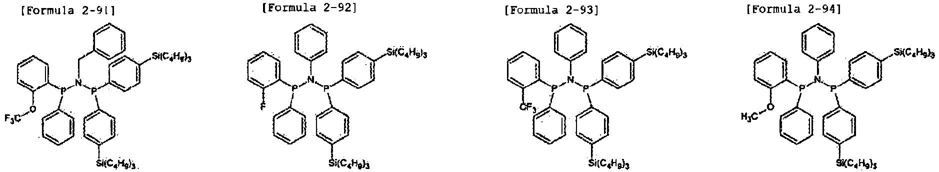

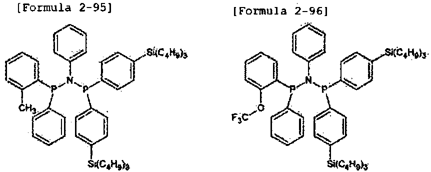

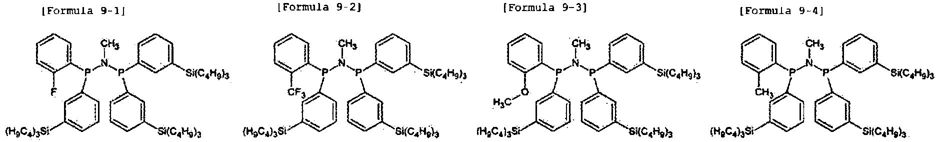

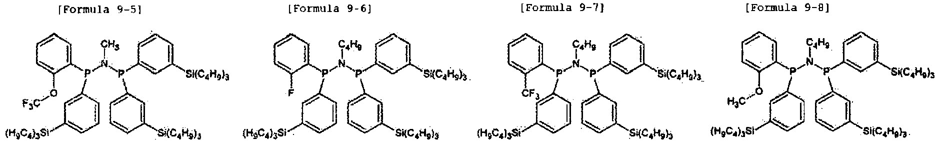

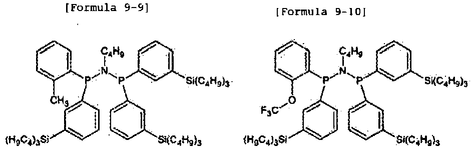

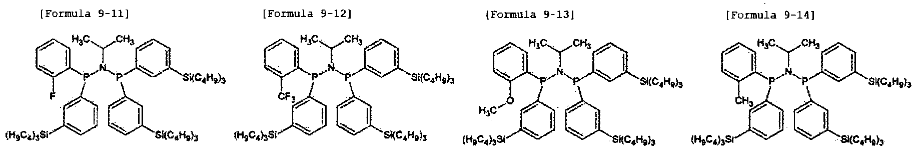

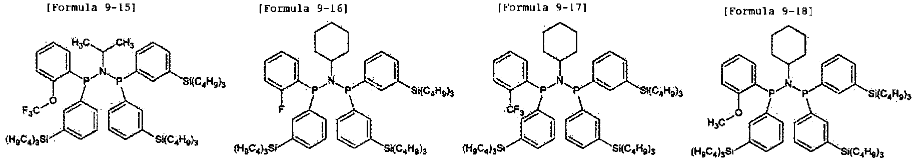

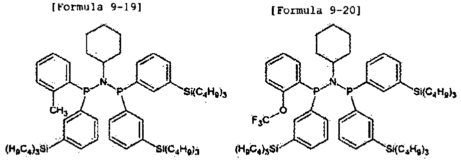

9. Composé organique du chrome comprenant le composé ligand selon la revendication 1 et du chrome lié de manière bidentée au composé ligand.

10. Le composé organique du chrome selon la revendication 9, dans lequel une paire d'électrons non partagée entre l'atome de N et l'un ou les deux atomes de P du composé ligand est liée de manière bidentée au chrome.

11. Composition catalytique comprenant le composé ligand selon la revendication 1, du chrome et un co-catalyseur.

12. La composition catalytique selon la revendication 11, dans laquelle le co-catalyseur est un ou plusieurs composés choisis parmi les composés représentés par les formules 10 à 13 ci-dessous :
[Formule 10] - [Al(R¹³)-O]ₐ-
-dans laquelle, dans la formule 10 ci-dessus,
chaque R¹³ est indépendamment un groupe halogène, un groupe hydrocarbyle ayant 1 à 20 atomes de carbone, ou un groupe hydrocarbyle ayant 1 à 20 atomes de carbone substitué par un groupe halogène, et
a est un nombre entier égal ou supérieur à 2 ;
[Formule 11] E(R¹⁴)₃
dans laquelle, dans la formule 11 ci-dessus,
E est l'aluminium ou le bore, et
chaque R¹⁴ est indépendamment un atome d'hydrogène, un groupe halogène, un groupe hydrocarbyle ayant de 1 à 20 atomes de carbone, ou un groupe hydrocarbyle ayant de 1 à 20 atomes de carbone substitué par un groupe halogène ;
[Formule 12] [L-H]⁺[G(Y)₄]⁻
[Formule 13] [L]⁺[G(Y)₄]⁻
dans lesquelles, dans les formules 12 et 13 ci-dessus,
L est un acide de Lewis neutre ou cationique,
[L-H]⁺ est un acide de Brønsted,
G est un élément du groupe 13, et
chaque Y est indépendamment un groupe alkyle substitué ou non substitué ayant 1 à 20 atomes de carbone, ou un groupe aryle substitué ou non substitué ayant 6 à 20 atomes de carbone, dans lequel, si le groupe alkyle ou le groupe aryle est substitué, le substituant est un groupe halogène, un groupe hydrocarbyle comportant 1 à 20 atomes de carbone, un groupe alcoxy comportant 1 à 20 atomes de carbone, ou un groupe aryloxy comportant 6 à 20 atomes de carbone.

13. Procédé de préparation d'une alpha-oléfine linéaire, le procédé comprenant (S10) l'oligomérisation de l'éthylène en présence de la composition catalytique selon la revendication 11.

14. Le procédé selon la revendication 13, dans lequel l'alpha-oléfine linéaire est le 1-hexène, le 1-octène ou un mélange de ceux-ci.
